(19) Europäisches Patentamt / European Patent Office / Office européen des brevets

(11) **EP 4 223 300 A1**

(12) **EUROPEAN PATENT APPLICATION**
published in accordance with Art. 153(4) EPC

(43) Date of publication:
**09.08.2023  Bulletin 2023/32**

(21) Application number: **21874578.4**

(22) Date of filing: **30.09.2021**

(51) International Patent Classification (IPC):
*A61K 35/741* (2015.01)        *A61K 35/742* (2015.01)
*A61K 35/747* (2015.01)        *A61K 35/745* (2015.01)
*A61K 36/064* (2006.01)        *A61K 45/06* (2006.01)
*A61K 39/00* (2006.01)         *A61P 35/00* (2006.01)
*A61P 37/04* (2006.01)         *A61P 29/00* (2006.01)
*A61P 5/00* (2006.01)          *A61P 17/00* (2006.01)
*A61K 35/12* (2015.01)         *A61K 36/00* (2006.01)
*A61K 36/02* (2006.01)

(52) Cooperative Patent Classification (CPC):
A61K 35/12; A61K 35/741; A61K 35/742;
A61K 35/745; A61K 35/747; A61K 36/00;
A61K 36/02; A61K 36/064; A61K 39/00;
A61K 45/06; A61P 5/00; A61P 17/00; A61P 29/00;
A61P 35/00; A61P 37/04

(86) International application number:
**PCT/CN2021/122132**

(87) International publication number:
**WO 2022/068924 (07.04.2022 Gazette 2022/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2020  CN 202011064448
30.09.2020  CN 202011059746
29.09.2021  CN 202111147464**

(71) Applicants:
• **Chengdu Kuachangaopu Medical Technology
Co., Ltd.**
**Chengdu, Sichuan 610000 (CN)**
• **Kuachang Inc.**
**Anaheim, CA 92804 (US)**

(72) Inventors:
• **ZOU, Fanglin**
**Chengdu, Sichuan 610041 (CN)**
• **ZOU, Lichang**
**Chengdu, Sichuan 610041 (CN)**
• **WANG, Jian Xia**
**Chengdu, Sichuan 610041 (CN)**

(74) Representative: **ABG Intellectual Property Law,
S.L.**
**Avenida de Burgos, 16D
Edificio Euromor
28036 Madrid (ES)**

(54) **USE OF PROBIOTIC COMPONENT AND PHARMACEUTICAL COMPOSITION CONTAINING PROBIOTIC COMPONENT**

(57)    Provided in the present invention are a new use of a probiotic component and a local pharmaceutical composition comprising the probiotic component, and a use of the probiotic component as an active ingredient capable of providing a therapeutic effect in the manufacture of a local pharmaceutical composition for the treatment of local lesion diseases. The local medicament of the present invention provides a completely new pharmacology, so as to realize chemical ablation-like local treatment comprising local effect (or local synergy) and/or immunotherapy comprising a secondary immunological effect associated with the local effect (or local synergy), which cannot be achieved by means of the existing probiotic composition. Moreover, the local medicament of the present invention has almost completely non-toxic systemic safety and a significantly higher long-term efficacy, has a tremendous potential against rapidly growing tumors, large tumors and hypovascular tumors, and is absent of drug resistance, is convenient to prepare and has low costs.

**Description**

**Technical Field**

The present invention relates to the technical field of pharmaceutical formulations, and in particular to a novel use of a probiotic component and to a pharmaceutical composition comprising the probiotic component.

**Background Art**

From the point of view of lesion tissue, a solid tumor is a representative model of a local lesion disease, which is a tumor disease having tumor body symptoms, and the tumor body comprises a tumor body tissue comprising tumor cells along with a larger number of other components (sometimes also referred to as a microenvironment of tumor cells), such as multiple other cells, multiple intercellular substances, multiple ducts, etc. In the case of a pancreatic cancer tumor body, for example, pancreatic cancer cells make up only about 30% by volume of the tumor body.

Cytotoxic drugs and chemical ablation agents are common drugs used in the treatment of solid tumors for nearly a hundred years. Although the intratumoral concentration of cytotoxic drugs can be increased through intratumoral administration, their drug efficacies are only slightly increased within the expected range of cancer cell inhibition dynamics (usually less than 200%); considering the patient's compliance, cytotoxic drugs are basically administered systemically by oral administration or intravenous injection. Chemical ablation agents, on the other hand, are usually characterized by tumor tissue destruction as a pharmacological characteristic, rather than tumor cell destruction, and their intra-tumor administration is much more pharmaceutically effective than their expected range of cancer cell inhibition dynamics, with pharmacology significantly different from systemic administration, such as high purity of ethanol, high concentration of acidifier or basifier, etc.; however, due to high irritation, low long-term effect and very limited intervention volume and intervention site, for example, no more than 0.2 ml/kg of acid and base dosages, as well as restrictions on the organ where the tumor is located, and limited ablation for a tumor body margin, etc., it has gradually faded from clinical use.

In recent years, there have been some advances in tumor immunotherapeutic approaches represented by tumor vaccines, cellular adoptive therapies, and immune checkpoint inhibitors. These therapeutic agents vary widely, and require very high patient genetic specificity and tumor specificity, causing that an obvious difference in tumor residual weight between the administered group and the negative control group can hardly be observed in common animal experiments, with a very small range of clinical indications.

The World Health Organization defines probiotics as live, non-pathogenic bacteria that confer some health benefit to a host when being taken in sufficient quantities. Research suggests that probiotics may have three mechanisms of action to support their beneficial properties: (1) competing with a pathogen for binding to pathogen attachment sites on intestinal epithelial cells or consuming nutrient substances for pathogen, thus to inhibit growth of the pathogenic bacterium; (2) improving intestinal barrier function; and (3) enhancing the immune function of an organism. Among them, the research on probiotics to improve the immune function of an organism is of great interest. There are studies on the use of yeast as a feed additive for enhancing the immune function of pigs (e.g. Patent Publication No.: CN106387398A). There are also studies on the use of inactivated yeast for enhancing immune function, anti-bacteria and anti-virus (e.g. Patent Publication No. CN108524925A).

In prior art, the antitumor effect of probiotics is believed to be mainly related to their bacterial immunogenicity. However, this immunogenicity does not show the therapeutic effect expected from a therapeutic vaccine antigen and thus probiotics is currently used mainly to enhance the immunity of a patient. The immune heterogeneity is one of the characteristics of tumor heterogeneity, and this pharmacology makes the immune enhancement of probiotics only exist for colorectal cancer, indicating a strong tumor specificity. Therefore, relative to other drugs, probiotics does not show meaningful tumor growth inhibition (tumor inhibition ratio ≤15% or relative tumor proliferation ratio ≥ 85%) in animal experiments, and these antitumor immune enhancement effects as provided are very limited, which only play an auxiliary treatment role, far from providing a therapeutic effect.

Developing a new pharmacology of probiotics to break through the prior art expectations of only provision of immune enhancement function and the like, can provide a new solution for clinical treatment.

**Summary of the invention**

In view of this, the present invention aims to propose a new pharmacology based on a probiotic component - i.e. providing a local effect (or a local synergy) and a secondary effect (or a secondary synergy) thereof, thus the manufactured local pharmaceutical composition achieves a high efficiency and a low toxicity, especially in medium- and long-term, to provide a variety of clinical options.

A use of a probiotic component as an active ingredient capable of providing a therapeutic effect in the manufacture of a local pharmaceutical composition for the treatment of local lesion diseases, wherein said therapeutic effect comprises a local treatment involving a local effect (or a local synergy) or/and an immunotherapy associated with the local effect (or the local synergy).

Preferably, said pharmaceutical composition further comprises a chemical active ingredient capable of synergizing with said probiotic component, and wherein an amount ratio of said probiotic component to said chemical active ingredient (weight concentration of the probiotic component/weight concentration of the co-administration substance) is (1-110)/(1-100).

A local pharmaceutical composition for the treatment of local lesion diseases comprising a probiotic component capable of providing a therapeutic effect, and a suitable pharmaceutically acceptable carrier, but not comprising a salt or a monosaccharide dedicated to increasing blood osmolarity to a normal physiological level, wherein said therapeutic effect comprises a local treatment or/and an immunotherapy involving a local effect (or a local synergy).

Preferably, it also optionally comprises a chemical active ingredient capable of synergizing with said probiotic component.

A local pharmaceutical composition for the treatment of local lesion diseases, comprising a probiotic component capable of providing a therapeutic effect, a chemical active ingredient capable of synergizing with said probiotic component, and a suitable pharmaceutically acceptable carrier, and an amount ratio of said probiotic component to said chemical active ingredient (weight concentration of the probiotic component/weight concentration of the co-administration substance) is (1-110)/(1-100), wherein said therapeutic effect comprises a local treatment or/and an immunotherapy involving a local effect (or a local synergy).

Preferably, said probiotic component is selected from: a preparation having a minimized bacterial immunogenicity and derived-from natural probiotics or engineered probiotics thereof, or engineered analogues of the preparation, wherein said preparation is preferably one or more selected from a group comprising: a probiotic water-soluble component, a probiotic semi-fluid-like component, a probiotic component water-insoluble particle, inactivated probiotics, and derivatives thereof.

Preferably, said probiotic water-soluble component is selected from a group comprising one or more of a probiotic or disrupted probiotic supernatant component, a probiotic extract, a probiotic intracellular water-soluble component, water-soluble derivatives of water-soluble components or non-water-soluble components from probiotic cell wall components, and derivatives thereof; said probiotic semi-fluid-like component is preferably one or more selected from probiotic components whose aqueous mixture can form a semi-fluid-like composition, such as one or more of probiotic polysaccharides and analogues thereof; said probiotic component water-insoluble particle is one or more selected from a group comprising of: a disrupted probiotic precipitation component, a probiotic cell wall polysaccharide particle, a probiotic cell wall polysaccharide nanoparticle, and derivatives thereof; said inactivated probiotics is preferably selected from but not limited to inactivated probiotics having intact morphology;

Wherein said probiotic water-soluble component is selected from a group comprising one or more of a probiotic water-soluble β-glucan, preferably selected from a water-soluble β-glucan of greater than 90% purity, a probiotic ribonucleic acid, and derivatives thereof.

Preferably, wherein said probiotic is selected from a group comprising one or more of natural or/and engineered probiotic Bacillus, probiotic Lactobacillus, probiotic Bifidobacterium, probiotic Fungus.

Preferably, said Bacillus comprises one or more selected from a group comprising of: Bacillus licheniformis, Bacillus subtilis, Bacillus pumilus, Bacillus natto; said Lactobacillus comprises one or more selected from a group comprising of: Lactobacillus acidophilus, Lactobacillus casei, Lactobacillus plantarum, Lactobacillus pumilus and Lactobacillus fermentum; said Bifidobacterium comprises one or more selected from a group comprising of: Bifidobacterium longum, Bifidobacterium adolescentis, Bifidobacterium breve, Enterococcus faecium, and Streptococcus fecalis; said Fungus comprises one or more selected from a group comprising of: yeasts and Brettanomyces bruxellensis (Saccharomyces boulardii), wherein said yeasts comprises one or more selected from a group comprising of: Saccharomyces cerevisiae, Saccharomyces delbouillis, Saccharomyces Wickham, Saccharomyces Pichia, Candida utilis, whey yeast.

Preferably, wherein said probiotics comprise selected from Saccharomyces cerevisiae or/and Brettanomyces bruxellensis (Saccharomyces brucella).

Preferably, wherein said suitable carrier is water; said chemical active ingredient is soluble in water and said probiotic component is water-insoluble and is one or more selected from a group comprising of inactivated probiotics, a probiotic component water-insoluble particle, a probiotic semi-fluid-like component, and derivatives thereof.

Preferably, wherein said probiotic component is present in the pharmaceutical composition at a concentration of > 0.1 %, ≥0.25 %, 0.25-25 %, preferably 0.5-15 %, more preferably 1-15% or 5-15%.

Preferably, wherein if said probiotic component comprises said inactivated probiotics, then said inactivated probiotics is present in the pharmaceutical composition at a concentration of > 0.3%, >_0.75%, 0.75-15, preferably 1.5-15% or 5-15%; if said probiotic component comprises said probiotic water-soluble component, then said probiotic water-soluble component is present in the pharmaceutical composition at a concentration of > 0.1, or 0.15-25%, preferably 0.35-5% or 5-15%; if said probiotic component comprises said probiotic water-insoluble component particle, then said probiotic water-insoluble component particle is present in the pharmaceutical composition at a concentration of > 0.5, or 0.5-15%, preferably 1.5-15% or 5-15%; if the pharmaceutical composition is a semi-fluid-like composition, then said probiotic semi-fluid-like component is present in the pharmaceutical composition at a concentration of > 2.5%, 2.6-25%, preferably 5-15%.

Preferably, wherein said chemical active ingredient is one or more selected from a group comprising of a weak local acting compound and/or a cytotoxic agent, and wherein when said chemical active compound comprises a cytotoxic agent, the amount ratio of the probiotic component to the cytotoxic agent (W $_{\text{probiotic component}}$ /W $_{\text{cytotoxic agent}}$) is (1-110)/(1-100); when said chemical active compound comprises a weak local acting compound, the amount ratio of the probiotic component to the weak local acting compound (W $_{\text{probiotic component}}$ /W $_{\text{weak local acting compound}}$) is (1-90)/(1-100).

Preferably, wherein said weak local acting compound is selected from a group comprising one or more of: an amino acid nutrient, a vital dye, a quinine drug, a low concentration acidifier, a low concentration basifier, a pH buffering system comprising an acidifier or/and a basifier, and wherein when said weak local acting compound comprises said amino acid nutrient, the amount ratio of the probiotic component to the amino acid nutrient, (W $_{\text{probiotic component}}$/W $_{\text{amino acid nutrient}}$) is (1-20)/(1-100); when said weak local acting compound comprises said vital dye, the amount ratio of the probiotic component to the vital dye, (W $_{\text{probiotic component}}$/W $_{\text{vital dye}}$) is (7-90)/(1-100); when said weak local acting compound comprises said quinine drug, then the amount ratio of the probiotic component to the quinine drug, (W $_{\text{probiotic component}}$/W $_{\text{quinine drug}}$) is (2-90)/(1-100); when said weak local acting compound comprises said acidifier or/and basifier, the amount ratio of the probiotic component to the acidifier or/and basifier, (W $_{\text{probiotic component}}$/W $_{\text{acidifier or/and basifier}}$) is (2-60)/(1-100).

Preferably, wherein said chemical active ingredient is selected from a vital dye and one or more other said chemical active ingredients. Preferably, wherein said chemical active ingredient is selected from the cytotoxic agent and the vital dye.

Preferably, wherein said amino acid nutrient is amino acids or salts thereof selected from a group comprising, or oligopeptides and polypeptides comprising or consisting of arginine, lysine, glycine, cysteine, alanine, serine, aspartic acid, glutamic acid, and wherein said amino acid nutrient is present at a concentration of >2.5%, or 5-30%, preferably 5-25%.

Preferably, wherein said vital dye is selected from a group comprising Bengal Red or/and one or more of the following methylene blue-like dyes: methylene blue, patent blue, isosulphur blue, neo-methylene blue, and wherein said Bengal Red is present at a concentration of 2.5%-20%; said methylene blue-like dyes are present at a concentration of ≥ 0.25%, or 0.25-2.5%, preferably 0.5-2.5%.

Preferably, wherein said acidifier is selected from one or more of a strong acid or/and a weak acid, and the amount ratio of said probiotic component to said acidifier (weight concentration of the probiotic component/weight concentration of the acidifier) is (1-20)/(0.5-50), and the concentration of said acidifier is ≥0.5%, 0.5-2% (strong acid), or 2-15% (weak acid), wherein said strong acid is, for example, hydrochloric acid; said weak acid is, for example, glycolic acid, acetic acid, lactic acid, citric acid, malic acid; said basifier is, for example, selected from one or more of strong or/and weak alkalis, and the amount ratio of said probiotic component to said basifier (weight concentration of the probiotic component/weight concentration of the basifier) is (1-20)/(0.5-50), and the concentration of said basifier is ≥ 0.5%, 0.5-5% (strong alkali), or 2-15% (weak alkali), wherein said strong alkali is, for example, sodium hydroxide, potassium hydroxide; said weak alkali is, for example, sodium dihydrogen phosphate, sodium bicarbonate, sodium carbonate.

Preferably, wherein said cytotoxic agent comprises one or more selected from a group comprising of: drugs that disrupt DNA structure and function, such as cyclophosphamide, carmustine, metal platinum complexes, doxorubicin drugs, topotecan, irinotecan; drugs intercalated in DNA to interfere with RNA transcription, such as antitumor antibiotics; drugs that interfere with DNA synthesis, such as 5-fluorouracil (5-Fu), furofluorouracil, difurofluorouracil, cytosine arabinoside, cyclic cytidine, 5-azacytidine; drugs that affect protein synthesis, such as colchicine drugs, vincristine drugs, and taxane drugs, and the concentration of said cytotoxic agent is ≥ 0.1%, 0.1-15%.

Preferably, wherein said pharmaceutical composition further optionally comprises a biologically active ingredient, wherein said biologically active ingredient is selected from a group comprising one or more of antigens, immunomodulatory antibodies, cytokines, adjuvants.

A method for treating a local lesion disease comprising the steps of locally administering a therapeutically effective amount of the above-described pharmaceutical composition to an individual in need thereof within a local lesion or/and outside a local lesion.

Preferably, the method comprises the step of administering a therapeutically effective amount of the above-described pharmaceutical composition to said individual within a local lesion, or within a local lesion and outside a local lesion.

Preferably, the method further comprises optionally administering one or more other treatments, such as chemotherapy, immunotherapy, radiotherapy, surgery, chemical ablation, physical ablation, before, during or after administration of said pharmaceutical composition.

Preferably, wherein the amount of said probiotic component in the pharmaceutical composition is such that it is administered at a local concentration of >0.1%, ≥ 0.25%, 0.25-25%, preferably 0.5-15%, more preferably 1-15% or 5-15%.

Preferably, wherein if said probiotic component comprises said inactivated probiotics, then the amount of said inactivated probiotics in the pharmaceutical composition should be such that it is administered at a local concentration of > 0.3%, ≥0.75%, 0.75-15, preferably 1.5-15% or 5-15%; if said probiotic component comprises said probiotic water-soluble component, then the amount of said probiotic water-soluble component in the pharmaceutical composition should be such that it is administered at a local concentration of > 0.1, or 0.15-25%, preferably 0.35-5%, or 5-15%; if said probiotic component comprises said probiotic water-insoluble component, then the amount of said probiotic water-insoluble component in the pharmaceutical composition should be such that it is administered at a local concentration of > 0.5, or 0.5-15%, preferably 1.5-15% or 3.5-15%.

Preferably, wherein the content or unit content of said pharmaceutical composition is such that its administration volume is a factor of > 0.1, 0.15-1.5, preferably 0.23-1.5 or 0.5-1.5 multiplied by the volume of the target region within the local lesion.

Preferably, wherein the content or unit content of said pharmaceutical composition is such that it is administered in a volume of ≥ 1 ml, or administered in a volume of 10-150 ml within a local lesion or/and 1.5-50 ml outside a local lesion.

Preferably, wherein said local lesion comprises tumor cells or/and fibroblasts.

Preferably, wherein said local lesion disease comprises a tumor, a non-tumor enlargement, a local inflammation, an endocrine gland dysfunction, and a skin disease, wherein said tumor comprises malignant and non-malignant solid tumors.

Preferably, wherein said solid tumor comprises one or more of the following tumors and secondary tumors thereof: breast cancer, pancreatic cancer, thyroid cancer, nasopharyngeal cancer, prostate cancer, liver cancer, lung cancer, intestinal cancer, oral cancer, esophageal cancer, gastric cancer, laryngeal cancer, testicular cancer, vaginal cancer, uterine cancer, ovarian cancer, brain tumor, and lymphoma.

Preferably, wherein said local effect (or local synergy) comprises a local chemical effect (or local chemical synergy) and optionally occurring other effects; said local treatment comprises a chemical ablation-like and optionally other chemotherapies of one or more local lesions; said immunotherapy comprises a secondary immunological effect of said local effect (or local synergy) and optionally other immunological effects within or/and outside said lesion.

Preferably, wherein the patient to which said treatment is applicable is selected from a group comprising one or more of an immunosuppressed patient, a patient who can be administered locally within the lesion, a patient whose local lesion tissue is amenable to a chemical ablation-like treatment, a patient who can produce a secondary immune substance within the local lesion, and a patient who can produce a secondary immune substance within the administration region outside a lesion.

A pharmaceutical composition comprising a probiotic component and a suitable pharmaceutically acceptable carrier, for the treatment of a local lesion disease, wherein said local lesion disease comprises a solid tumor.

A pharmaceutical kit comprises one or more containers containing the pharmaceutical composition as described above. Preferably, the pharmaceutical kit further comprises instructions or labels on how to administer said pharmaceutical composition to an individual in need thereof. Preferably, wherein said administration comprises administration within the local lesion, or administration within the local lesion and outside the local lesion, wherein said administration outside the local lesion comprises, for example, subcutaneous injection under the axilla of said individual.

To achieve the above objects, the technical solution of the present invention is realized as follows: a local medication (or medicine) for the treatment of a local lesion, comprising a probiotic component and a pharmaceutically acceptable carrier, and the concentration of said probiotic component is 0.25-25%.

Preferably, said local medication further comprises a weak local acting compound and/or a cytotoxic agent, wherein said weak local acting compound is at a concentration of 1 -20% and said cytotoxic agent is at a concentration of 1-5%.

Preferably, the constitution of said local medication comprise 0.25%-10% probiotic component, further comprise 0.1-2.5% cytotoxic agent and/or 5-20% amino acid nutrient and/or 0.5-1% active dye.

Preferably, the constitution of said local medication comprise 0.5%-10% probiotic component, further comprise 0.5-1% methylene blue or 5-20% lysine or 0.1-2.5% gemcitabine or 5-fluorouracil; the constitution of said local medication comprise 0.25-0.5% probiotic component and 5-10% arginine.

The constitution of said local medication are 2.5% probiotic component, 1% 5-fluorouracil, and water for injection as remainder; or 2.5% probiotic component 2.5%, 10% arginine, and water for injection as remainder; or 2.5% probiotic component, 1% 5-fluorouracil, 10% arginine, and water for injection as remainder; or 5% heat-inactivated Saccharomyces boulardii, 1% methylene blue, and water for injection as remainder; or 5% heat-inactivated Saccharomyces boulardii, 1% 5-fluorouracil, and water for injection as remainder; or 5% heat-inactivated Saccharomyces boulardii, 1% 5-fluorouracil, 1% methylene blue, and water for injection as remainder; or 7.5% β-glucan in a semi-fluid composition, 1% methylene blue, 1% 5-fluorouracil, and water for injection as remainder; or 5% disrupted Saccharomyces boulardii supernatant component, 1% methylene blue, 1% 5-fluorouracil, and water for injection as remainder; or 5% disrupted Saccharomyces boulardii, 1% methylene blue, 1% 5-fluorouracil, and water for injection as remainder; or 5% heat-inactivated Saccharomyces boulardii, 1% methylene blue, 1% 5-fluorouracil, and water for injection as remainder; or a semi-fluid formed after heating 5% β-glucan, 1% methylene blue, 1% 5-fluorouracil in aqueous suspension and then cooling down; or 5% water-soluble Saccharomyces cerevisiae β-glucan, 1% methylene blue, 1% 5-fluorouracil, and water for injection as remainder; or 20% Saccharomyces cerevisiae ribonucleic acid, 1% methylene blue, 1% 5-fluorouracil, and water for injection as remainder. Preferably, the constitution of said local medication comprise 10% probiotic component, 1% methylene blue, 1% 5-fluorouracil. Preferably, the constitution of said local medication comprise 0.5% probiotic component/7% sodium bicarbonate/3% sodium hydroxide or 10% probiotic component/0.7% sodium bicarbonate/0.3% sodium hydroxide. Preferably, said probiotic component comprises at least one of inactivated probiotics, probiotic water-soluble components, probiotic water-insoluble component particles, probiotic semi-fluid-like components. Preferably, said probiotics comprise at least one of Bacillus, Lactobacillus, Bifidobacterium, probiotic fungi. Preferably, said probiotic is Saccharomyces boulardii. Preferably, said local medication does not comprise an osmotic regulator, a flavoring agent.

Relative to the prior art, the local medication described in the present invention have the following advantages: providing a novel pharmacology (a local effect or a local synergy, and a secondary immunological effect therefrom), thus allowing for a local treatment including chemical ablation-like treatment or/and allowing for an immunotherapy including secondary immunological effect, which cannot be performed by the probiotic compositions in the prior art. The local treatment can produce a therapeutic efficacy (e.g. more than 3-fold tumor inhibition rate) which far exceeds that of prior art protocols, as well as indications (e.g. breaking through the limitation of tumor specificity in prior art, and the limitation of dependence on the patient's immune function). The immunotherapy can also produce an immunological efficacy that far exceeds that of prior art protocols (e.g., said secondary immunological effect or greatly enhanced immunological efficacy produced within the lesion region or/and outside the lesion region greatly exceeds the expectation of the body's immune enhancement) and indications. In addition, embodiments of the present invention further have the benefit of greatly reducing the safety risks of prior art embodiments that do not limit the administration mode.

Embodiments according to the present invention have the following advantages over the prior art of other compositions for the treatment of local lesion diseases: an almost non-toxic systemic safety and significantly higher long-term efficacy compared to existing cytotoxic agents; less demanding indication screening, and great potential for targeting fast-growing tumors, large tumors and hypovascular tumors compared to existing molecular targeted agents; significantly lower local irritation and better long-term effects compared to existing chemical ablation agents. The use and the composition of the present invention are also not plagued by the drug resistance issue encountered with existing cytotoxic drugs and existing molecularly targeted drugs. In addition, the use and the composition are easy and inexpensive to prepare and are particularly helpful in providing safe and effective treatment for a wide range of people who have difficulty affording high costs.

The present invention also provides a use of the above-described local medication in the preparation of a local pharmaceutical composition for the treatment of a local lesion disease, wherein said therapeutic effect comprises a local treatment involving a local effect (or a local synergy) or/and an immunotherapy. Preferably, the content or the unit content of said pharmaceutical composition makes the administration volume $V1 = (0.15-1.5) \times V_2$, preferably $(0.23-1.5) \times V_2$ or $(0.5-1.5) \times V_2$, wherein $V_2$ is the volume of the target region within the local lesion. Preferably, said administration volume Vi is 1.5-150 ml, or if the drug is administered within the local lesion, the administration volume Vi is 10-150 ml, or if the drug s administered outside the local lesion, the administration volume Vi is 1.5-50 ml.

Preferably, said local lesion comprises a tumor, a non-tumor enlargement, a local inflammation, an endocrine gland dysfunction, and a skin disease, wherein said tumor comprises malignant and non-malignant solid tumors. Preferably, said solid tumor is one of breast cancer, pancreatic cancer, thyroid cancer, nasopharyngeal cancer, prostate cancer, liver cancer, lung cancer, intestinal cancer, oral cancer, esophageal cancer, gastric cancer, laryngeal cancer, testicular cancer, vaginal cancer, uterine cancer, ovarian cancer, brain tumor, and lymphoma.

The present invention also provides a pharmaceutical kit comprising one or more containers containing the pharmaceutical composition as above described. Preferably, said pharmaceutical kit further comprises instructions or labels on how to administer said pharmaceutical composition to an individual in need thereof. Preferably, said administration comprises administering within the local lesion, or administering within the local lesion and outside the local lesion, wherein the administration outside the local lesion is a subcutaneous injection under the axilla.

The present invention also provides a method for treating a local lesion disease comprising administering a therapeutically effective amount of the pharmaceutical composition as above described to the patient within the local lesion or within the local lesion and outside the local lesion. Preferably, at least one of chemotherapy, immunotherapy, radiotherapy, surgery, chemical ablation, physical ablation is performed before and/or during and/or after administration of said pharmaceutical composition. Preferably, after the administration of said pharmaceutical composition, the probiotic component therein has an local administration concentration of 0.25-25%, preferably 0.5-15%, more preferably 1-15% or 5-15%.

Preferably, said patient is any of patient selected from a group comprising one or more of an immunosuppressed patient, a patient who can be administered locally within the lesion, a patient whose local lesion tissue is amenable to a chemical ablation-like treatment, a patient who can produce a secondary immune substance within the local lesion, and a patient who can produce a secondary immune substance within the administration region outside a lesion.

**Detailed Description of the Embodiments**

It is to be noted that the embodiments and the features in the embodiments of the present invention can be combined with each other unless a conflict happens. L-amino acids are abbreviated as amino acids in the present application, e.g., L-arginine is abbreviated as arginine.

If a substance exhibits therapeutic activity under specific conditions, this indicates that it can be used as an active ingredient in the treatment of a local lesion disease. The same substance can exhibit different activities or pharmacological effects under different conditions, for example, ethanol is commonly used as a bactericidal agent, while a high concentration of ethanol exhibits pharmacology of chemical ablation against tumor tissue through intratumoral administration.

The inventors unexpectedly discovered in experiments using tumor-bearing nude mice that inactivated probiotics, which are usually used as immune enhancers, can actually produce a significant local chemical effect, and potentially even a chemical ablation-like effect under certain specific conditions. The specific conditions are not the application conditions of the probiotic component in prior art, but are as defined below.

An inventive object of the present application is to provide a use of a probiotic component in the manufacture of a local pharmaceutical composition for the treatment of a local lesion disease, and said probiotic component can be used as an active ingredient having a therapeutic effect. The relevant terms used in the present application are described below.

The term "probiotics" refers to live, non-pathogenic bacteria and fungus capable of producing beneficial effects on the health of a host. The term "probiotic component" refers to a preparation (e.g., a cell wall polysaccharide) derived from natural probiotics or engineered probiotics, or an analog of such a preparation. The term "analog" refers to a similar substance with similar activity to the preparation, comprising a derivative of the preparation (e.g., a derivative of a cell wall polysaccharide, which is capable of improving water solubility or activity), a synthetic product (e.g., a synthetic polysaccharide similar to a cell wall polysaccharide), other source preparations similar to the probiotic preparation (e.g., another source polysaccharide similar to a cell wall polysaccharide).

The term "medicament" or "pharmaceutical composition" refers to a substance that comprises an active ingredient and exhibits pharmaceutical characteristics such as pharmacological method and pharmacological constitutions necessary to achieve its pharmacology in the patient. The term "active ingredient" refers to a substance that can perform a certain specific pharmacology under specific conditions. The term "pharmacological method" refers to an administration route necessary to achieve a certain specific pharmacology, e.g., the pharmacological method for improving the intestinal barrier by probiotics is oral administration. The term "pharmacological constitutions" refers to the components constituting a pharmaceutical composition necessary to achieve a certain specific pharmacology, in particular a pharmaceutical composition entering into a target region. Different pharmacological effects usually require different preferred pharmacological constitutions (active ingredients, active dynamic conditions, dosage form conditions, etc.). The term "active dynamic conditions" refers to the quantitative conditions that must be met in order for an achievement of a certain specific pharmacology by an active ingredient, and it is possible that different dynamic conditions may need to be met for the same substance used as different active ingredients. The term "dosage form conditions" refers to the environmental conditions of pharmacological reactions that must be met in order for achievement of a specific pharmacology by an active ingredient, and it is possible that different dosage form conditions may need to be met for the same substance used as different active ingredients. For example, in the case of a pharmaceutical composition comprising probiotics and components thereof, when used as a systemic-acting active ingredient in the prior art, it enters the reactor in a target region as blood carrying a drug (containing many substances), so a conventional dosage form with an open-ended constitution (e.g., a flavouring agent in an oral dosage form, an osmotic regulator in a conventional injectable dosage form, etc.) can be employed; when used as a local active ingredient in the present invention, it is highly sensitive to the reaction environment, and certain commonly used additives (e.g., sodium chloride, often used as an osmotic regulator in the following examples) may have a negative effect on the local activity of a probiotic component, and therefore a pharmacological dosage form with a close-ended constitution (preferably comprising only an active ingredient and a solvent) must be employed to minimize exogenous interference.

The term "a therapeutic drug" is distinguished from "an adjunctive drug (for therapy)", the former refers to a drug that provides a therapeutic effect, and the latter refers to a drug that provides an adjunctive effect (for therapy). The term "therapeutic effect" is distinguished from an adjunctive effect (for therapy), wherein the former is a primary pharmacological effect that results in effective remission, improvement or cure of a disease (e.g., local treatment of a local lesion or/and immunotherapy), while the latter is a secondary pharmacological effect that does not result in effective remission, improvement or cure of a disease but is nevertheless beneficial to a patient (e.g., immune enhancement of an organism). A therapeutic effect usually includes a significant effect when used alone, or a primary or equivalent effect when used in combination with other drugs or in combination with other treatments to produce a significant efficacy, while an adjunctive effect (for therapy) usually provides only an advantageous but unobvious effect when used alone, or an advantageous but non-primary or not equivalent effect when used in combination with other drugs or in combination with other treatments to produce a significant efficacy. The term "significant efficacy " refers to effective inhibition of local lesion growth or progression compared to classical chemotherapeutic agents or classical chemical ablation agents, for example, a clinical pathologic efficacy rate (PR+CR) of $\geqq$ 30 %for local lesions, or a non-negligible pharmacologic significance of local lesion growth inhibition in animal experiments, such as tumor proliferation rate $\leqq$ 85% or tumor inhibition rate $\geqq$ 15%, preferably, tumor proliferation rate $\leqq$ 60% or tumor inhibition rate = 40%. The same substance used as different active ingredients may then provide completely different effects. For example, in the case of a pharmaceutical composition comprising probiotics and components thereof, when used as a systemic-acting active ingredient in the prior art, it provides mainly adjunctive effects, such as immune enhancement effects, which are usually not sufficient to observe a tumor inhibition effect; when used as a local active ingredient in the present invention, it provides mainly a therapeutic effect.

The term "local medicine" or "medicine for local administration" is distinguished from "conventional medicine" or "medicine administered conventionally ", and the former refers to a drug based primarily on local pharmacology, while the latter refers to a drug based on systemic pharmacology. Differences between them are: the former is usually a therapeutic drug, while the latter can be a therapeutic drug or an adjunctive drug (for therapy); their effects on indications may be completely different, wherein the former's pharmacological method is local administration, especially administration to a local lesion, while the latter is mainly administered conventionally (systemic administration), which may be completely different for the attentions of the side effects of the administrations; the pharmacological active ingredient of the former must provide local effect, while the pharmacological active ingredient of the latter must provide systemic effect, and their preferred directions may be completely opposite; an essential factor in the pharmacodynamics of the former is the concentration of administration, while the latter is concerned with the dosage of administration, and the meanings of their drug concentrations may be completely different; the pharmacological dosage form of the former is a dosage form for a local administration, while the pharmacological dosage form of the latter is a dosage form for a conventional administration, and the constitutions of their pharmacological dosage forms may have completely different requirements.

The term "local administration" is distinguished from conventional administration (systemic administration) and refers to any means of administration for the purpose of producing a local effect, including intra-lesional administration or/and extra-lesional beneficial local intra-administration capable of producing local therapeutic effect (e.g. subcutaneous injection which can produce a local effect and facilitate a secondary immunological effect from the local effect), while conventional administration means any means of administration for the purpose of producing systemic effects, including administration via the digestive tract (e.g., oral) or vascular (e.g., intravenous injection, intraperitoneal injection), to deliver to a target region via blood. The term "intra-lesional administration" refers to any administration means that allows the drug to enter the local lesion directly rather than indirectly in a way of drug-carrying blood, such as intratumoral administration, implantation within local lesion, daubing on a local lesion, spraying on a local lesion, subcutaneous or transvascular injection within a local lesion, insertion into a local lesion, and the like.

The term "local treatment" differs from "systemic treatment" in that the former refers to treatment that primarily uses the local pharmacology (local activity) of the drug itself to target the local region where the patient's local lesion is located (e.g., administration to a local lesion and other lesion regions connected with the local lesion), while the latter refers to treatment that primarily uses the systemic pharmacology (conventional activity) of drug carried by blood to target the patient's systemic lesion (e.g., a tumor body, a region connected with the tumor body; tumor cells contained in other parts of the body). In the prior art, the primary function of the probiotic component used in the treatment of local lesions is based on an adjunctive effect on a therapy (e.g., immune enhancement activity) as an xenoantigen, rather than a therapeutic, much less a local therapeutic effect.

The term "local activity" is distinguished from conventional activity, wherein the former can provide a local effect (a local synergy) or/and a secondary effect (a medium- and long-term synergy) associated with the local effect (the local synergy), whereas conventional activity usually provides only systemic effects (e.g. cytotoxic effects, immune enhancement effects), they are two completely different pharmacological activities. The term "local effect" is distinct from conventional effect and refers to the pharmacological effect produced by a drug after local administration within the local area of tissue interstitial penetration (e.g., tumor or/and extra-tumoral local region), usually including local chemical effect, whereas conventional effect refers to the pharmacological effect produced by a drug after conventional administration via the digestive tract or blood vessels and delivery in a way of drug carrying blood to a target region. The term "local chemical effect" refers to a local effect that includes a chemical action. The term "local synergy" refers to a local effect that includes a synergy. The term "local chemical synergy" refers to a local chemical effect that includes a synergy. Through a secondary effect (e.g., a secondary cross-distance effect), a local activity can sometimes produce a systemic effect.

The local chemical effect (or local chemical synergy) described in the present application pharmacologically comprises common local chemical effect, chemical ablation and chemical ablation-like. The term "common local chemical effect" refers to local chemical effect where the drug effect does not exceed the maximum expected difference in dynamics (e.g., within 200%) of the conventional chemical effect of the same drug, such as chemotherapeutic effects resulting from conventional administration of cytotoxic drugs. The term "chemical ablation" refers to a local chemical effect that exceeds the maximum expected difference in dynamics (e.g., greater than 200%, preferably greater than 400%) of the conventional chemical effect of the same drug (e.g., the chemotherapeutic effect produced by the conventional administration of high concentrations of ethanol), usually referring to the local chemical effect of a classical chemical ablation agent (e.g., high concentrations of ethanol, high concentrations of acids, high concentrations of bases). The term "chemical ablation-like" refers to a local effect of a drug that is similar to chemical ablation (surpassing expectation of a conventional effect). Although not caused by a classical chemical ablation agent, chemical ablative-like effects are pharmacologically distinct from common local chemical effects.

The term "secondary effect" is distinguished from the term "direct effect", wherein the former refers to an effect related to but not identical to the latter, and occurring at a later time, while the latter refers to an effect of a drug which reacts directly with the target after entering the target region (e.g., the local effect occurs upon a local medicine administered conventionally entering a local lesion and interacting with the lesion tissue).

The term "immunotherapy" is distinguished from the term "immune enhancement", wherein the former refers to immunological effects that are therapeutic when used alone (e.g., immunological effects of therapeutic vaccines, specific antibodies, etc.), while the latter refers to immunological effects that are not therapeutic when used alone but still have an adjunctive effect (e.g., immune enhancement agents have the effect of enhancing immune function of an organism). The term "secondary immunological effect" is distinguished from the term "antigenic effect of a drug ", wherein the former refers to immunological effects associated with drug administration but distinct from the antigenic effects of the drug, such as in situ vaccination due to local chemical effects of the drug), while the latter refers to the antigenic effect of the drug itself (e.g., the antigenic effect of any drug entering the body as a foreign substance). The term "*in situ* secondary immunity" is distinguished from the term "*non-in situ* secondary immunity", wherein the former refers to secondary immunity within a lesion (e.g., a tumor body), while the latter refers to a secondary immunity outside a lesion (e.g., in the sub-axillary intradermal tissue). The term "secondary immune substance" refers to an immune substance that is formed in the administration region as a result of local administration, and is distinct from the administered drug itself, such as antigens, adjuvants, or/and other immune molecules that are released, generated, activated, or/and recruited for any reason after drug administration. The term "*in situ* secondary immune substance" is distinguished from the term "*non-in situ* secondary immune substance", wherein the former refers to secondary immune substance within the lesion (e.g., within the tumor) (e.g., in situ antigens, in situ adjuvants, or/and other immune molecules released, generated, activated, or/and recruited within the lesion), while the latter refers to a secondary immune substance outside the lesion (e.g., at the sub-axillary intradermal tissue) (e.g., nodular immune substances formed at the administration site, other immune molecules that are released, generated, activated, or/and recruited as a result of the other drug effects at the nodule or/and the administration site, etc.).

The term "vaccine antigen" is distinguished from the term "antigen", wherein the former refers to an antigen capable of inducing a therapeutic effect in an organism against a specific disease, e.g. the same substance can be used as a vaccine antigen and an immune enhancer antigen respectively through very different technical solutions, while the latter refers to any substance capable of inducing an immune response in an organism. The term "adjuvant" refers to a substance that can enhance the immunotherapeutic effect of an antigen in a vaccine. The term "*in situ* antigen" refers to an in situ secondary immune substance that can be used as an antigen. The term "vaccine-like drugs" or "vaccine-like" refers to a therapeutic drug (different from an immune enhancer and a conventional vaccine) that can provide a secondary immunological effect similar to the effect of a vaccine and optionally the presence of an exogenous antigenic effect.

In the scope of the present invention, said probiotic component is administered locally together with a chemical active ingredient and/or a biologically active ingredient capable of producing a synergy with the probiotic component. In one embodiment, said probiotic component and said co-administration active ingredient are included in a same medicament.

The term "synergy" refers to such a combined effect (e.g., a short-term and/or medium- to long-term efficacy) of a specific active ingredient (e.g., the probiotic component of the present application) with another active ingredient (e.g., a weak local acting compound and/or a cytotoxic drug) under specific conditions (e.g., a pharmacological component preferably providing minimized bacterial immunity and a pharmacological concentration preferably providing minimized local effect) that exceeds the sum of the respective effects expected from their individual administrations. The term "local synergy" refers to a local effect comprising a synergy. The term "local chemical synergy" refers to a local chemical effect comprising a synergy.

The term "amount ratio" refers to the weight ratio of multiple active ingredients (e.g., the probiotic component and co-administration substances thereof in the present application) in the same composition. The term "concentration ratio" refers to the concentration ratio of multiple active ingredients (e.g., the probiotic component and co-administration substances thereof in the present application) in the same composition.

Suitable patients for said treatment are selected from a group comprising one or more of immunosuppressed patients (e.g., immunocompromised patients, elderly patients), patients who can be administered locally within the lesion, patients whose local lesion tissue is amenable to a chemical ablation-like treatment, patients who can produce a secondary immune substance within the local lesion, and patients who can produce a secondary immune substance within the administration region outside a lesion.

Said "immunosuppressed patient" refers to any patient who can be represented by a tumor-bearing nude mouse model, such as a patient whose immune function is at low level for any reason and cannot reach normal levels using other methods (e.g., immune enhancing method) within a period of time for which the compositions in the technical embodiments of the present invention can provide a local effect (e.g., within 7 days after the first dosing in Example 2 below), , such as those who have difficulty with radiotherapy or conventional chemotherapy due to low immunity.

Said therapeutic effect comprises local treatment involving said local effect (or local synergy) or/and immunotherapy. The immunotherapy comprises a secondary immunological effect of said local effect (or local synergy) and optionally other immunological effects within or/and outside said lesion; and the local treatment comprises a chemical ablation-like and optionally other chemotherapies of one or more local lesions. Preferably, the pharmaceutical composition is a chemical ablation-like in situ and non-in situ immunotherapeutic agent, and the patient is selected from one or more of patients who can be administered within a local lesion and the local lesion tissue can be subject to chemical ablation-like and/or secondary immunological effects can be produced within the local lesion, patients who can produce secondary immunological effects within the administration region outside the lesion. The pharmaceutical composition is a chemical ablation-like drug or an *in situ* immunotherapeutic drug, and the *in situ* immunotherapeutic drug may be an immunotherapeutic drug providing *in situ* vaccine activation and optionally other immunological effects in the target region within the lesion; and the pharmaceutical composition may also be an immunotherapeutic drug providing secondary immunological effects outside the lesion and optionally other immunological effects, such as a vaccine-like drug. The secondary immunological effect outside the lesion includes immunological effect from non-normal structures (e.g. nodules) secondary to the local effect (or local synergy).

The probiotic component may provide at least one of a local effect, a secondary effect of a local effect, and an immunological effect of a local administration, wherein the secondary effect of a local effect comprises an immunological effect involving secondary immune substances in the drug administration region.

The probiotic component is selected from one or more of a probiotic water-soluble component, a probiotic semi-fluid-like component, a probiotic component water-insoluble particle, an inactivated probiotic, and has low bacterial immunogenicity. As an example of the present invention, said probiotic component is selected from a probiotic water-soluble component and/or engineered analogues thereof, and said composition is a solution composition. In one embodiment, said probiotic component is selected from probiotic component water-insoluble particles, or/and inactivated probiotics and said composition is a suspension composition. In one embodiment, said probiotic component is selected from a probiotic semi-fluid-like component, and said composition is a semi-fluid-like composition. In one embodiment, said composition is preferably a semi-fluid-like composition or an aqueous solution composition, more preferably a semi-fluid-like composition.

"Bacterial immunogenicity" refers to the ability of a bacterium as an intact foreign object to produce an immune response in a recipient, and different bacteria have different bacterial immunogenicity. Live probiotics have the strongest bacterial immunogenicity, but also have a strong safety risk upon entering an organism directly. The "inactivated probiotics" refers to the preparation obtained after inactivation treatment such as high temperature inactivation, high temperature and high pressure inactivation, ultraviolet inactivation, chemical reagent inactivation, radiation inactivation, etc. The "probiotic water-insoluble component" refers to any component derived from probiotics with a water solubility of < 0.1%, and the "probiotic water-soluble component" refers to any component derived from probiotics with a water solubility of $\geq$ 0.1%, and the "disrupted probiotics" refers to a mixture obtained from probiotics upon a disrupting procedure, and the "disrupted probiotic precipitation component" refers to a water-insoluble precipitate and its further isolated component and its analogues, isolated from the disrupted probiotics (e.g. by filtration or/and centrifugation). The term "supernatant component of disrupted probiotics" refers to a supernatant and its further isolated component and its analogues, isolated from the disrupted probiotics (e.g. by filtration or/and centrifugation). The term "probiotic cell wall polysaccharide" refers to the polysaccharide comprised in the cell wall and its analogues, such as $\beta$-glucan. The term "probiotic semi-fluid-like component" refers to the probiotic component that provides a semi-fluid-like form for the composition of the present invention.

The term "semi-fluid-like" refers to a type of physical form between liquids and semi-solids, which includes semi-fluids and their analogs. The term "semi-fluid" refers to an object that does not flow visually without external pressure within the lower time limit (e.g., 20 seconds) at room temperature, but can flow and cause irreversible deformation under clinically acceptable external pressure (at the time of administration) (e.g., external pressure that can be applied to a syringe propulsion device), as distinguished from liquids (which also flow without external pressure) and semi-solids (which undergo only reversible deformation under clinically acceptable external pressure). The term "semi-fluid analog" refers to a physical form between a liquid (suspension) and a semi-fluid, which is close to a semi-fluid and not stratified, while the suspension is significantly stratified, after placing at room temperature for about 1 minute; which exhibits flow visible to the naked eye after placing at room temperature without external pressure for about 1 minute, while the semi-fluid does not so. It is possible to transform a suspension into a semi-fluid-like object, such as 5% $\beta$-glucan is mixed with water into a suspension, and the 5% $\beta$-glucan aqueous suspension can be heated and prepared into a semi-fluid analog, while 5% $\beta$-glucan/1% methylene blue/1% 5-fluorouracil aqueous suspension can be heated and prepared into a semi-fluid .

The probiotic water-soluble component is selected from at least one of a supernatant component from disrupted probiotics, a probiotic extract, and a probiotic intracellular water-soluble component. The probiotic semi-fluid-like component is selected from one or more of the probiotic components whose aqueous mixture can be formed (e.g., by heating followed by cooling, alkalinization, etc.) into a semi-fluid-like composition, such as polysaccharides and analogs thereof.

The probiotic water-insoluble particles are selected from at least one of disrupted probiotic precipitation component, probiotic cell wall polysaccharide particles, probiotic cell wall polysaccharide nanoparticles. The inactivated probiotics are not limited to inactivated probiotics that are all intact, for example, the amount ratio of the non-intact probiotics in the inactivated probiotics > 20% or > 30%, or the number of intact probiotics < $10^5$ per ml or < $0.5 \times 10^5$ per ml.

The probiotic bacterium is selected from at least one of Bacillus, lactic acid bacteria, Bifidobacterium, and Fungus, which may be natural-occurring or engineered. The Bacillus is one or more of Bacillus cereus, Bacillus licheniformis, Bacillus subtilis, Bacillus megaterium, Bacillus firmus, Bacillus coagulans, Bacillus lentus, Bacillus pumilus, Bacillus natto. The lactic acid bacteria are Lactobacillus and/or Bifidobacterium, wherein said Lactobacillus is selected from at least one of Lactobacillus acidophilus, Lactobacillus salivarius, Lactobacillus casei, Lactobacillus plantarum, Lactobacillus pumilus and Lactobacillus fermentum. The Bifidobacteria include at least one of Bifidobacterium longum, Bifidobacterium adolescentis, Bifidobacterium breve, Bifidobacterium infantis, Lactobacillus bruce, Lactobacillus helveticus, Lactobacillus thermophilus, Enterococcus faecium, and Streptococcus fecalis. The fungi include yeasts and/or Brettanomyces bruxellensis (Saccharomyces boulardii), wherein said yeasts include at least one of Saccharomyces cerevisiae, Torulaspora delbrueckii, Candida, Wickerhamomyces, Pichia, Torulopsis candida, Saccharomyces chevalieri, Rhodotorula rubra, Schizosaccharomyces, Saccharomyces boulardii, Candida utilis.

The pharmacological constitution required for said probiotic component to provide therapeutic effect as above described satisfies the pharmacological concentration (local administration concentration) of >0.1%, $\geq$0.25%, 0.25-25%, preferably 0.5-15%, more preferably 1-15% or 5-15%, which is required for said probiotic component to provide said therapeutic effect.

The term "concentration" is the weight-to-volume percentage concentration (w/v) of a specified component in a drug. The term "pharmacological concentration" refers to a concentration of a specified component in the target region, such as the initial concentration at intra-lesion, necessary to achieve its specific pharmacological response. The term "formulation concentration" refers to a concentration of a specified component in a pharmaceutical formulation form (e.g., an injection formulation or a perfusion liquid). The term "administration concentration" refers to a concentration of the specified component in the delivery form of the pharmaceutical formulation (e.g., dilution of the formulation). The term "initial concentration at intra-lesional" refers to a concentration of the specified component in the drug-containing medium (e.g., drug-containing blood) at the time the drug enters the lesion. Even if the probiotic component of the composition of the present invention is administered at the same concentration as that of the probiotic component in a conventional injection formulation, the intratumoral initial concentration necessary for their respective pharmacology effects (local chemical effect vs. immune enhancement effect) can be quite different. One of the features of the technical solutions of the uses, compositions and methods of the present invention is to guarantee the pharmacological concentration (local administration concentration) necessary for the described effect, especially for the local chemical effect.

The initial concentration at intra-lesional necessary for their respective pharmacology effects (local chemical effect vs. immune enhancement effect) can also be quite different even if the administration concentration of the probiotic component in the composition described in the present application is the same as the administration concentration of the probiotic component in a conventional injection formulation. And one of the features of the technical solutions of the uses, compositions and methods of the present invention is to guarantee the initial local concentration (local administration concentration) necessary for the described effect, especially for the local chemical effect. The local administration concentration of the probiotic component in the pharmaceutical composition of the present application is usually the concentration of the probiotic component in the drug at the end point (e.g. needle hole, catheter outlet, etc.) of the drug delivery device such as a syringe, puncture device, perfusion catheter, etc. For powder formulation for injection, said administration concentration is the concentration of the probiotic component after mixing the dry powder and the liquid vehicle.

The term "target" refers to prime objective of pharmacology, such as cytotoxic drugs targeting tumor cells, immunomodulatory drugs targeting regulators of the immune system, and chemical ablation agents targeting tumor tissues. The term "target region" refers to the spatial area where the drug is administered to the target, such as the tumor or a portion thereof. For example, when the tumor is small in diameter and the required single administration dosage is clinically feasible, the target region is one tumor body targeted for this treatment; or when the tumor body is large in diameter and the required single administration dosage is not clinically feasible, the target region is a portion of the tumor targeted for this treatment.

When used to treat a patient, the components or unit components of the composition must meet the conditions of the pharmacological volume required to enable the probiotic component to provide said effect, wherein said pharmacological volume (local administration volume) is $\geq$ 1 ml, or 10-150 ml for local intra- lesion administration or/and 1.5-50 ml for local extra-lesion administration. As one of the features of the technical solutions of the uses, compositions and methods of the present invention, the extra conventional administration volume is particularly required for the local effect of said probiotic component.

The components of said composition requires a minimization of components other than synergists, so as to meet the local pharmacological environmental conditions required to provide said local effect, preferably, the components of said composition do not comprise inactive ingredients required for pharmaceutics and/or administration safety in conventional compositions, such as solid excipients, flavoring agents in oral formulations and osmolarity increasing agents in conventional injection formulations.

In one embodiment, the carrier of said pharmaceutical composition is water, the co-administration substance is water-soluble, and said probiotic component is a probiotic water-soluble component, or an inactivated probiotic, a water-insoluble particle of a probiotic component, one or more of probiotic semi-fluid-like component or a water-insoluble component of a derivative thereof. In one embodiment, the carrier of said pharmaceutical composition is water, said co-administration substances and/or are insoluble in water, and said probiotic component is a probiotic semi-fluid-like component.

Preferably, the chemical active ingredient described in the present application is selected from a weak local acting compound and/or a cytotoxic drug, for example, for example, said co-administration substances are at least two chemical active ingredients. If said chemical active ingredient is a cytotoxic drug, the amount ratio of said probiotic component to the cytotoxic drug, ($W_{probiotic\ component}/W_{cytotoxic\ drug}$), is (1-110)/(1-100), and if said chemical active compound is a weak local acting compound, the amount ratio of said probiotic component to the weak local acting compound, ($W_{probiotic\ component}/W_{weak\ local\ acting\ compound}$), is (1-90)/(1-100).

The term "chemical active ingredient" refers to any active ingredient that provides a chemical effect, including cytotoxic drugs and local chemical ingredients. The term "local chemical ingredient" refers to any active ingredient that can provide a local chemical effect when administered locally. The term "weak local acting compound" refers to a chemical active ingredient that is less locally effective than a classical chemical ablation agent under specific conditions (e.g., under the concentrations when used as a co-administration substance). The weak local acting compound comprises: amino acid nutrients, vital dyes, quinine drugs, low concentration acidifiers, low concentration basifiers, pH buffering systems comprising acidifiers or/and basifiers. The co-administration substance may be a vital dye and at least one other chemical active ingredient, or a vital dye and a cytotoxic drug, or a vital dye and an amino acid nutrient, or a cytotoxic drug and at least one other chemical active ingredient.

If said weak local acting compound is an amino acid nutrient, then the amount ratio of said probiotic component to the amino acid nutrient, ($W_{probiotic\ component}/W_{amino\ acid\ nutrient}$) is (1-20)/(1-100); if said weak local acting compound is a vital dye, then the amount ratio of said probiotic component to the vital dye, ($W_{probiotic\ component}/W_{vital\ dye}$) is (7-90)/(1-100); if said weak local acting compound is quinine drugs, then the amount ratio of said probiotic component to the quinine drugs, ($W_{probiotic\ component}/W_{quinine\ drugs}$) is (2-90)/(1-100); if said weak local acting compound is an acidifier or/and basifier, then the amount ratio of said probiotic component to the acidifier or/and basifier, ($W_{probiotic\ component}/W_{acidifier\ or/and\ basifier}$) is (2-60)/(1-100).

The term "amino acid nutrients" refers to amino acid compounds having nutritional health effects, preferably amino acid nutritional agents or amino acid auxiliary materials with nutritional health effects, as described in official pharmacopoeias or guidelines. The amino acid nutrients include amino acids, amino acid salts, oligopeptides and polypeptides. Preferably, said amino acid nutrients comprise amino acids or salts thereof selected from a group comprising, or oligopeptides and polypeptides consisting of alanine, valine, leucine, isoleucine, phenylalanine, proline, tryptophan, tyrosine, serine, cysteine, methionine, threonine, lysine, arginine, histidine, aspartic acid, glutamic acid, $\beta$-alanine, taurine, gamma amino butyric acid (GABA), theanine, citrulline, ornithine. Preferably, said amino acid nutrients comprise amino acids or salts thereof, or oligopeptides and polypeptides comprising or consisting of arginine, lysine, glycine, cysteine, alanine, serine, aspartic acid, glutamic acid. In one embodiment, the concentration of said amino acid nutrient is >2.5%, or 5-30%, preferably 5-25%.

The term "vital dye" refers to aromatic compound dyes that are capable of dyeing tissues, cells, subcellular units, and the like after entry into living animal tissues, but are not unacceptably harmful to the animal as a whole. The vital dye comprises at least one of methylene blue, patent blue, isosulphur blue, Bengal red, toluidine blue, trypan blue, alkaline blue, eosin, alkaline fuchsin, crystal violet, gentian violet, neutral red, Janus green B, Safranine. Preferably, said vital dye is methylene blue type dye such as methylene blue, patent blue, isosulphur blue, neo-methylene blue. In one embodiment, said vital dye is at a concentration of ≥ 0.25%, or 0.25-10%, preferably 0.25-1.5% or 2.5%-10%. Preferably, said vital dye is a methylene blue type dye at a concentration (w/v) of ≥ 0.35%, preferably 0.35-2.5%, more preferably 0.35-1.5% or 0.5-1%. In one embodiment, said vital dye is Bengal red at a concentration (w/v) of 1-10%.

The quinine compounds comprise at least one of quinine, quinine monohydrochloride, quinine dihydrochloride at a concentration of ≥ 0.5%, or 0.5-5%, preferably 1.5-5% or 1.5%-3%. The term "acidifier" refers to an acid used primarily as an auxiliary materials in the preparation of a drug, more specifically for pH adjustment, which provides acidity and normally does not introduce specific biological activity when used; preferably a strong and/or weak acid approved by the official administrative authority of each country, or comprised in the official pharmacopoeia or guidelines of each country. The strong acid may be hydrochloric acid, sulfuric acid, nitric acid, perchloric acid, selenic acid, hydrobromic acid, hydroiodic acid, chloric acid, etc., preferably hydrochloric acid. The weak acid may be carbonic acid, boric acid, acetic acid, phosphoric acid, sulfurous acid, pyruvic acid, oxalic acid, tartaric acid, nitrous acid, etc., preferably acetic acid. In an embodiment, the amount ratio of said probiotic component to the acidifier, that is, weight concentration ($W_{probiotic\ component}/W_{acidifier}$) is (1-20)/(0.5-50). In one embodiment, the concentration of said acidifier is ≥ 0.5%, 0.5-2% (strong acid) or 2-15% (weak acid).

In one embodiment, the amount ratio of said probiotic component to the basifier (weight concentration of the probiotic component/weight concentration of basifier) is (1-20)/(0.5-50). In one embodiment, the concentration of said basifier is ≥ 0.5%, 0.5-5% (strong alkali), or/and 2-15% (weak alkali). The term "basifier" refers to an alkaline compound used primarily as an auxiliary materials in a drug preparation, more specifically for pH adjustment; preferably selected from basifiers approved by the official administrative authority of each country, or comprised in the official pharmacopoeia or guidelines of each country, including strong and weak alkalis. The strong alkalis are alkali metal hydroxides or/and organic strong alkalis, which has a concentration (w/v) of ≥ 0.5%, preferably 0.5-7.5% or 0.75-7.5% in the pharmaceutical composition. Preferably, said strong alkali is an alkali metal hydroxide such as sodium hydroxide, potassium hydroxide, calcium hydroxide, more preferably sodium hydroxide. The weak alkali comprises acid inorganic salt of polybasic weak acid, alkali inorganic salt of polybasic weak acid, a nitrogen-comprising weak alkali, wherein the acid inorganic salt of polybasic weak acid comprises sodium dihydrogen phosphate, disodium hydrogen phosphate, sodium bicarbonate, potassium bicarbonate, calcium bicarbonate, sodium hydrosulfate, preferably sodium bicarbonate. In one embodiment, the concentration (w/v) of said acid inorganic salt of polybasic weak acid in said pharmaceutical composition is ≥ 1%, preferably 2-10% or 3-10%.

The alkali inorganic salt of polybasic weak acid comprises sodium phosphate, sodium carbonate, potassium carbonate, borax, preferably sodium carbonate; and the concentration (w/v) of said alkali inorganic salt of polybasic weak acid in said pharmaceutical composition is ≥ 1%, preferably 2-10% or 3-10%. The nitrogen-comprising weak alkali comprises ammonia, ammonia chloride, 2-aminoethanol, tristearin, triethanolamine, trihydroxymethyl aminomethane, 2-aminoethanol, tristearin, triethanolamine, meglumine, N-ethyl glucamine; and the concentration (w/v) of nitrogen-comprising weak alkali in said pharmaceutical composition is ≥ 2%, preferably 2-35% or 3-35%. The basifier may be sodium hydroxide and sodium bicarbonate, and the concentrations (w/v) of the two compounds in said pharmaceutical composition are 2-5%, 3-10%, respectively. The basifier may also be sodium carbonate and sodium bicarbonate, and the concentrations (w/v) of the two compounds in said pharmaceutical composition is 3-10%, 3-10%, respectively. Preferably, said pharmaceutical composition comprises an acidifier or/and a basifier and has a pH buffering capacity.

The chemical active ingredient comprises at least one cytotoxic drug, and said cytotoxic drug comprises an active ingredient of the antitumor drug; wherein the concentration of the active ingredient of the antitumor drug is ≥ 0.1%, 0.1-15%.

The term "cytotoxic drug" refers to an active ingredient that primarily targets diseased cells or intracellular structures of diseased cells to achieve its drug effect, such as a conventional chemotherapeutic drug. The term "conventional chemotherapeutic drug" refers to a drug that can be used to treat local lesion disease effectively by conventional administration at a safe dose, preferably a conventional chemotherapeutic drug (e.g., an antineoplastic drug) that has been or will be approved by an official administrative authority in each country (e.g., FDA or the Chinese Pharmacopoeia), or is or will be included in an official pharmacopoeia in each country.

An active ingredient of an antitumor drug can be selected from: 1) drugs that disrupt DNA structure and function, such as a) alkylating agents such as cyclophosphamide, carmustine, etc., b) metal platinum complexes such as cisplatin, carboplatin, etc., c) DNA topoisomerase inhibitors such as doxorubicin drugs, topotecan, irinotecan, etc., 2), drugs that are intercalated into DNA to interfere with RNA transcription, such as antitumor antibiotics such as actinomycins, erythromycin, doxorubicin, etc.; 3) drugs that interfere with DNA synthesis, comprising a) pyrimidine antagonists, such as 5-fluorouracil, furofluorouracil, difurofluorouracil, cytosine derivatives such as cytosine arabinoside, cyclic cytidine, 5-azacytidine and other uracil derivatives, b) purine antagonists such as lysolecithin, thioguanine, c) folic acid antagonists such as methotrexate and the like; 4) drugs that affect protein synthesis, such as colchicines, vincristines, and taxanes such as paclitaxel, doxorubicin, and the like.

The active ingredient of an antitumor drug comprises at least one of uracil derivatives, cyclophosphamides, gemcitabines, epirubicins, antitumor antibiotics, teniposide, metal platinum complexes, taxanes; preferably, the active ingredient of an antitumor drug is selected from at least one of 5-fluorouracil, cyclophosphamide, gemcitabine, epirubicin, antitumor antibiotics, teniposide, metal platinum complexes, paclitaxel.

The active ingredient of an antitumor drug can be an alkylating agent such as cyclophosphamide, carmustine, etc., and the concentration (w/v) thereof in said pharmaceutical composition is 0.5-6%, preferably 0.75-1.5%; or a metal platinum complex such as cisplatin, carboplatin, etc., and the concentration (w/v) thereof in said pharmaceutical composition is 0.03-0.15%, preferably 0.05-0.15%; or DNA topoisomerase inhibitors such as doxorubicin, topotecan, irinotecan, etc., and the concentration (w/v) thereof in said pharmaceutical composition is 0.05-0.20%, preferably 0.075-0.15%; or an antitumor antibiotic such as actinomycin, erythromycin, etc., and the concentration (w/v) thereof in said pharmaceutical composition is 1-4%, preferably 1-2%; or uracil derivatives such as 5-fluorouracil, furofluorouracil, difurofluorouracil, cytosine derivatives such as cytosine arabinoside, cyclic cytidine, 5-azacytidine, and other pyrimidine antagonists, and the concentration (w/v) thereof in said pharmaceutical composition is 0.5-2%, preferably 0.75-1.5%.

Preferably, said pharmaceutical composition comprises a biologically active ingredient, and said biologically active ingredient comprises at least one of an antigen, an immunomodulatory antibodies, a cytokine, an adjuvant. The antigen is a microbial antigen or a tumor antigen, wherein said microbial antigen comprises 1) non-probiotic bacteria, such as Streptococcus pyogenes, Serratia heteroplastica, BCG, Clostridium tetani, Clostridium butyricum, Lactobacillus acidophilus; 2) viruses such as hepatitis B virus, adenovirus, Scarrash virus simplex, vaccinia virus, mumps virus, Newcastle fever virus, poliovirus, measles virus, West Nica Valley virus, Coxsackie virus, reovirus and other viruses; 3) parasites such as Plasmodium etc.; said tumor antigen is selected from at least one of breast cancer, pancreatic cancer, thyroid cancer, nasopharyngeal cancer, prostate cancer, liver cancer, lung cancer, intestinal cancer, oral cancer, stomach cancer, colorectal cancer, bronchial cancer, laryngeal cancer, testicular cancer, vaginal cancer, uterine cancer, ovarian cancer, malignant melanoma, brain tumor, renal cell carcinoma, astrocytoma, glioblastoma; said immunomodulatory class of antibodies comprising 1) blocking antibodies against inhibitory receptors, such as blocking antibodies against CTLA-4 molecule and PD-1 molecules; 2) blocking antibodies against ligands of inhibitory receptors, activating antibodies against immune response cell surface stimulating molecules, such as anti-OX40 antibodies, anti-CD137 antibodies, anti-4-1BB antibodies; 3) neutralizing antibodies against immunosuppressive molecules in the microenvironment of local lesion diseases, such as anti-TGF-p1 antibodies. The cytokine comprises tumor necrosis factor, interferon, and interleukins.

Preferably, the dosage form of the pharmaceutical composition described in the present application is a local dosage form, and said pharmaceutical composition may be any local dosage form comprising a probiotic component and meeting the conditions necessary to enable said probiotic component to provide the desired effect, such as an injection formulation, a daubing formulation or a cream,.

The term "injection formulation" refers to a sterile preparation comprising active ingredients and liquid carriers for in vivo administration, which are divided into local injection formulation, intravenous injection formulation, etc. according to mode of administration, and a local injection formulation can be used as a local injection formulation only after the concentration of local administration is given; according to the commercial form, a local injection formulation is divided into a liquid injection agent, a semi-fluid injection agent, a powder for injection. The powder for injection comprises sterile dry powder and solvents, and the sterile dry powder comprises part or all of active ingredient, and the solvents comprise all liquid carriers. The concentration of said active ingredient in the injection formulation is the concentration of active ingredient in a mixture of the active ingredient and all of said liquid carrier, typically the concentration of active ingredient in the liquid drug at the end point (e.g. needle hole, catheter outlet, etc.) of the local adminstration device such as a syringe, puncture device, injection catheter, etc. For the powder for injection, the concentration of said active ingredient is the concentration of the active ingredient in the mixture of the dry powder and the solvent (e.g., reconstitution solution or said pharmaceutically acceptable liquid carrier).

Preferably, the pharmaceutical composition described in the present application further comprises excipients such as dispersing medium, preservative, stabilizer, wetting agent and/or emulsifier, solubilizer, viscosifier, etc., wherein said viscosifier may be sodium carboxymethyl cellulose, carboxymethyl cellulose, Polyvinylpyrrolidone or gelatin, and said preservative may be an antioxidant such as ascorbic acid. Preferably, the pharmaceutical composition described in the present application further comprises a tracer such as iodinated oil.

The present application also provides a pharmaceutical kit comprising one or more individual containers containing a pharmaceutical composition disclosed according to the present application, wherein said individual containers may be ampoules, small glass vials, etc. Preferably, said pharmaceutical kit further comprises instructions or labels on how to administer said pharmaceutical composition to an individual in need thereof. The administration comprises administration within said local lesion, or administration within the local lesion and outside the local lesion, said administration outside the local lesion is, for example, axillary subcutaneous injection. At the time of administration, the ratio of the administration amount of said pharmaceutical composition to the volume of the target region within said local lesion is >0.1, 0.15-1.5, preferably 0.23-1.5 or 0.5-1.5.

The preparation of the pharmaceutical composition of the present invention comprises: preparing a local pharmaceutical formulation comprising the probiotic component and optionally other substances present, or preparing a local pharmaceutical formulation comprising the probiotic component, the co-administration substance, and optionally other substances. Wherein said drug may be a liquid drug or a semi-fluid drug, and the liquid drug may be a solution (e.g. a solution of a hydrophilic solvent, preferably an aqueous solution), a suspension, an emulsion. In one embodiment, said drug may be an in vivo administration agent or a body surface daubing agent.

The inactivated probiotics in the present application can be prepared by high temperature inactivation, high temperature and high pressure inactivation, ultraviolet inactivation, chemical reagent inactivation, radiation inactivation, etc. The disrupted probiotic component in the present application can be prepared by 1) subjecting the live probiotics to high pressure homogenization disrupting method, oscillating bead disrupting method, high speed stirring bead grinding disrupting method, ultrasonic disrupting method, impact disrupting method, osmotic pressure impact disrupting method, freeze-thaw disrupting method, enzymatic dissolution disrupting method, chemical disrupting method, detergent disrupting method, etc.; if necessary, 2) preparing the preparation of 1) as isolated component of the disrupted probiotics, such as water-insoluble component particles of the disrupted probiotics, water-soluble component of the disrupted probiotics, by separation engineering such as filtration or/and centrifugation. In one embodiment, the probiotic water-soluble component of the present invention is prepared by the method of preparation of water-soluble component of the disrupted probiotics (e.g. supernatant component of the disrupted probiotics) as described above or the method of preparation of probiotic water-soluble component (e.g. probiotic extract) in the prior art. In one embodiment, the water-insoluble component particles of the probiotics of the present invention is prepared by the method described above for the preparation of the water-insoluble component particles of the disrupted probiotics (e.g. disrupted probiotic precipitation component) or the method in prior art for the preparation of probiotic water-insoluble component particles (e.g. cell wall polysaccharides). In one embodiment, the probiotic semi-fluid-like component of the present invention may be prepared by a method of a heating step of a mixture of a probiotic polysaccharide or its analog and water. In one embodiment, said probiotic polysaccharide/water mixture is preferably heated at a temperature of 50-110°C by means of microwave heating, electric oven heating, steam heating, etc. In one embodiment, the preparation of the pharmaceutical composition of the present invention comprising the probiotic semi-fluid-like component and the co-administration substance comprises: mixing said probiotic polysaccharide or its analogues, said co-administration substance and water homogeneously and then performing said heating. In one embodiment, the preparation of the pharmaceutical composition of the present invention further comprises: making the concentrations of said probiotic component, said co-administration substance and optionally other substances present in said pharmaceutical formulation be greater than or equal to a desired concentration for administration in the technological solution of the present invention. When the concentration is greater than that for administration in the pharmaceutical composition of the present invention, it may be further diluted for use.

Preferably, the preparation of the pharmaceutical composition of the present invention further comprises: making the contents of said probiotic component, said co-administration substance and optionally other substances present in said pharmaceutical formulation be suitable to meet its required drug volume/target region volume ratio in the technical solution of the present invention, such as dispensing and sealing the drug according to the drug volume/target region volume ratio. Preferably, the preparation of the pharmaceutical composition of the present invention further comprises: sterilizing the preparation by high temperature sterilization, high temperature and high pressure sterilization, ultraviolet sterilization, chemical reagent sterilization, radiation sterilization, etc.

In accordance with the principles of these methods described above, the person skilled in the art may prepare a variety of specific dosage forms comprising the compositions of the present invention employing any suitable specific method. For example, variations in the pharmaceutical compositions of the present invention comprises: inclusion of the pharmaceutical composition of different kinds and concentrations, inclusion of other drugs of different kinds and concentrations, inclusion of other additives of different kinds and concentrations (e.g., pain relievers, activators, etc.).

The term "local lesion disease" refers to a disease with symptoms of a local lesion. The term "local lesion (also referred to as lesion)" refers to a primary or secondary abnormal region of the body of an animal (preferably a human) that comprises structurally (e.g., diseased tissue), morphologically or functionally symptomatic regions and abnormal regions connected thereto. Local lesion diseases described in the present application comprise solid tumors, non-neoplastic enlargements such as non-neoplastic nodules, local inflammatory conditions such as cervical erosions, abnormalities in the function of secretory glands, and skin diseases. If the local lesion disease is a solid tumor, the local lesion is a tumor body and its tissue where the tumor cells are located, and the abnormal regions connected thereto are adjacent regions connected to the tumor body (e.g., through lymphatic vessels or blood vessels) and having or suspected of having tumor cells; if the local lesion disease is a non-neoplastic enlargement, the local lesion is an abnormal non-tumor mass such as a hyperplasia, cyst, nodule, etc.; a nonneoplastic nodule such as a polyp in the thyroid, breast, liver, intestine, etc. If the local lesion disease is a local inflammation, the local lesion is an inflamed region such as inflamed surface or inflamed body; if the local lesion disease is an abnormal secretion, the local lesion is the source of the abnormality or the secretory gland where it is located; for example, if the disease is an abnormal insulin secretion, the source of the abnormality is the islet of the pancreas, and the local tissue is the islet of the pancreas or the pancreas where the islet is located; if the disease is a skin disease, the local tissue is the diseased skin or the appendages of the diseased skin.

The term "tumor" refers to a mass formed by the abnormal proliferation of cells or mutated cells, and comprises solid tumors. The term "solid tumor" refers to a tumor having a tumor body, which can be a tumor due to any pathology (malignant and non-malignant) and a tumor at any stage, if it is classified according to the type of tumor cells, there is epithelial cell tumor, sarcoma, lymphoma, germ cell tumor, embryo cell tumor, etc.; if it is named according to the organ or tissue where the tumor cells are concentrated, it comprises tumors named according to the following organs or tissues: brain, skin, bone, muscle, breast, kidney, liver, lung, gallbladder, pancreas, brain, esophagus, bladder muscle, large intestine, small intestine, spleen, stomach, prostate, testis, ovary, or uterus. Particularly, said tumor comprises malignant and non-malignant tumors, wherein said malignant tumors comprise breast cancer, pancreatic cancer, thyroid cancer, nasopharyngeal cancer, prostate cancer, liver cancer, lung cancer, intestinal cancer, oral cancer, esophageal cancer, stomach cancer, laryngeal cancer, testicular cancer, vaginal cancer, uterine cancer, ovarian cancer, malignant lymphoma, malignant brain tumor, etc.; said non-malignant tumors include breast tumor, pancreatic tumor, thyroid tumor, prostate tumor, hepatoma, lung tumor, intestinal tumor, oral cancer, esophageal tumor, gastric tumor, nasopharyngeal tumor, laryngeal tumor, testicular tumor, vaginal tumor, uterine tumor, tubal tumor, ovarian tumor, lymphoma, brain tumor, etc. The local lesion diseases can also be chronic mucosal skin candidiasis, various ringworms and other skin diseases.

When the pharmaceutical compositions described in the present application are used to treat diseases, they can also be administered in combination with other interventional therapies, systemic chemotherapy, immunotherapy, photodynamic therapy, acoustodynamic therapy, surgical interventions, etc., to further enhance their efficacy.

Based on the studies described in more detail below, and although the specific mechanism needs further study, the pharmaceutical composition of the present invention exhibits the pharmacological effect of promoting effective destruction of tumor tissue with only minimal damage to the patient's normal tissues, resulting in safe and effective treatment of local lesion disease.

The probiotics used in the present application and some of the probiotic components (Saccharomyces water-insoluble β-glucan particles, Saccharomyces water-soluble β-glucan, Saccharomyces hydroxymethyl glucan, Saccharomyces carboxymethyl glucan, Saccharomyces ribonucleic acid, etc.) can be commercially available, and other probiotic components (inactivated probiotics, probiotic disrupt, a supernatant component of probiotic disrupt, a precipitate fraction of probiotic disrupt) are made in-house using the previously described methods (e.g. inactivating at a high temperature of 100°C for 2 hours, pulverizing in a pulverizer at 5,000 rpm, separating supernatant and precipitating in a centrifuge at 10,000 rpm).

The experimental animals used in the following examples are SPF (Specific Pathogen Free) grade animals purchased from professional laboratory animal companies, where mice and nude mice are healthy females of 6-8 week old, weighing 17.5-20.5 g, unless otherwise stated.

In the following examples, unless otherwise stated, animal tests for subcutaneous transplantation of cells to generate a local lesion (e.g., tumor body) were performed in accordance with test guidelines issued by the drug regulatory authorities and by conventional methods of subcutaneous inoculation of cells. Unless otherwise stated, the modeling was successful when the local lesion (e.g., tumor body) grows to a desired volume (e.g., 50-500 mm$^3$ in tumor-bearing mice). After modeling, the animals were divided into 6 animals per group and observed periodically to determine general status, body weight, food intake, animal graft-versus-host disease, tumor volume, tumor weight, survival time, etc. The models comprised a tumor body comprising tumor cells (representing the tumor body), fibroblast-containing nodules (representing a fibroblast-comprising tumor body and other local lesions), and normal biopsies (representing local lesions comprising normal tissues), respectively.

Local lesion (e.g., tumor) volume (V), relative local lesion (e.g., tumor) proliferation rate (R), local lesion (e.g., tumor) inhibition rate (r'), and tumor inhibition rate (r) were calculated according to the following equation:

$$\text{Volume of the local lesion (e.g. tumor): } V = 0.5 \times a \times b^2,$$

wherein a and b represent the length and width of the local lesion, respectively;

$$\text{Local lesion (e.g., tumor) proliferation rate (R)} = TV/CV \times 100,$$

wherein TV and CV are the local lesion volumes in the study group and negative control group, respectively;

$$\text{Local lesion (e.g., tumor) inhibition rate (r')} = 100\% - R,$$

wherein R is the local lesion proliferation rate.

$$\text{Tumor inhibition rate } r = (CW-TW)/CW \times 100\%,$$

wherein TW and CW are the mean tumor weights of the study group and negative control group, respectively.

In the following examples, the pharmaceutical effect of drug i is designated as Ei, which could be represented as ri' or ri. The action type of a drug, i.e. pharmacology, could be studied by the pharmaceutical effect, in particular the pharmaceutical effect of the same drug in different regimens. For example, when the difference in pharmaceutical effects of drug i between regimens X and Y is not significant (e.g., Ei x/Ei $_Y$ < 200%), it indicates that the pharmacology of the drug is same; when the difference in pharmaceutical effects of drug i between regimens X and Y is not significant (e.g., $Ei_X/Ei_Y$ > 200%), it indicates that the pharmacology of the drug i in regimen X greatly exceeds the expected range of its pharmacology in regimen Y in terms of dynamics and is likely to involve a new pharmacology different from that of regimen Y. If two drugs show significantly different $Ei_X/Ei_Y$ relationships, they are likely to involve different pharmacology; if two drugs show similar $Ei_X/Ei_Y$ relationships, they are likely to involve the same pharmacology, or at least similar pharmacology, such as chemical ablation-like and chemical ablation pharmacology.

In the following examples, experimental results (e.g., tumor weight, tumor volume, lesion tissue volume ) were expressed as mean $\pm$ standard deviation (x $\pm$ s), and the differences between two experimental animal groups and group mean value were tested for significance using the statistical software SPSS13.0 or SPSS19.0, and the test was performed using the statistical quantity t. The test level of $\alpha$ = 0.05, P < 0.05 indicated a statistically significant difference, while P > 0.05 was not statistically significant.

Positive controls for chemotherapy comprised a classical cytotoxic drug (e.g., 0.5-1% 5-fluorouracil, which had a tumor inhibition rate of $\geq$ 30% under the conditions of the following examples) and a classical chemical ablation agent (e.g., 75-99% ethanol, which had a tumor inhibition rate of $\geq$15% under the conditions of the following examples). Immune-enhancing positive controls comprised immune-enhancing agents such as cytokines (e.g., interleukin-12), bacterial components (e.g., attenuated bacterial vaccines, etc.), and vaccine adjuvants.

Within the scope of the present invention, the combination of drug A and drug B was designated as B/A, and their combination effect was determined by q = actual combination effect/theoretically expected purely additive effect, wherein the drug efficacies of drug A alone and drug B alone was designated as $E_A$ and $E_B$, respectively, and the actual combinational drug efficacies of A/B was designated as E $_{A+B}$, such as for the tumor inhibition rate. When q = 1, the actual combination effect was in accordance with the theoretical expectation, showing an additive effect; when q < 1, the actual combination effect was weaker than the theoretical expectation, showing an antagonistic effect; when q > 1, the actual combination effect exceeded the theoretical expectation, showing a synergy.

The method for determining the effect of combined drug administration in animal experiments was Burgi method (Burgi Y. Pharmacology; Drug actions and reactions.Cancerres. 1978, 38(2), 284-285). The Burgi method was modified by Zhengjun JIN (Zhengjun JIN, Equal probability and curve and "Q50", Journal of the Second Shanghai Medical College; 1981, 1, 75-86) with q calculated as: q = $E_{A+B}/(E_A+E_B-E_A \times E_B)$. In the following examples of the present invention, the combinational pharmaceutical efficacies (drug efficacies) of drug A and drug B co-administration was determined by ratio q of actual pharmaceutical efficacy (drug efficacy) /theoretical pharmaceutical efficacy (drug efficacy) according to Zhengjun JIN method, as follows.

When the combination group of drug A and drug B did not show meaningful drug efficacy (e.g., r or r' $\leqq$ 15 %), the combination administration did not show a meaningful combination effect either and was regarded as a negligible combination effect in the present invention. When the combination group showed meaningful drug efficacy (e.g. tumor inhibition rate $\geqq$ 15 %), if the ratio q of actual drug efficacy/expected drug efficacy =1.00, then the drug efficacy of the combination group was additive (the actual effect was consistent to that of theoretical purely additive expectation); if the ratio q of actual drug efficacy /expected drug efficacy >1.00, then the combinational drug efficacy was significantly synergistic drug efficacy (the actual effect exceeded theoretical purely additive expectation); if the ratio q of actual drug efficacy /expected drug efficacy <1.00, then the combinational drug efficacy was significantly antagonistic drug efficacy (the actual effect was less than the theoretical purely additive expectation).

**Example 1. Preparation of compositions**

Numerous different compositions of the present invention could be formulated according to the preparation method of the compositions of the present invention described above. The constitutions of some of the compositions of the present invention prepared in this Example were listed in Table 2 (the probiotic component is used as an active ingredient) and Table 3 (the probiotic component is used as a synergistic active ingredient).

Table 2. Components, pharmacological concentrations ( > 0.1 % , ≥0.25 % , 0.25-25 %, preferably 0.5-15 %, more preferably 1-15% or 5-15 %) of the aqueous formulations of the compositions comprising probiotic components

| Types of the probiotic component | Specific examples of the compositions | |
|---|---|---|
| | No. | Specific examples |
| 1.1 inactivated probiotics, pharmacological concentration ≥ 0.75%, 0.75-15%, preferably | A1 | 2.5% inactivated Saccharomyces cerevisiae |
| | A2 | 2.5% inactivated Saccharomyces boulardii |
| | A3 | 2.5% inactivated Bacillus licheniformis |
| 1.5-15% or 2.0-10% | A4 | 2.5% inactivated Bacillus subtilis |
| | A5 | 2.5% inactivated Enterococcus faecium |
| | A6 | 2.5% inactivated Bifidobacterium longum |
| | A7 | 2.5% inactivated lactic acid bacteria |
| | A8 | 1.5% inactivated Saccharomyces cerevisiae /1% inactivated Saccharomyces boulardii |
| 1.2 a water-insoluble particle of a probiotic component /a probiotic water-soluble component, pharmacological concentration ≥0.75%, 0.75-15%, preferably 1.5-15% or 2.0-10% | A9 | 2.5% disrupted Saccharomyces cerevisiae |
| | A10 | 2.5% disrupted Saccharomyces boulardii |
| | A11 | 2.5% disrupted Bacillus licheniformis |
| | A12 | 2.5% disrupted Bacillus subtilis |
| | A13 | 2.5% disrupted Enterococcus faecium |
| | A14 | 2.5% disrupted Bifidobacterium longum |
| | A15 | 2.5% disrupted Lactobacillus |
| | A16 | 1.5% disrupted Saccharomyces cerevisiae /1% disrupted Saccharomyces boulardii |
| 1.3 a water-insoluble particle of a probiotic component, pharmacological concentration 0.5-15%, preferably 1.5-15% or 2.0-10% | A17 | 1.4% disrupted Saccharomyces cerevisiae precipitation component |
| | A18 | 1.4% disrupted Bacillus licheniformis precipitation component |
| | A19 | 5% water-insoluble $\beta$-glucan particle |
| 1.4 a probiotic semi-fluid-like component, pharmacological concentration 2.6-15%, preferably 5-15% | A20 | 10% water-insoluble $\beta$-glucan particle, which is heated to obtain a semi-fluid |
| | A21 | 10%% disrupted Saccharomyces cerevisiae precipitation, which is heated to obtain a semi-fluid |

(continued)

| Types of the probiotic component | Specific examples of the compositions | |
|---|---|---|
| | No. | Specific examples |
| 1.5 a probiotic water-soluble component 1 (supernatant components and extracts), pharmacological concentration 0.15-25%, preferably 0.35-15% or 5-15% | A22 | 1.1% disrupted Saccharomyces boulardii supernatant components |
| | A23 | 1.1% disrupted Saccharomyces boulardii supernatant components |
| | A24 | 1.1% disrupted Bacillus licheniformis supernatant components |
| | A25 | 10% Saccharomyces cerevisiae extracts |
| 1.6 a probiotic water-soluble component 2 (glucan and derivatives thereof), pharmacological concentration > 0.1, or 0.15-25%, preferably 0.35-15% or 5-15% | A26 | 5% water-soluble yeast β-glucan |
| | A27 | 10% hydroxymethyl yeast β-glucan |
| | A28 | 10% yeast β-glucan sulfate |
| | A29 | 10% degraded yeast β-glucan |
| 1.7 a probiotic water-soluble component 3 (nucleic acids and derivatives thereof), pharmacological concentration 1-30%, preferably 1-25% or 15-25% | A30 | 20% yeast RNA |
| | A31 | 20% Pichia yeast DNA |

Several preparation examples for the preparations in Table 2 were listed below.

Preparation method of A1-A8: probiotics (e.g., 2.5 g of dried Saccharomyces cerevisiae powder), other optionally existent components, and a liquid carrier (e.g., water for injection) for making a constant volume to the total volume (e.g., 100 ml) were measured and taken at the desired concentration (e.g., required for local chemical effect) and mixed slowly and homogeneously to obtain a probiotic mixture. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The preparation (e.g., 2.5% Saccharomyces cerevisiae) was subjected to Pasteur inactivation (60°C, 48 h) in a Pasteur inactivation cabinet type machine to obtain A1. Different inactivated probiotics (e.g., preparations A1-A8 in the Table above) could be prepared separately from different probiotics using the same method as for the preparation of A1.

Preparation method of A9-A16: probiotics (e.g., 2.5 g of dried Saccharomyces cerevisiae powder), other optionally existent components, and a liquid carrier (e.g., water for injection) for making a constant volume to the total volume (e.g., 100 ml) were measured and taken at the desired concentration (e.g., required for local chemical effect), mixed slowly and homogeneously, and disrupted using a homogenizer. By adjusting the homogenizing process parameters (e.g., rotational speed 10,000-25,000 rpm, rotational time 0.5-1 min, number of homogenizations 2-10 times), disrupted probiotics having different disrupting degrees (preferably 100% disrupted) could be obtained. If the liquid mixture was dispensed (e.g., 10 ml/bottle) and sealed at the desired ratio of drug volume/target region volume (e.g., 30 cm$^3$ of the average volume of a clinically common solid tumor), a dosage form and format (or specification) of the pharmaceutical composition that provided intratumoral local effect was obtained. The resulting preparation was A9. Different compositions (e.g., preparations A9-A16 in the Table above) could be prepared separatelyfrom different probiotics using the same method as for the preparation of A9.

In addition, using the above preparation method for preparing disrupted probiotics, a highly concentrated disrupted probiotic suspension (e.g., 10% disrupted Saccharomyces cerevisiae suspension) could be prepared from the probiotics (e.g., 10 g of dried Saccharomyces cerevisiae powder) and water for injection for making a constant volume to 100 ml. The suspension was added to a centrifuge bottle and centrifuged in a centrifuge. By adjusting the centrifuge speed (e.g. 1000-25000 rpm), centrifugation time (e.g. 0.5-30 min) and the number of centrifugations (e.g. 2-4 times), the disrupted probiotic supernatant components and the disrupted probiotic precipitation component under different degrees of centrifugation could be obtained. After centrifugation, the supernatant was decanted out and used separately, and the remaining precipitation component was dried (e.g., 125°C, 90 minutes) and prepared into a dry powder of the disrupted probiotic precipitation component. The difference between the raw probiotics (e.g. 10 g Saccharomyces cerevisiae) and the dry powder of the disrupted probiotic precipitation component (e.g. 4 g) prepared therefrom was calculated as the dry weight of the disrupted probiotic supernatant component (e.g. 6 g).

Preparation method of A17-A19: the probiotic water-insoluble component particles (e.g., 1.4 g of dried powder of disrupted Saccharomyces cerevisiae prepared according to the method of Example 1a), other optionally existent components, and a liquid carrier (e.g., water for injection) for making a constant volume to the total volume (e.g., 100 ml) were measured and taken at the desired concentration (e.g., required for local chemical effect) and slowly mixed well. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was A17. Using the same method as for the preparation of A17, the preparations having different disrupted probiotic precipitation component prepared separately from different probiotics (e.g., preparations A17 and A18 in the Table above) and commercially available water-insoluble β-glucan particles (e.g., preparation A19 in the Table above) could be prepared.

The preparation method of A20-A21: the semi-fluid-like component producible from probiotics (e.g., 10 g of water-insoluble β-glucan particles), other optionally existent components, and a liquid carrier (e.g., water for injection) for making a constant volume to the total volume (e.g., 100 ml) were measured and taken at the desired concentration (e.g., required for local chemical effect), mixed slowly and homogeneously as a suspension. Then the suspension was heated (e.g. temperature 50-110°C and time 0.5-24h), and a semi-fluid was formed after cooling. If the semi-fluid was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was A16. Using the same method as for the preparation of A16, different semi-fluid compositions could be prepared separately from semi-fluidizable components of different probiotics. Experiments showed that only when the concentration of the probiotic semi-fluidizable component was greater than a certain threshold (e.g., β-glucan ≥ 2.5%) could the liquid comprising it be converted to a semi-fluid like upon heating.

Preparation method of A22-A24: the probiotic water-soluble component (e.g., 1.1 g of the supernatant composition of the disrupted Saccharomyces cerevisiae prepared according to the method of A17-A18 or 20 ml of the equivalent supernatant solution), other optionally existent components, and a liquid carrier (e.g., water for injection) for making a constant volume to the total volume (e.g., 100 ml) were measured and taken at the desired concentration (e.g., required for local chemical effect), slowly mixed well. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was A22. Using the same method as for the preparation of A12, different compositions (e.g., preparations A22-A24 in the Table above) could be prepared separately from different probiotics.

Preparation method of A25-A31: the probiotic water-soluble component (e.g., 5 g of water-soluble yeast β-glucan obtained from a commercial source), other optionally existent components, and a liquid carrier (e.g., water for injection) for making a constant volume to the total volume (e.g., 100 ml) were measured and taken at the desired concentration (e.g., required for local chemical effect), slowly mixed well. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was A26. Using the same method as for the preparation of A26, different compositions (e.g., preparations A25-A32 in the Table above) could be prepared separately from different probiotic components obtainable from commercial sources.

The aqueous formulations of the compositions obtained using the above preparation method could be further added a chemical active ingredient according to prior art method to produce aqueous formulations of compositions comprising a probiotic component* and a chemical active ingredient.

Table 3. Components and component ratios of aqueous formulations of compositions comprising a probiotic component* and a chemical active ingredient

| Type of probiotic component*/each co-administration substance, the amount ratio of the probiotic component to the co-administration substance | Specific examples of compositions | |
|---|---|---|
| | No. | Specific examples |
| A. a composition comprising a probiotic component and a weak local acting compound | | |
| 2.1 probiotic component/ amino acid nutrient, $W_{probiotic\ component}/W_{amino\ acid\ nutrient}$ : (1-20)/(1-100) | B1 | 10% water-soluble yeast β-glucan /20% arginine |
| | B2 | 1.5% disrupted Saccharomyces boulardii /20% lysine |
| | B3 | 1.5% disrupted Saccharomyces boulardii /10% arginine /10% lysine |
| | B4 | 2% disrupted Saccharomyces cerevisiae precipitation component /10% reduced glutathione |
| 2.2 probiotic component/vital dye, $W_{probiotic\ component}/W_{vital\ dye}$: (7-90)/(1-100) | B5 | 10% Saccharomyces cerevisiae extracts /1% methylene blue /10% Bengal red |
| | B6 | 20% yeast ribonucleic acid /1% methylene blue |
| | B7 | 5% hydroxymethyl yeast β-glucan /1% methylene blue |
| 2.3 probiotic component/quinine drugs, $W_{probiotic\ component}/W_{quinine\ drugs}$. (2-90)/(1-100) | B8 | 1.5% disrupted Saccharomyces cerevisiae/5% quinine dihydrochloride |
| | B9 | 5% hydroxymethyl yeast β-glucan //5% quinine dihydrochloride |
| 2.4 probiotic component/acidifier or/and basifier, $W_{probiotic\ component}/W_{acidifier\ or/and\ basifier}$: (2-60)/(1-100) | B10 | 10% water-soluble yeast β-glucan /3% sodium hydroxide /7% sodium bicarbonate |
| | B11 | 20% yeast ribonucleic acid /3% sodium hydroxide /7% sodium bicarbonate |
| | B12 | 5% hydroxymethyl yeast β-glucan /3% sodium hydroxide /7% sodium bicarbonate |
| 2.5 probiotic component/ amino acid nutrient /vital dye, $W_{probiotic\ component}/W_{amino\ acid\ nutrient}/W_{vital\ dye}$: (5-100)/(5-500)/(1-100) | B13 | 10% water-soluble yeast β-glucan /20% arginine /1% methylene blue |
| | B14 | 20% yeast ribonucleic acid /20% lysine /1% methylene blue |
| | B15 | 5% hydroxymethyl yeast β-glucan /10% arginine /10% glycine /1% methylene blue |
| 2.6 probiotic component/ amino acid nutrient /acidifier or/and basifier, $W_{probiotic\ component}/W_{amino\ acid\ nutrient}/W_{acidifier\ or/and\ basifier}$: (5-100)/(5-500)/(1-100) | B16 | 10% water-soluble yeast β-glucan /20% arginine /3% sodium bicarbonate |
| | B17 | 20% yeast ribonucleic acid /20% arginine /3% sodium bicarbonate |
| | B18 | 5% hydroxymethyl yeast β-glucan /20% glycine /5% acetic acid |

(continued)

| B. a composition comprising a probiotic component and a cytotoxic drug | | |
|---|---|---|
| 2.7 probiotic component/ cytotoxic drug, W probiotic component/W cytotoxic drug. (1-300)/(0.1-15) | B19 | 7.5% β-glucan semi-fluid /1% 5-fluorouracil |
| | B20 | 10% β-glucan semi-fluid /1% 5-fluorouracil /0.1 cisplatin |
| | B21 | 7.5% β-glucan semi-fluid /2.5% gemcitabine |
| C. a composition comprising a probiotic component, a weak local acting compound and a cytotoxic drug | | |
| 2.8 composition comprising a probiotic component, a vital dye and a cytotoxic drug, W probiotic component/W co-administration substance : (1-110)/(1-100) | B22 | 7.5% β-glucan semi-fluid /1% methylene blue /1% 5-fluorouracil |
| | B23 | 5% water-insoluble β-glucan particle/1% methylene blue /1% 5-fluorouracil |
| | B24 | 2.5% inactivated Saccharomyces cerevisiae/1% methylene blue /1% 5-fluorouracil |
| | B25 | 20% yeast ribonucleic acid /1% methylene blue /0.1% cisplatin /1% 5-fluorouracil |
| | B26 | 5% hydroxymethyl yeast β-glucan /1% methylene blue /1% 5-fluorouracil |
| 2.9 composition comprising a probiotic component, an amino acid nutrient and a cytotoxic drug, W probiotic component/W co-administration substance : (1-110)/(1-100) | B27 | 2.5% inactivated Saccharomyces boulardii 20% arginine //1% 5-fluorouracil |
| | B28 | 2.5% inactivated Bacillus licheniformis/ 20% arginine /1% 5-fluorouracil |
| | B29 | 10% water-soluble yeast β-glucan /20% arginine /1% 5-fluorouracil |
| 2.10 composition comprising a probiotic component, an amino acid nutrient, a cytotoxic drug and acidifier or/and basifier, W probiotic component/W co-administration substance: (1-110)/(1-100) | B30 | 10% water-soluble yeast β-glucan /20% arginine /1% 5-fluorouracil /2% sodium bicarbonate |
| | B31 | 25% yeast RNA/20% arginine /1% 5-fluorouracil /2% sodium bicarbonate |
| | B32 | 10% water-insoluble yeast β-glucan particle/10% reduced glutathione /1% 5-fluorouracil /1% sodium hydroxide |
| *: The types of a probiotic component and their concentrations were those in Table 2 | | |

Several preparation examples for the preparations in Table 3 were exhibited below.

Preparation method of the aqueous formulations (B1, B5-B7, B9-B18, B25, B26, B29-B32) of the compositions comprising a probiotic component (water-soluble components 1-3) and a chemical active ingredient: the probiotic water-soluble component (e.g., 10 g of water-soluble yeast β-glucan), co-administration drug (e.g. 20 g of arginine), other optionally existent components, and water for injection for making a constant volume to the total volume of 100 ml were measured and taken at the desired synergistic amount ratio and synergistic concentration (e.g., required for local synergy or/and medium- and long-term synergy) , and slowly mixed well. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was B1. Using the same method as for the preparation of B1, different compositions (e.g., preparations B 1, B5-B7, B9-B18, B25, B26, B29-B32 in the Table above) could be prepared from different probiotic water-soluble components and their co-administration drugs, respectively.

Preparation method of the aqueous formulations (B2-B4, B8) of the compositions comprising a probiotic component (water-insoluble particles/water-soluble components) and a chemical active ingredient: the probiotics (e.g. 1.5 g Saccharomyces cerevisiae), co-administration drug (e.g. 20 g of lysine), other optionally existent components, and water for injection for making a constant volume to the total volume of 100 ml were measured and taken at the desired synergistic amount ratio and synergistic concentration (e.g., required for local synergy or/and medium- and long-term synergy), and slowly mixed well and disrupted using a homogenizer to obtain a suspension of disrupted probiotics/co-administration drug by using the same method as for the preparation of A10. If the suspension was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was B2. Using the same method as for the preparation of B2, different compositions (e.g., preparations B2-B4, B8 in the Table above) could be prepared from different probiotics or/and different co-administration drugs, respectively.

Preparation method of the aqueous formulations (B19-B22) of the compositions comprising a probiotic component (semi-fluidizable component) and a chemical active ingredient: the probiotic semi-fluidizable component (e.g., 7.5 g of water-soluble β-glucan), co-administration drug (e.g. 1 g of 5-fluorouracil), other optionally existent components, and a liquid carrier (e.g., water for injection) for making a constant volume to a total volume (e.g., 100 ml) were measured and taken at the desired synergistic amount ratio and synergistic concentration (e.g., required for local synergy or/and medium- and long-term synergy), mixed slowly and homogeneously into a suspension, which was then heated (e.g., temperature: 50-110° C and time: 0.5-24 hours) and cooled to form a semi-fluid. If the semi-fluid was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was B19. Using the same method as for the preparation of B17, different semi-fluid compositions (e.g., preparations B20-B22 in the Table above) could be prepared.

Preparation method of the aqueous formulations (B23) of the compositions comprising a probiotic component (a water-insoluble component particles) and a chemical active ingredient: the probiotic water-insoluble component particles (e.g., 10 g of water-insoluble β-glucan particles in A19), co-administration drug (e.g. 10 g of reduced glutathione, 1 g of 5-fluorouracil, 1 g of sodium hydroxide), other optionally existent components, and water for injection for making a constant volume to a total volume of 100 ml were measured and taken at the desired synergistic amount ratio and synergistic concentration (e.g., required for local synergy or/and medium- and long-term synergy), and mixed slowly and homogeneously. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The resulting preparation was B23. Using the same method as for the preparation of B6, different compositions could be prepared from different probiotic components (water-insoluble particles such as probiotic precipitation component) and different co-administration drugs, respectively.

Preparation method of the aqueous formulations (B24, B27, B28) of the compositions comprising a probiotic component (inactivated probiotics) and a chemical active ingredient: the probiotics (e.g., 2.5 g of dried Saccharomyces cerevisiae powder), other ingredients (e.g., 1 g of methylene blue, 1 g of 5-fluorouracil), and water for injection for making a constant volume to a total volume of 100 ml were measured and taken at the desired synergistic amount ratio and synergistic concentration (e.g., required for local synergy or/and medium- and long-term synergy), and slowly mixed well to obtain probiotics/co-administration drug liquid mixture. If the liquid mixture was dispensed (e.g., 10 ml/bottle) and sealed at the desired ratio of drug volume/target region volume (e.g., 30 cm$^3$ of the average volume of a clinically common solid tumor), a dosage form and format of the pharmaceutical composition that provided intratumoral local effect was obtained. The preparation (e.g., 2.5% Saccharomyces cerevisiae) was Pasteurized (60°C, 48 h) in a Pasteur inactivation cabinet to obtain B24. Using the same method as for the preparation of B24, different inactivated probiotics (e.g., preparations B27 and B28 in the Table above) could be prepared from different probiotics, respectively.

If the above inactivated preparations (e.g., A1-B31, B1-B32) were placed in a Pasteur inactivation cabinet machine for Pasteur sterilization (60°C, 48 hours), sterilized liquid injection formulations were obtained.

It should be noted that during the preparation of A1-16 and B1-B19, said sterilization was performed via Pasteur sterilization at 60°C for 48 hours to obtain sterilized liquid injection formulations.

When a lyophilized powder formulation was prepared, the above liquid formulations were freeze-dried, respectively, and the process conditions of said freeze-drying were: -45° C held for 4h for pre-freezing, held for at least 10h for sublimation when ramped up to - 15°C at a rate of 0.1°C/min; ramped up to 30°C and held for 6h for desorption drying, and the lyophilized powder formulation was obtained. The water for injection was dispensed as required, such as 7.5ml/bottle, and the bottle was sealed by a cap, to obtain a solvent bottle for injection. When used, the sterile solvent in the bottle was pumped into the above lyophilized powder bottle for injection and mixed well to form a liquid drug, i.e. injectable drug, such as 1.5% disrupted component of Saccharomyces cerevisiae /20% amino acid.

**Example 2. Pharmacological studies in immunosuppressed animal models**

The tumor-bearing nude mice were widely used for drug studies on chemotherapy rather than immunotherapy in patients with solid tumors. Taking nude mice as experimental subjects, human liver cancer HepG2 cells were used as modeling cells, and $1 \times 10^5$ cells/mouse were injected into the right axilla subcutaneously for transplantation tumor modeling, and the average tumor volume of successfully modeled nude mice was 161.3 mm$^3$. The model animals were randomly divided into 17 groups. The drugs were administered according to the drug and dosing method in Table 4. Each group was dosed once with an injection volume of 150 μl/mouse. On the 7th day after drug administration, the animals were euthanized, and after dissection, the tumor tissue was taken out to determine the tumor weight, and the tumor inhibition rate (r) was calculated relative to the negative control group.

Table 4 Data of tumor inhibition rate in different groups

| Groups | Drug i | Description | Administration route | Tumor weight (x±s_g) | Tumor inhibition rate r |
|---|---|---|---|---|---|
| 01 | Negative control | physiological saline | intravenous injection | 0.241±0.113 | - |
| 1 | Cytotoxic positive control | 1% 5-fluorouracil | intravenous injection | 0.139±0.094 | 42.3% |
| 2 | Local action positive control | Anhydrous ethanol | intravenous injection | 0.226±0.113 | 6.1% |
| 3 | Immunoenhancement control | $4 \times 10^4$U/ml interleukin-12 | intravenous injection | 0.230±0.102 | 4.7% |
| 4 | 5% inactivated probiotics | 5% heat inactivated Saccharomyces cerevisiae | intravenous injection | 0.233±0.107 | 3.5% |
| 5 | 5% probiotic water-insoluble component particle | 5% β-glucan particle | intravenous injection | 0.228±0.114 | 5.2% |
| 6 | 5% probiotic water-soluble component | 5% disrupted Saccharomyces cerevisiae supernatant components | intravenous injection | 0.238±0.093 | 1.3% |
| 02 | Negative control | physiological saline | intratumoral injection | 0.263±0.129 | - |
| 7 | Cytotoxic positive control | 1% 5-fluorouracil | intratumoral injection | 0.133±0.094 | 49.5% |
| 8 | Local action positive control | anhydrous ethanol | intratumoral injection | 0.122±0.085 | 53.6% |
| 9 | Immunoenhancement control | $4 \times 10^4$U/ml interleukin-12 | intratumoral injection | 0.241±0.111 | 8.1% |
| 10 | 5% inactivated probiotics | 5% heat inactivated Saccharomyces cerevisiae | intratumoral injection | 0.165±0.063 | 37.2% |
| 11 | 5% probiotic water-insoluble component particle | 5% β-glucan particle | intratumoral injection | 0.183±0.097 | 30.4% |
| 12 | 5% probiotic water-soluble component | 5% disrupted Saccharomyces cerevisiae supernatant components | intratumoral injection | 0.180±0.079 | 31.7% |
| 13 | 5% probiotic semi-fluid-like component | semi-fluid formed by heating 5% β-glucan particle and cooling | intratumoral injection | 0.157±0.081 | 40.2% |

(continued)

| Groups | Drug i | Description | Administration route | Tumor weight (x±s_g) | Tumor inhibition rate r |
|---|---|---|---|---|---|
| 14 | 5% probiotic component mixture | 5% disrupted Saccharomyces cerevisiae | intratumoral injection | 0.168±0.081 | 36.2% |
| 15 | 5% live probiotics | 5% Saccharomyces cerevisiae | intratumoral injection | 0.201±0.101 | 23.6% |

It was generally believed that intravenous administration of 5-fluorouracil may provide conventional chemical effect, while administration by intratumoral injection may provide common local chemical effect, and the difference in efficacy resulted from the two modes of administration was correlated with cytotoxic dynamics. The ratio of tumor inhibition rate in group 7 compared to group 1 was $E_7/E_1 < 200\%$, indicating that 5-fluorouracil acted as a cytotoxic drug under different administration conditions (systemic vs. local administration). High concentration (75-99%) of ethanol provided a drunkenness-like response by intravenous injection; and a chemical ablation effect by intratumoral injection. The increase of tumor inhibition rate in group 8 compared to group 2 far exceeded the expected limit of dynamic increase based on the latter ($E_8/E_2 > 200\%$), suggesting that ethanol could be applied as different active ingredients (drunkenness agent vs. chemical ablation agent) under different administration conditions (systemic vs. local administrations).

Interleukin-12 was usually used clinically as an immunoenhancement agent, and it did not show short-term tumor inhibition effect by intratumoral injection (group 9) as expected. In prior art, probiotics and components thereof could also be used as adjunctive drugs, the antitumor effects of which were also based on their immune enhancement effects, and the results for groups 4-6 were consistent with that of group 3. It was noteworthy that one animal died in each of groups 4 and 5 after injection, indicating the safety risk of the suspension of 5% probiotic component by intravenous injection.

Based on the immune enhancement expectation in prior art, the result of group 10 should be consistent with that of the immune enhancement control group 9. Unexpectedly, the pharmacological behavior between them was significantly different: there is almost no difference in drug efficacy between groups 9 and 3 with the same dose of the same drug; while the difference in drug efficacy between group 10 and group 4 with the same dose of the same drug exceeds the expected limit for kinetic improvement ($E_{10}/E_9 > 200\%$), which was also reflected in the significantly different drug potency between them ($E10/E9 = 459\% > 200\%$). In contrast, the difference in tumor inhibition rates between group 10 and groups 7 and 8, which were outside the pharmacological expectations, was not significant (e.g. $E_7/E_{10} = 133\% < 200\%$), and the pharmacological behaviors of group 10 and group 8 were also clearly consistent: the difference in tumor inhibition rates between group 8 and group 2 with the same dose of the same drug was significant ($E_{10}/E_9 > 200\%$). And the same drug efficacy and pharmacological behavior existed in groups 11 and 12 as in group 10.

In fact, only intra-intestinal administration enables the probiotic component to regulate the bacterial flora in the intestine; and both intra- and extra-intestinal administrations could enable the probiotic component to enhance immune effect. Therefore, when conventional administration routes such as oral and intravenous administration were excluded, local administration, especially intra-lesional administration, enables the probiotic component to provide new functions that were different from those of the prior art ($E_{10}/E_4 >> 200\%$).

The immune enhancement effect of probiotics and components thereof is based on their bacterial and fungi immunogenicity. It is usually believed that the order of bacterial immunogenicity from strong to weak is: live bacteria, inactivated bacteria which basically keep the shape of live bacteria, water-insoluble particles which have more or less the shape similar to live bacteria, semi-fluid which loses the shape of live bacteria, water-soluble components. However, the order of the pharmaceutical effect of each group of locally administered probiotics and components thereof from strong to weak was: semi-fluid-like component (group 13), inactivated probiotics that do not require essentially maintaining the shape of live bacteria (inactivated probiotics having the amount ratio of non-intact bacteria body 20%, group 10), a mixture of water-insoluble component/water-soluble component (group 14), water-soluble component (group 12), water-insoluble component (although its chemical composition was identical to that of semi-fluid-like component) (group 11), and live bacteria and fungi (group 15). This further indicated that the new function of the probiotic component did not depend on its function in prior art (e.g., bacterial immunogenicity). Thus, the concentration of the probiotic component in the pharmaceutical composition could be expressed in terms of weight concentration rather than the number of intact bacteria per unit volume.

Pharmacological function was a fundamental characteristic of a drug, and new pharmacology discovered for older drugs could often creatively yield new applications as that of a new drug. The effective application of a drug usually depends on the relative advantage of the pharmacological function, i.e., the preferred pharmacological action, provided by the efficacy and safety under experimental conditions limited by clinical treatment, such as minimizing the frequency and dose of administration. The same substance may thus be used as different active ingredients under different conditions, and the same type of substances may have different preferential options suitable for use as different active ingredients. In the above-mentioned experiments, the positive controls (5-fluorouracil, ethanol, interleukin-12) each showed the expected drug efficacy and pharmacological behavior in accordance with their preferred pharmacology (cytotoxic pharmacology, immune enhancement pharmacology, and local effect pharmacology, respectively) in different pharmacological approaches, and a comparative pharmacological study system was given. The drug efficacy shown by the probiotic component via intravenous injection might be in line with its pharmacological expectations of the prior art (e.g., immune enhancement), but its drug efficacy resulted from intra-lesional administration clearly exceeded those expectations. The pharmacological behavior even exceeded cytotoxic pharmacological expectation (if any). The pharmacological differences shown in the different modes of administration resulted in great differences in drug efficacy, a pharmacological behavior very similar to that of the local effect positive control, ethanol, suggesting that its preferred pharmacology for intra-lesional administration was local effect. In conclusion, the probiotic component as an active ingredient providing local effect (local active ingredient) greatly exceeded its expectation as an active ingredient providing any non-local effect (e.g. cytotoxic effect or immune enhancement effect) (non-local active ingredient) in terms of pharmacology and efficacy.

In order to further study the above new function, the applicant conducted the following comparative experiments using normal animal tissues as a local lesion model (the model was a classical research model of chemical ablation agent) - nude mice were used as experimental animals, and randomly divided into two groups A and B, with 6 animals in each group, and 75% ethanol aqueous solution, 5% inactivated probiotics as experimental drug, were injected into the outer lateral muscle mass of the right leg at an injection dose of 100ul/mouse, respectively. The animals were euthanized on the 7th day after drug administration, and the specimens of the outer lateral muscle mass of the right leg from the nude mice were taken out by autopsy, and the areas of abnormal regions different from normal muscles, such as of necrosis and nodules, were measured after sectioning and washing. The results showed that the areas of the abnormal regions were $35.14 \pm 15.92$ mm$^2$ and $34.12 \pm 15.81$ mm$^2$ in groups A and B, respectively, and there was no statistical difference between them (P>0.05). These results further confirmed that the local effect pharmacology of the probiotic component was similar to that of ethanol. Like ethanol, this local effect provided by the probiotic component allowed it to be used for any local lesion comprising lesion tissue.

## Example 3. Pharmacological study of local effect and optimization of pharmacological concentration

Nude mice were used as experimental subjects, and breast cancer cells (MDA-MB231) were used as modeling cells, $1 \times 10^5$ cells/mouse were injected into the right axilla subcutaneously for transplantation tumor modeling, and the average tumor volume of successfully modeled nude mice was 158.2 mm$^3$. The model animals were randomly divided into 16 groups, and the drugs were administered by intratumoral injection according to the drug components and doses in Table 5, and each group was dosed once. On the 7th day after drug administration, the animals were euthanized, and the tumor tissues were taken out after dissection to determine the tumor weight, the tumor inhibition rate (r) was calculated relative to the negative control group.

Table 5 Effect of different drug components and doses on tumor inhibition rate

| Groups | Description | Specific components | Injection volume | Tumor weight (x±s_g) | Tumor inhibition rate r |
|---|---|---|---|---|---|
| 0 | Negative control group | Physiological saline | 150ul | 0.216±0.127 | - |
| 01 | Local action control group | 50% ethanol | 150ul | 0.201±0.115 | 7.1% |
| 02 | | 75% ethanol | 107ul | 0.163±0.091 | 24.4% |
| 03 | | Anhydrous ethanol | 75ul | 0.131±0.074 | 39.3% |
| 1 | Disrupted Saccharomyces boulardii supernatant components | 0.1% supernatant components* | 500ul | 0.204±0.090 | 5.6% |
| 2 | | 0.25% supernatant components | 200ul | 0.175±0.065 | 19.1% |
| 3 | | 0.5% supernatant components | 100ul | 0.165±0.085 | 23.4% |

(continued)

| Groups | Description | Specific components | Injection volume | Tumor weight (x±s_g) | Tumor inhibition rate r |
|---|---|---|---|---|---|
| 4 | Heat inactivated Saccharomyces boulardii | 0.3% inactivated fungi** | 500ul | 0.198±0.077 | 8.3% |
| 5 | | 0.75% inactivated fungi | 200ul | 0.171±0.081 | 20.7% |
| 6 | | 1.5% inactivated fungi | 100ul | 0.161±0.093 | 25.6% |
| 7 | Probiotic water-insoluble component particle | 0.5% β-glucan particle*** | 500ul | 0.202±0.100 | 6.7% |
| 8 | | 1.25% β-glucan particle | 200ul | 0.170±0.069 | 21.1% |
| 9 | | 2.5% β-glucan particle | 100ul | 0.157±0.092 | 27.5% |
| 10 | A mixture of probiotic water-insoluble /water-soluble components | 0.25% disrupted Saccharomyces boulardii *** | 500ul | 0.195±0.097 | 9.6% |
| 11 | | 0.50% disrupted Saccharomyces boulardii | 200ul | 0.168±0.082 | 22.3% |
| 12 | | 1.25% disrupted Saccharomyces boulardii mixture | 100ul | 0.159±0.071 | 26.4% |

*: supernatant component was disrupted Saccharomyces boulardii supernatant component; **: Inactivated fungi were heat inactivated Saccharomyces boulardii; ***; Particles were Saccharomyces cerevisiae β-glucan particles; ****: Disrupted fungi were disrupted Saccharomyces boulardii

As could be seen from Table 5, ethanol in the technical solution of group 01 did not provide chemical ablation activity, and was only similar to intravenous ethanol injection; the tumor inhibition rates of groups 02 and 03 were close ($E_{03}/E_{02}$ < 200%) but much higher than that of group 01 ($E_{02}/E_{01}$ > 200%), showing the expected chemical ablation; Indicating that only ethanol having the concentration above a certain threshold (e.g., 70%) could be used as a chemical ablation agent.

Groups 1-3 were injected with the same dose of different concentrations of a probiotic component into the same volume of tumors, wherein the tumor inhibition rate of group 3 was not significantly different from that of group 02 ($E_3/E_{02}$=79%), indicating that it showed a similar local effect. Groups 5, 8, and 11 had similar local effects as that of group 02, while data of the tumor inhibition rates from groups 3, 6, 9, and 12 suggest the local effect occur in an administration concentration -dependent way.

Although groups 1 and 2 were administered intratumorally at the same dose, the difference in the pharmacological efficacy of the probiotic component at different administration concentrations exceeded the expected dynamic difference ($E_2/E_1$ > 200%). Therefore the administration concentration of the probiotic component in the pharmaceutical composition of the present application was the pharmacological concentration required for local effect but not for immune enhancement.

A necessary condition for the probiotic component to provide a local effect was that the probiotic component was present in the pharmaceutical composition in such content that its pharmacological concentration (local administration concentration) was > 0.1 %, ≥0.25%, 0.25-25 %, preferably 0.5-15 %, more preferably 1-15% or 5-15%. Wherein, when said probiotic component was inactivated probiotics, its pharmacological concentration was > 0.3 %, ≥0.75 %, 0.75-15 %, preferably 1.5-15% or 5-15%; when said probiotic component was probiotic water-soluble component, its pharmacological concentration was >0.1%, such as 0.15-25 %, preferably 0.35-25 % or 5-25% (wherein, when the water-soluble component was probiotic supernatant component, its pharmacological concentration was 0.35-3.5%; when the water-soluble component was water-soluble probiotic-derived polysaccharide, its pharmacological concentration was 2-5 % or 5-15%; when the water-soluble component was probiotic-derived ribonucleic acid, its pharmacological concentration was 15-25%); when the probiotic component was probiotic water-insoluble component particles, its pharmacological concentration was > 0.5% or 0.5-15%, preferably 1.5-15% or 5-15%; when said probiotic component was probiotic semi-fluid-like component, its pharmacological concentration was >2.5%, 2.6-25 %, preferably 5-15 %.

It was possible that the same substance if used as different active ingredients might need to meet different pharmacological dynamic profiles. The results of the aforementioned administration concentration-dependent comparative studies of pharmacological efficacy further confirmed that the local activity of the probiotic component described in the present invention was similar to chemical ablation and was far from conventional activity (cancer cell inhibition, tumor vascular inhibition, or immune enhancement, etc.). The pharmacological concentration of the probiotic component as the locally active ingredient described in the present application was not at all simply comparable to the formulation concentration or administration concentration of the probiotic component as the non-locally active ingredient in the prior art. The formulation concentration of a non-locally active drug was often limited only by pharmaceutics, such as a high concentration formulation to save transportation and storage costs, and an appropriately high concentration for injection to reduce injection volume to shorten dosing time. It was well known that formulation concentrations could range widely, but they were inherently different from the administration concentration required for pharmacological action of the drug. For example, when administered intravenously, the administration concentration upon dilution was much lower than the formulation concentration, to avoid safety risks arising from rapid entry of the drug into the bloodstream. The pharmacological concentration in the composition of the present invention was not only a characteristic limiting its composition and preparation, but it must also appear as a pharmacological condition in the New Drug Submission and as an administration condition in the instructions for use of the drug.

**Example 4: Pharmacological study of local action and optimization of pharmacological volume**

The component contents of pharmaceutical compositions of different pharmacology usually needed to be defined by different characteristics, and usually conventional pharmaceutical compositions did not have a volume dependent pharmacological volume in the lesion target region except of an administration dose. The following experiment investigated this.

Nude mice were used as experimental subjects, and human pancreatic cancer cells (PANC-1) were used as modeling cells, $1 \times 10^5$ cells/mouse were injected into the right axilla subcutaneously for transplantation tumor modeling, and the average tumor volume of successfully modeled nude mice was 213.1 mm$^3$. The model animals were randomly divided into 16 groups, and the drugs were administered intratumorally according to the drug components and administration volumes in Table 6, and each group was administered once. The drugs in groups 1-12 were prepared according to the method of Example 1, wherein the inactivated probiotics were heat-inactivated Saccharomyces boulardii, the probiotic water-insoluble component particles were β-glucan particles, the probiotic water-soluble component was disrupted Saccharomyces boulardii supernatant component, and the mixture of the probiotic water-insoluble component particles and water-soluble component (abbreviated as probiotic component mixture) was disrupted Saccharomyces boulardii, preparation method used was the same as that of Example 3. On the 7th day after drug administration, the animals were euthanized, and the tumor tissues were taken out after dissection to determine the tumor weight, and the tumor inhibition rate (r) was calculated relative to the negative control group.

Table 6 Data of tumor inhibition rate using different injection concentrations and volumes

| Groups | Injection concentration | Injection volume | Ratio of administration volume/tumor volume | Tumor weight (x±s_g) | Tumor inhibition rate r |
|---|---|---|---|---|---|
| 0 | (physiological saline) | 100ul | 0.50 | 0.274±0.124 | - |
| 01 | 75% ethanol | 20ul | 0.09 | 0.257±0.110 | 6.2% |
| 02 | 75% ethanol | 50ul | 0.23 | 0.218±0.115 | 20.3% |
| 03 | 75% ethanol | 100ul | 0.47 | 0.187±0.064 | 31.7% |
| 1 | 10% probiotic component mixture | 20ul | 0.09 | 0.259±0.091 | 5.6% |
| 2 | 4% probiotic component mixture | 50ul | 0.23 | 0.215±0.104 | 21.4% |
| 3 | 2% probiotic component mixture | 100ul | 0.47 | 0.193±0.083 | 29.7% |
| 4 | 10% inactivated probiotics | 20ul | 0.09 | 0.254±0.109 | 7.3% |
| 5 | 4% inactivated probiotics | 50ul | 0.23 | 0.220±0.081 | 19.8% |
| 6 | 2% inactivated probiotics | 100ul | 0.47 | 0.198±0.099 | 27.6% |

(continued)

| Groups | Injection concentration | Injection volume | Ratio of administration volume/tumor volume | Tumor weight (x±s_g) | Tumor inhibition rate r |
|---|---|---|---|---|---|
| 7 | 10% probiotic water-insoluble component particle | 20ul | 0.09 | 0.252±0.093 | 8.1% |
| 8 | 4% probiotic water-insoluble component particle | 50ul | 0.23 | 0.212±0.114 | 22.4% |
| 9 | 2% probiotic water-insoluble component particle | 100ul | 0.47 | 0.204±0.105 | 25.6% |
| 10 | 10% probiotic water-soluble component | 20ul | 0.09 | 0.264±0.087 | 3.7% |
| 11 | 4% probiotic water-soluble component | 50ul | 0.23 | 0.210±0.098 | 23.4% |
| 12 | 2% probiotic water-soluble component | 100ul | 0.47 | 0.195±0.077 | 28.7% |

As could be seen from Table 6, groups 01-03 injected ethanol into the same volume of tumor with different ratios of administration volume/target region volume, wherein ethanol in group 01 failed to provide chemical ablation, similar to intravenously injected ethanol; and the tumor inhibition rate of group 02 was extremely different from that of group 01 ($E_{02}/E_{01}$ > 200%), while the tumor inhibition rate of group 02 was not significantly different from that of group 03 ($E_{03}/E_{02}$ < 200%), showing the expected chemical ablation. This suggests that only ethanol exceeding a certain threshold of administration volume/tumor volume ratio, such as of 0.15, could be applied as a chemical ablation agent.

In groups 1 -3, a same dose of a probiotic component with different administration volume/target region volume ratios was injected into tumors having a same tumor volume, wherein the tumor inhibition rate of group 2 was similar to that of group 02 ($E_3/E_{02}$=105%), showing a similar local effect; and groups 5, 8, and 11 also had local effects similar to that of group 02, and groups 3, 6, 9, and 12 further demonstrated that the local effect was correlated with the target region volume and was dependent on the administration volume. In summary, even for higher concentrations of pharmaceutical composition administered intratumorally, the difference in drug efficacy of probiotic components with the same dose but different administration volume/target region volume ratio could far exceed the expected dynamic difference (e.g., E2 /E1 >200%)). In this regard, the administration volume of the probiotic component ((administration volume/target region volume ratio) was no longer a pharmacodynamics issue, but a pharmacological one.

Preferably, the content of the probiotic component in the pharmaceutical composition results in administration volume/target region volume ratio > 0.09, 0.1-1.5, preferably 0.23-1.5 or 0.5-1.5. Clinically, although many tumor volumes were ≥30cm$^3$, the administration volume of an immune-enhancing pharmacology-based drug within tumor region was generally very low (e.g. for cytokines, volume ≤ 2 ml); the size of such drug formulations were usually in small volume, such as vials for injection solution, or vials for powder-injection formulation. However, the pharmaceutical compositions described in the present application could only be administered under conditions that satisfy the above-mentioned administration volume/target region volume ratio, for example, if a tumor target volume was ≥ 30 cm$^3$ and an administration volume/target region volume ratio was 0.2, then an administration volume ≥6.0 cm$^3$ was required, thus the volume size of the drug formulation was 6 ml or an integral multiple thereof. It was known that in fact the drug size could also be one of the commonly used forms of the required content of an active ingredient to achieve the desired pharmacological effect, for example, "BAY ASPIRIN" tablets containing different contents of aspirin each has a different range of indications.

The above-described preferred embodiment in the present application for the probiotic component as a local active ingredient administered according to the lesion volume exceeded what was expected in the prior art for a probiotic component. Particularly, the probiotic component used as a non-local active ingredient (e.g., immune-enhancing active ingredient) in the prior art might be used as an adjunctive treatment but not as a primary drug for treating a local lesion disease, particularly not for a local lesion treatment that could reduce the local lesion volume, thus its administration volume in the prior art was not based on the local lesion volume. Furthermore, according to the results of the experiments in Example 2 above and other similar experiments, the safety volume (e.g., the administration volume in the event of death) for the same concentration of drug administered in the lesion could be more than two times higher than that administered by intravenous injection. Thus, the probiotic component described in the present application as a local active ingredient was particularly suitable for larger (e.g., average diameter greater than 3 cm) lesions.

The results of the aforementioned comparative study about the dependency of drug efficacy on the administration volume further confirmed that the local activity of the probiotic component described in the present invention was similar to chemical ablation and was far from conventional activities (cancer cell inhibition, tumor vascular inhibition, or immune enhancement, etc.). The pharmacodynamic profile of the probiotic component as a non-locally active ingredient in the prior art was characterized by blood drug concentration (usually very low, e.g. $0.25 \times 10^{-5}$%), and the administration volume was only related to the dose required for the blood drug concentration, but not to the volume of the lesion target region. Since the dosage was associated with systemic safety, the administration volume was then extensively faded out from the pharmacodynamic profile and was determined by the physician based on the patient's status such as body weight. The pharmacological volume of the probiotic component as a local active ingredient described in the present application was an important pharmacodynamic feature (relating to the pharmacological relationship between the effective penetration region and the drug-free region and its consequences), which not only limits its constitution (e.g. unit formulation volume) and preparation, but which must also appear in the new drug submission as a pharmacological condition and also in the instructions for use of the drug as an application condition. The pharmacological volume of the probiotic component as a local active ingredient described in the present application was not at all simply comparable to the unit formulation volume or administration volume of the probiotic component as a non-local active ingredient in the prior art.

Based on the results of above Examples 2-4 and more similar studies, the local pharmaceutical composition of the present invention comprising a probiotic component (the composition of the present invention) exceeded the expectations of a prior art non-local pharmaceutical composition comprising a probiotic component (a prior art composition, e.g., comprising a probiotic component that could be used for oral or intravenous infusion) in terms of

1) its fundamental pharmacology exceeded expectations: the probiotic component as a locally active ingredient in the composition of the present invention was based on a discovery of its new pharmacology - the tissue-destroying effect in the drug penetration region - i.e. the local effect, which mainly included the local chemical action (e.g. chemical ablation-like), exceeding the pharmacological expectations of any non-local activity of a prior art probiotic component (e.g. immunological effect, antiviral effect, tumor cytotoxic effect, or tumor vascular disruption effect, etc.).

2) its pharmacological method exceeded expectations: the probiotic component in the composition of the present invention must be strictly limited to local administration in order to enter the target region reactor to achieve its new function, while the pharmacological method of the probiotic component in the prior art composition was not limited to local administration, but preferably systemic administration to achieve its target function.

3) the active ingredient selection scheme exceeded expectations: the probiotic component in the prior art immune enhancing composition was preferably a stronger bacterial immune component (such as live probiotics and a component with the highest possible morphological proximity, such as an inactivated probiotics that basically retains live probiotic morphology), while the composition of the present invention was preferably an immune minimizing bacterial component (a component with the lowest possible morphological proximity to live bacteria, such as a probiotic water-soluble component, a probiotic semi-fluid component).

4) its pharmacodynamic scheme exceeded expectations: the dynamic conditions of the probiotic component in the prior art composition to achieve its systemic effect (pharmacological concentration was blood drug concentration) depended on the administration dose rather than the administration concentration, not to mention the formulation concentration (usually prepared as a concentrate for the convenience of preparation, storage and transportation), and the dilution of the formulation to be administered in order to avoid, for example, the local effect damage of the injection site. However, the dynamic conditions of the probiotic component in the composition of the present invention to achieve its local effect were, on the contrary, dependent on the administration concentration rather than the administration dose (blood drug concentration), and the local administration concentration was its pharmacological concentration and often also its formulation concentration. Moreover, the administration volume of prior art compositions was related only to the dose of blood drug concentration required for systemic pharmacology and not to the volume of the lesion target region, whereas the administration volume of the compositions of the present invention was related to the volume of the target region to be penetrated for their local activity.

5) the time required for its pharmacological response exceeded expectations: the systemic activity of the prior art compositions must be achieved by a prolonged high frequency (e.g. tens of times in 3 months) of pharmacological response in the target region, and accordingly the prior art compositions must be supplied at high frequency and for a long time, whereas the local activity of the compositions of the present invention was similar to chemical ablation, which could be achieved by only a few times (e.g. $\leqq$ 3 times in 3 months) of pharmacological response in the administration region. This also brought a significant difference in dosage form (e.g. tens of injections vs. several injections) and preparation protocol.

6) the reaction environment required for its pharmacological reaction exceeded expectations: the composition of the invention was strictly limited to the corresponding constitutions of dosage form for local administration (it should not comprise certain excipients in the dosage form for the systemic delivery, a solvent which is uniform required a well-mixed mixture when composition is administered, etc.), etc.

7) its technical effect exceeded expectations: first, the therapeutic effect on local lesions exceeded expectations. For example, at least in the administration region, the drug effect of the invention ($r_{composition\ of\ the\ invention}/r_{prior\ art\ composition} > 200\%$, preferably $r_{composition\ of\ the\ invention}/r_{prior\ art\ composition} > 400\%$) that was comparable to recognized effective drugs and substantially greater than that expected in the short term (e.g. 21 days after the first dose in the case of multiple doses, or 7 days after the last dose) for prior art compositions can be obtained. This drug efficacy profile also led to the range of indications beyond what would be expected, for example, in immunodeficient patients as represented by the animal models described above, in elderly patients, in patients having a reduced immunity after various treatments. These also further indicated that the differences between the two compositions were not dynamic but pharmacological difference (e.g., local effect vs. conventional effect).

**Example 5: Pharmacological studies of co-administration in immunosuppressed animal models and preferred technical solutions**

Nude mice were used as experimental subjects, and lung tumor cells (A549) were used as modeling cells, $1 \times 10^5$ cells/mouse were injected into the right axilla subcutaneously for transplantation tumor modeling, and the average tumor volume of successfully modeled nude mice was 153.7 mm$^3$. The model animals were randomly divided into 18 groups, and the drugs were administered according to the drug components and administration route in Table 7. Each group was dosed once with an injection volume of 150 $\mu$l/mouse. Wherein, the probiotic component was heat-inactivated Saccharomyces boulardii, and the administration route of group 16 was intra-tumoral injection of probiotic component followed by intra-tumoral injection of 10% arginine about 2 hours later. On the 7th day after drug administration, the animals were euthanized, and after dissection, the tumor tissue was taken out to determine the tumor weight, and the tumor inhibition rate (r) was calculated relative to the negative control group. Further, the actual efficacy/expected efficacy ratio q of a drug could also be calculated according to the therapeutic effect (tumor inhibition rate).

Table 7 Effect of different components and administration routes on tumor inhibition rate

| Groups | Drug | Administration route | Tumor weight (x±s_ g) | Tumor inhibition rate r |
|---|---|---|---|---|
| 01 | physiological saline | intravenous injection | 0.209±0.125 | |
| 1 | 75% ethanol | | 0.203±0.095 | 2.7% |
| 2 | 1% 5-fluorouracil | | 0.122±0.065 | 41.3% |
| 3 | 10% arginine | | 0.196±0.077 | 6.4% |
| 4 | 2.5% probiotic component | | 0.200±0.114 | 4.7% |
| 5 | 2.5% probiotic component/75% ethanol | | 0.201±0.107 | 3.8% |
| 6 | 2.5% probiotic component/1% 5-fluorouracil | | 0.117±0.091 | 44.1% |
| 7 | 2.5% probiotic component/10% arginine | | 0.204±0.103 | 2.6% |

(continued)

| Groups | Drug | Administration route | Tumor weight (x±s_ g) | Tumor inhibition rate r |
|---|---|---|---|---|
| 02 | physiological saline | intratumoral injection | 0.218±0.115g | |
| 8 | 75% ethanol | | 0.145±0.084 | 33.7% |
| 9 | 1% 5-fluorouracil | | 0.116±0.076 | 46.6% |
| 10 | 10% arginine | | 0.164±0.061 | 24.7% |
| 11 | 2.5% probiotic component | | 0.170±0.099 | 21.8% |
| 12 | 2.5% probiotic component/75% ethanol | | 0.118±0.062 | 45.7% |
| 13 | 2.5% probiotic component/1% 5-fluorouracil | | 0.070±0.045 | 68.1% |
| 14 | 2.5% probiotic component/10% arginine | | 0.092±0.027 | 57.9% |
| 15 | 2.5% probiotic component/1% 5-fluorouracil /10% arginine | | 0.018±0.039 | 91.6% |
| 16 | 2.5% probiotic component+10% arginine | | 0.167±0.083 | 23.3% |

As could be seen from the above Table, the ratio q of actual co-administration effect/the expected theoretical purely additive effect for groups 5, 6 and 7 were not greater than 1.00, indicating that in the circumstances the probiotic component and the chemical active substance co-administered via intravenous injection failed to produce any synergy.

Group 12 of the same probiotic component/chemical active substance (ethanol) mixture via a different administration route showed much higher drug efficacy than that of group 5, but their actual co-administration effect/ expected theoretical purely additive effect ratios q were not greater than 1.00, thus this high drug efficacy was mainly due to the local effect of ethanol (group 8). It was expected that the same probiotic component should have similar pharmacological behavior when combined with other chemical active substances. However, groups 13 and 14 showed significantly higher drug efficacy than those of groups 6 and 7 (using the same probiotic component/chemical active substance mixture, respectively) respectively, indicating that their higher drug efficacies were directly related to their local effects. Moreover, the actual co-administration effect/ expected theoretical purely additive effect ratios q in both groups 13 and 14 were greater than 1.00, indicating that the local effects were local synergy of the probiotic component and these chemical active substances.

Further, group 15 had the highest drug potency and its actual co-administration effect/ expected theoretical purely additive effect ratio q > 1.00, indicating that the three-component composition (probiotic component/weak local acting compound/cytotoxic drug) could actually further produce synergy based on the synergy of its two components (probiotic component/weak local acting compound, and probiotic component//cytotoxic drug). Finally, the pharmaceutical composition of group 16, which was not in the same formulation despite of having the same components as those of group 14, did not show synergy with its actual co-administration effect/ expected theoretical purely additive effect ratio q < 1.00.

In the experiment, it was observed that most of the experimental animals in groups 11 and 12 produced stronger response after injection, indicating that a probiotic component alone or a non-synergistic pharmaceutical composition should be used under analgesic conditions to avoid safety risks; however, no strong irritation response as described above was observed in groups 13-15, indicating that, the synergistic pharmaceutical compositions of a probiotic component and a weak local acting compound and/or a cytotoxic drug had superior safety to that expected from the single drug efficacy. The same results existed for other probiotic component, such as probiotic semi-fluid component, probiotic water-soluble component, and probiotic water-insoluble component particles and the like, in combination with a chemical drug, which were not detailed here due to the limitation of the length of the specification.

The applicant conducted in-depth research on compositions of probiotic components, especially compositions formed by three components.

Nude mice were used as experimental subjects, and gastric tumor cells (BGC823) were used as modeling cells, $1 \times 10^5$ cells/ mouse were injected into the right axilla subcutaneously for transplantation tumor modeling, and the average tumor volume of successfully modeled nude mice was 164.4 mm$^3$. The model animals were randomly divided into 14 groups, and the drugs according to the pharmacological constitutions in Table 8 were administered via intratumoral injection. Each group was dosed once with an injection volume of 150 μl/mouse; wherein the pharmaceutical compositions of groups 5, 11 were semi-fluid, the pharmaceutical compositions of other groups are liquid. On the 7th day after drug administration, the animals were euthanized, and after dissection, the tumor tissue was taken out to determine the tumor weight, and the tumor inhibition rate (r) was calculated relative to the negative control group. Further, the actual efficacy/expected efficacy ratio q of a drug could also be calculated according to the therapeutic effect (tumor inhibition rate).

Table 8 Tumor inhibition results of different drug combinations

| groups | drug types | Specific components | tumor weight (x±s_g) | tumor inhibition rate r |
|---|---|---|---|---|
| 01 | negative control | physiological saline | 0.225±0.127 | - |
| 1 | a weak local acting compound | 1% methylene blue | 0.197±0.062 | 12.5% |
| 2 | cytotoxic drug | 1% 5-fluorouracil | 0.132±0.069 | 41.4% |
| 3 | a weak local acting compound and a cytotoxic drug | 1% methylene blue /1% 5-fluorouracil | 0.072±0.034 | 68.1% |
| 4 | inactivated probiotics | 5% heat inactivated Saccharomyces boulardii | 0.145±0.088 | 35.7% |
| 5 | a probiotic semi-fluid-like component | 7.5% β-glucan semi-fluid | 0.150±0.095 | 33.2% |
| 6 | probiotic water-soluble component | 5% disrupted Saccharomyces boulardii supernatant components | 0.140±0.076 | 37.7% |
| 7 | probiotic component mixture | 5% disrupted Saccharomyces boulardii | 0.147±0.061 | 34.6% |
| 8 | inactivated probiotics/ a weak local acting compound | 5% heat inactivated Saccharomyces boulardii /1% methylene blue | 0.087±0.029 | 61.4% |
| 9 | inactivated probiotics/ cytotoxic drug | 5% heat inactivated Saccharomyces boulardii /1% 5-fluorouracil | 0.059±0.051 | 73.2% |
| 10 | inactivated probiotics/ a weak local acting compound and cytotoxic drug | 5% heat inactivated Saccharomyces boulardii /1% methylene blue /1% 5-fluorouracil | 0.005±0.038 | 97.7% |
| 11 | a probiotic semi-fluid-like component/a weak local acting compound and cytotoxic drug | 7.5% β-glucan /1% methylene blue /1% 5-fluorouracil | 0.010±0.019 | 95.6% |
| 12 | probiotic water-soluble component/ a weak local acting compound and cytotoxic drug | 5% disrupted Saccharomyces boulardii supernatant components/1% methylene blue /1% 5-fluorouracil | 0.013±0.042 | 94.3% |
| 13 | probiotic component mixture/ weak local acting compound and cytotoxic drug | 5% disrupted Saccharomyces boulardii /1% methylene blue /1% 5-fluorouracil | 0.003±0.065 | 98.7% |

When injected intratumorally, the ratios q of actual co-administration effect/ expected theoretical purely additive effect for groups 8-13 were all greater than 1.00, all showing significant synergy. The applicant further investigated the drug efficacy of this new function. Nude mice were used as experimental subjects, and randomly divided into 3 groups, with 6 mice in each group, using 75% ethanol aqueous solution, 5% heat inactivated Saccharomyces boulardii /1% methylene blue, and 7.5% β-glucan/1% methylene blue/1% 5-fluorouracil in a semi-fluid composition as the study drugs, respectively. Each group was dosed once with an injection volume of 100 μl/mouse by administering to the outer lateral muscle mass of the right leg. On the 7th day after drug administration, the animals were euthanized, and the specimens of the outer lateral muscle mass of the right leg were taken out after dissection to perform a general pathological analysis. The sections were measured for the area of abnormal regions that distinguished from normal muscle, such as necrosis and nodules. The results showed that the area of abnormal regions in groups 1, 2 and 3 were $32.24\pm13.71$ mm$^2$, $46.78\pm13.64$ mm$^2$ and $72.35\pm23.71$ mm$^2$, respectively, indicating that the local synergy provided by the probiotic components was similar, which was mainly a local chemical synergy, and stronger than that of ethanol.

**Example 6: Pharmacological study and optimization in a non-tumor local lesion model**

Nude mice were used as experimental subjects, and mouse embryonic fibroblasts 3T3 were used as modeling cells. $2\times10^4$ cells/mouse were injected into the right axilla subcutaneously for transplantation nodule modeling, and the average volume of tumor-like nodules in successfully modeled nude mice was 171.5mm$^3$. The model animals were randomly divided into 16 groups, and the drugs were administered according to Table 9. Each group except for groups 21-24 were administered by intraperitoneal injection, intra-nodal injection. The drugs used in groups 21-24 were a composition of non-local administration dosage form (dosage form that a probiotic component and a co-administration substance could be administered separately). The administration route of series A was: intraperitoneal injection of probiotic component + intra-nodal injection of co-administration substance; and the administration route of series B was: intraperitoneal injection of co-administration substance + intra-nodal injection of probiotic component. Each group was dosed twice at an interval of 2 days, with an injection volume of 150 µl/mouse/time. On the 7th day after the final administration, the volume (V) of local lesions comprising fibroblasts was measured, and the relative local lesion inhibition rates ($r'_A$, $r'_B$)were calculated for series A and series B, respectively, as compared with the negative control group. Further, the actual drug efficacy /expected efficacy ratio q could also be calculated according to the therapeutic effect (local lesion inhibition rate).

Table 9 Data of relative local lesion inhibition rate of different drugs and administration routes

| Groups | Drug category | Specific components | $r'_A$ | | $r'_B$ | |
|---|---|---|---|---|---|---|
| | | | V (x±s cm$^3$) | r' | V (x±s cm$^3$) | r' |
| 01 | negative control | physiological saline | 1.357±0.208 | 0 | 1.431±0.194 | 0 |
| 02 | cytotoxic positive control | 1% 5-fluorouracil aqueous solution | 1.225±0.211 | 9.7% | 1.226±0.217 | 14.3% |
| 03 | immune-enhancement positive control | medical Lentinan injection liquid (Jinling Pharmaceutical Co., Ltd.) | 1.288±0.193 | 5.1% | 1.332±0.211 | 6.9% |
| 04 | non-specific cytotoxic positive /Local action positive control | anhydrous ethanol | 1.300±0.197 | 4.2% | 1.078±0.221 | 24.7% |
| 1 | inactivated probiotics | 5% heat inactivated Saccharomyces boulardii aqueous suspension | 1.278±0.201 | 5.8% | 1.123±0.216 | 21.5% |
| 2 | probiotic component semi-fluid | semi-fluid formed after heat treatment and cooling treatment of 5% β-glucan | 1.295±0.207 | 4.6% | 0.990±0.193 | 30.8% |
| 3 | probiotic water-soluble component 1 | 5% water-soluble Saccharomyces cerevisiae β-glucan aqueous solution | 1.310±0.213 | 3.5% | 0.985±0.207 | 31.2% |
| 4 | probiotic water-soluble component 2 | 20% Saccharomyces cerevisiae ribonucleic acid aqueous solution | 1.320±0.179 | 2.7% | 0.943±0.195 | 34.1% |
| 11 | dye | 1% methylene blue aqueous solution | 1.288±0.205 | 5.1% | 1.020±0.210 | 28.7% |
| 12 | dye/cytotoxic drug | 1% methylene blue /1% 5-fluorouracil aqueous solution | 1.229±0.191 | 9.4% | 0.839±0.189 | 41.4% |

(continued)

| Groups | Drug category | Specific components | $r'_A$ | | $r'_B$ | |
|---|---|---|---|---|---|---|
| | | | V ($x \pm s$ cm$^3$) | r' | V ($x \pm s$ cm$^3$) | r' |
| 21 | inactivated probiotics+dye/ cytotoxic drug | 5% heat inactivated Saccharomyces boulardii aqueous suspension +1% methylene blue /1% 5-fluorouracil aqueous solution | 0.810±0.137 | 40.3% | 1.056±0.147 | 26.2% |
| 22 | probiotic component semi-fluid +dye/ cytotoxic drug | semi-fluid formed after heat treatment and cooling treatment of 5% β-glucan+1% methylene blue /1% 5-fluorouracil aqueous solution | 0.790±0.142 | 41.8% | 1.013±0.151 | 29.2% |
| 23 | probiotic water-soluble component 1+ dye/ cytotoxic drug | 5% yeast β-glucan aqueous solution+1% methylene blue /1% 5-fluorouracil aqueous solution | 0.818±0.151 | 39.7% | 1.078±0.161 | 24.7% |
| 24 | probiotic water-soluble component 2+dye/ cytotoxic drug | 20% Saccharomyces cerevisiae ribonucleic acid aqueous solution+1% methylene blue /1% 5-fluorouracil aqueous solution | 0.824±0.149 | 39.3% | 0.920±0.157 | 35.7% |
| 31 | inactivated probiotics/dye/ cytotoxic drug | 5% heat inactivated Saccharomyces boulardii /1% methylene blue /1% 5-fluorouracil aqueous suspension | 1.247±0.186 | 8.1% | 0.448±0.193 | 68.7% |
| 32 | probiotic component semi-fluid /dye/ cytotoxic drug | semi-fluid formed after heat treatment and cooling treatment of 5% β-glucan /1% methylene blue /1% 5-fluorouracil aqueous suspension | 1.219±0.179 | 10.2% | 0.341±0.173 | 76.2% |
| 33 | probiotic water-soluble component 1/dye/ cytotoxic drug | 5% water-soluble Saccharomyces cerevisiae β-glucan /1% methylene blue /1% 5-fluorouracil aqueous solution | 1.224±0.203 | 9.8% | 0.202±0.217 | 85.9% |
| 34 | probiotic water-soluble component 2/dye/ cytotoxic drug | 20% Saccharomyces cerevisiae ribonucleic acid /1% methylene blue /1% 5-fluorouracil aqueous solution | 1.250±0.197 | 7.9% | 0.283±0.219 | 80.2% |

In the Example, 5-fluorouracil in group 02 was administered at a dose effective against the tumor body as a cytotoxic positive control; Lentinan injection liquid in group 02 was administered at 10 times the maximum clinical dose as an immune enhancing control, and anhydrous ethanol in group 04 was used as a non-specific cytotoxic positive/local effect positive control, said anhydrous ethanol showing non-specific cytotoxic positive under non-clinical conditions and only local effect positive under clinical conditions, wherein said clinical conditions were conventional conditions acceptable in terms of clinical safety and compliance, such as less than 10 injections in a course of treatment (40 days), while said non-clinical conditions were conventional conditions which were far from clinical safety and compliance, such as cytometric conditions or greater than 10 injections in a course of treatment (40 days).

As could be seen from the above Table, the tumor inhibition rate of the drug in group 03 was less than 15% in both series A and B, which was consistent with the pharmacological expectation that the drug was only used as an adjuvant drug for immune enhancement. The tumor inhibition rate of the drug in group 04 via intraperitoneal injection was r < 15%, while tumor inhibition rate via intra-tumoral injection was r > 15%, showing a certain therapeutic effect, which were in line with the expectations that the drug may exhibit non-specific cytotoxic effect pharmacology under extreme conditions, but only local effect pharmacology under normal conditions ($E_{B04}/E_{A04}$ > 200%). The tumor inhibition rate of the drug in group 02 via intraperitoneal injection was r < 15%, significantly less than its drug efficacy in Example 2, in line with the pharmacological expectation of cytotoxic effect, while the drug efficacy was significantly higher via intra-tumoral injection ($E_{B02}/E_{A02}$ > 200%, showing the pharmacological expectations that it had a local effect similar to ethanol and an effect beyond of cytotoxic effect.

The drug efficacy of the drugs in groups 1-4 via intraperitoneal injection were consistent with those of groups 01-04, and the possible immune enhancement effect, cytotoxic effect, or any other effects provided via systemic administration, of the probiotic component did not exceed the pharmacological expectations of the control drug 01-04. However, the efficacy of the drugs via intra-tumoral injection in groups 1-4 were much more potent than that via intraperitoneal injection ($E_B/E_A$ > 200%), presenting a significantly different and novel pharmacology. In fact, the efficacy of the drugs in groups 1-4 were similar to those of the control drugs 01-04, showing only local effects under conventional conditions. Similarly, the drugs in groups 11 and 12 also showed similar potency and pharmacology to the control drug 04.

The compositions via different administration routes in groups 21-24 showed that q values of intratumoral injection, intraperitoneal injection were not greater than 1.0, indicating that the combination efficacy of the composition (X+Y) formed by the probiotic component (X) and the co-administration substance (Y) in the non-local administration dosage form via administration in different ways did not exceed the expected additive efficacy of single drugs, and there was no synergy when the two drugs were co-administered; while in groups 31-34, when the local administration dosage form (mixed co-administration drug) with the same composition (X, Y) was administered via intraperitoneal injection, q values were not greater than 1.0; and via intra-lesional injection, q values were greater than 1.00, showing local synergy.

**Example 7: Pharmacological study and amount ratio optimization of local synergy**

Nude mice were used as experimental subjects, and human hepatoma cells (HepG2) were used as modeling cells. $1 \times 10^5$ cells/mouse were injected into the right axilla subcutaneously for transplantation tumor modeling, and the average tumor volume of successfully modeled nude mice was 155.7 mm$^3$. The model animals were randomly divided into 13 groups, and the drugs were administered according to Table 10. The relevant drugs were all aqueous liquids, which were formulated according to the method of Example 1. Each group was dosed once with an injection volume of 150 μl/mouse/time. On the 7th day after drug administration, the animals were euthanized, and the tumor tissues were taken out after dissection to determine the tumor weight, and the tumor inhibition rate was calculated relative to the negative control group. Further, the actual efficacy/expected efficacy ratio q of a drug could also be calculated according to the therapeutic effect (tumor inhibition rate).

Table 10 Data of tumor weight and tumor inhibition rate from different groups

| Groups | Research drugs | tumor weight (x±s_g) | tumor inhibition rate |
|---|---|---|---|
| 01 | (physiological saline) | 0.261±0.098 | - |
| 1 | 0.2% probiotic component | 0.231±0.113 | 11.5% |
| 2 | 0.5% probiotic component | 0.208±0.109 | 20.3% |
| 3 | 10% probiotic component | 0.149±0.091 | 43.1% |
| 4 | 20% probiotic component | 0.127±0.085 | 51.4% |
| 5 | 10% sodium bicarbonate /4.3% sodium hydroxide | 0.032±0.021 | 87.6% |
| 6 | 7% sodium bicarbonate /3% sodium hydroxide | 0.064±0.036 | 75.4% |
| 7 | 0.7% sodium bicarbonate /0.3% sodium hydroxide | 0.175±0.088 | 33.1% |
| 8 | 0.2% sodium bicarbonate /0.09% sodium hydroxide | 0.228±0.104 | 12.5% |
| 9 | 0.2% probiotic component/10% sodium bicarbonate /4.3% sodium hydroxide | 0.038±0.044 | 85.6% |
| 10 | 0.5% probiotic component/7% sodium bicarbonate /3% sodium hydroxide | 0.013±0.012 | 95.1% |
| 11 | 10% probiotic component/0.7% sodium bicarbonate /0.3% sodium hydroxide | 0.048±0.018 | 81.7% |

(continued)

| Groups | Research drugs | tumor weight (x±s_g) | tumor inhibition rate |
|---|---|---|---|
| 12 | 20% probiotic component/0.2% sodium bicarbonate /0.09% sodium hydroxide | 0.096±0.079 | 63.4% |

Sodium bicarbonate-sodium hydroxide buffer system reduced the solution of sodium hydroxide alone from pH >12.5 to about 11, and it was generally believed that the pH reduction was not conducive to local chemical effect. From the above Table, it could be seen that for group 9, the actual combination effect/expected theoretical purely additive effect ratio q < 1.00, thus the pharmaceutical composition therein did not show synergy, while the pHs of the pharmaceutical compositions in groups 10, 11 and 12 were pH 10 $\pm$ 1.0 or pH 4 $\pm$ 1.0, respectively, and their actual combination effect/expected theoretical purely additive effect ratios q > 1.00, all of them showed synergy with a synergistic amount ratio (W $_{probiotic\ component}$/W $_{co-administration\ substance}$) of (0.2-20)/(0.29-14.3).

Nude mice were used as experimental subjects, and human hepatoma cells (HepG2) were used as modeling cells. $1 \times 10^6$ cells/ mouse were injected into the right axilla subcutaneously for transplantation tumor modeling, and the average tumor volume of successfully modeled nude mice was 163.2 mm$^3$. The model animals were randomly divided into 13 groups, and the drugs were administered according to Table 11. The relevant drugs were aqueous liquids, which were formulated according to the method of Example 1, wherein the probiotic component was inactivated Saccharomyces cerevisiae, and the weak local effect compound was methylene blue. Each group was dosed twice with an interval of 7 days, and the injection volume was 150 $\mu$l/mouse/time. On the 7th day after final administration, the animals were euthanized, and the tumor tissues were taken out after dissection to determine the tumor weight, and the tumor inhibition rate was calculated relative to the negative control group.

Table 11 Data of tumor weight and tumor inhibition rate of different groups

| Group | Research drugs | Tumor weight (x±s_g) | Tumor inhibition rate |
|---|---|---|---|
| 01 | (physiological saline) | 0.253±0.117 | - |
| 1 | 0.2% probiotic component | 0.240±0.109 | 5.3% |
| 2 | 0.5% probiotic component | **0.207**±0.105 | 18.1% |
| 3 | 10% probiotic component | 0.155±0.070 | 38.6% |
| 4 | 20% probiotic component | 0.144±0.081 | 43.1% |
| 5 | 3% methylene blue | 0.073±0.033 | 71.1% |
| 6 | 1% methylene blue | **0.186**±0.096 | 26.4% |
| 7 | 0.50% methylene blue | 0.208±0.115 | 17.7% |
| 8 | 0.15% methylene blue | 0.227±0.107 | 10.1% |
| 9 | 0.2% probiotic component/3% methylene blue | 0.063±0.026 | 75.2% |
| 10 | 0.5% probiotic component/1% methylene blue | 0.112±0.094 | 55.9% |
| 11 | 10% probiotic component/0.50% methylene blue | 0.071±0.031 | 71.8% |
| 12 | 20% probiotic component/0.15% methylene blue | 0.111±0.062 | 56.1% |

From the above Table, it could be seen that the composition in group 9 did not show synergy (q<1.00), while the compositions in groups 10, 11 and 12 showed synergy (q >1.00, respectively), and their synergistic amount ratio (W $_{probiotic\ component}$/W $_{co-administration\ substance}$) was (0.2-20)/(0.15-1.0), indicating that the probiotic component provided local chemical synergy, regardless of the strength of the local chemical action provided by a weak local acting compound.

Nude mice were used as experimental subjects, and human hepatoma cells (HepG2) were used as modeling cells. $1 \times 10^5$ cells/mouse were injected into the right axilla subcutaneously for transplantation tumor modeling, and the average tumor volume of successfully modeled nude mice was 168.7 mm$^3$. The model animals were randomly divided into 13 groups, and the drugs were administered according to Table 12. The relevant drugs were aqueous liquids, which were formulated according to the method of Example 1, wherein the probiotic component was the supernatant component of disrupted Saccharomyces cerevisiae, and the weak local acting compound was lysine. Each group was dosed twice at an interval of 7 days with an injection volume of 150 µl/mouse/time. On the 7th day after final administration, the animals were euthanized, and the tumor tissues were taken out after dissection to determine the tumor weight, and the tumor inhibition rate was calculated relative to the negative control group.

Table 12 Data of tumor weights and tumor inhibition rates of different groups

| Group | Research drugs | tumor weight (x±s_g) | tumor inhibition rate |
|---|---|---|---|
| 01 | (physiological saline) | 0.238±0.191 | - |
| 1 | 0.2% probiotic component | 0.230±0.106 | 3.2% |
| 2 | 0.5% probiotic component | 0.199±0.099 | 16.4% |
| 3 | 10% probiotic component | 0.126±0.061 | 47.1% |
| 4 | 20% probiotic component | 0.111±0.065 | 53.4% |
| 5 | 25% lysine | 0.109±0.074 | 54.1% |
| 6 | 20% lysine | 0.134±0.073 | 43.6% |
| 7 | 5% lysine | 0.211±0.091 | 11.3% |
| 8 | 1% lysine | 0.226±0.112 | 5.2% |
| 9 | 0.2% probiotic component/25% lysine | 0.093±0.020 | 61.1% |
| 10 | 0.5% probiotic component/20% lysine | 0.076±0.038 | 67.9% |
| 11 | 10% probiotic component/5% lysine | 0.065±0.026 | 72.6% |
| 12 | 20% probiotic component/1% lysine | 0.094±0.055 | 60.3% |

As could be seen from the above table, the composition in group 9 did not show synergy (q>1.00), while the compositions in groups 10, 11 and 12 showed significant synergy (q>1.00, respectively) and their synergistic amount ratio ($W_{probiotic\ component}/W_{co-administration\ substance}$) was (0.2-20)/(1-25), again indicating that the probiotic component provided local chemical synergy, regardless of the strength of the local chemical action provided by a weak local acting compound.

Nude mice were used as experimental subjects, and human pancreatic cancer cells (PANC-1) were used as modeling cells. $1 \times 10^5$ cells/mouse were injected into the right axilla subcutaneously for transplantation tumor modeling, and the average tumor volume of successfully modeled nude mice was 157.3 mm$^3$. The model animals were randomly divided into 13 groups, and the drugs were administered according to Table 13. The relevant drugs were aqueous liquids, which were formulated according to the method of Example 1, wherein the probiotic component was the supernatant component of disrupted Saccharomyces cerevisiae, and the chemotherapeutic drug was gemcitabine. Each group was dosed once with an injection volume of 100 µl/mouse /time. On the 7th day after administration, the animals were euthanized, and the tumor tissues were taken out after dissection to determine the tumor weight, and the tumor inhibition rate was calculated relative to the negative control group.

Table 13 Data of tumor weights and tumor inhibition rates of different groups

| Group | Research drugs | Tumor weight (x±s_g) | Tumor inhibition rate |
|---|---|---|---|
| 01 | (physiological saline) | 0.294±0.189 | - |
| 1 | 0.2% probiotic component | 0.284±0.115 | 3.3% |
| 2 | 0.5% probiotic component | 0.278±0.109 | 5.6% |
| 3 | 10% probiotic component | 0.161±0.080 | 45.1% |
| 4 | 20% probiotic component | 0.140±0.096 | 52.4% |
| 5 | 5% gemcitabine | 0.191±0.077 | 35.1% |
| 6 | 2.5% gemcitabine | 0.204±0.103 | 30.4% |
| 7 | 0.1% gemcitabine | 0.271±0.114 | 7.8% |
| 8 | 0.05% gemcitabine | 0.278±0.101 | 5.6% |

(continued)

| Group | Research drugs | Tumor weight (x±s_g) | Tumor inhibition rate |
|---|---|---|---|
| 9 | 0.2% probiotic component/5% gemcitabine | 0.172±0.062 | 41.4% |
| 10 | 0.5% probiotic component/2.5% gemcitabine | 0.107±0.069 | 63.7% |
| 11 | 10% probiotic component/0.1% gemcitabine | 0.101±0.070 | 65.6% |
| 12 | 20% probiotic component/0.05% gemcitabine | 0.117±0.073 | 60.2% |

Similarly, the composition in group 9 did not show synergy (q>1.00), while the compositions in groups 10, 11 and 12 showed significant synergy (q>1.00, respectively) and their synergistic amount ratio (W $_{probiotic\ component}$/W $_{co-administration\ substance}$) was (0.2-20)/(0.05-2.5), also indicating that the probiotic component provided local chemical synergy, regardless of the strength of the local chemical action provided by a weak local acting compound.

The synergistic amount ratio of probiotic components in said pharmaceutical composition was not required for immune enhancing synergy, but for local synergy, thus was a pharmacological amount ratio. Preferably, the pharmacological amount ratio of the probiotic component to the chemical active compound, (W $_{probiotic\ component}$/W $_{chemical\ active\ compound}$) was (1-110)/(1-100). If said chemical active compound was a cytotoxic drug, then (W $_{probiotic\ component}$/W $_{cytotoxic\ drug}$) was (1-110)/(1-100); If said chemical active compound was a weak local acting compound, then (W $_{probiotic\ component}$/W $_{weak\ local\ acting\ compound}$) was (1-90)/(1-100); If said weak local acting compound was an amino acid nutrient, then W $_{probiotic\ component}$/W $_{amino\ acid\ nutrient}$ was (1-20)/(1-100); If said weak local acting compound was a vital dye, then W $_{probiotic\ component}$/W $_{vital\ dye}$ was (7-90)/(1-100); If said weak local acting compound was an acidifier and/or a basifier, then W $_{probiotic\ component}$/W $_{acidifier\ or/and\ a\ basifier}$ was (2-60)/(1-100).

**Example 8: Pharmacological study of local synergy and optimization of pharmacological concentration**

Nude mice were used as experimental subjects, and human pancreatic cancer cells (PANC-1) were used as modeling cells. $1 \times 10^6$ cells/mouse were injected into the right axilla subcutaneously for transplantation tumor modeling, and the average tumor volume of successfully modeled nude mice was 152.8 mm$^3$. The model animals were randomly divided into 10 groups, and the drugs were administered by intratumoral injection according to the drugs and doses in Table 14. The relevant drugs were liquid formulation, wherein the probiotic component was the supernatant component of disrupted Saccharomyces cerevisiae, and the chemical active compound was arginine, which were formulated according to the method of Example 1. Each group was dosed once. On the 7th day after administration, the animals were euthanized, and the tumor tissues were taken out after dissection to determine the tumor weight, and the tumor inhibition rate was calculated relative to the negative control group.

Table 13 Data of tumor weights and tumor inhibition rates of different drugs and doses

| Groups | Drugs | Injection volume μl | Tumor weight (x±s_g) | Tumor inhibition rate |
|---|---|---|---|---|
| 0 | physiological saline | 100 | 0.246±0.123 | - |
| 1 | 0.1% probiotic component | 100 | 0.218±0.096 | 11.3% |
| 2 | 0.25% probiotic component | 200 | 0.189±0.069 | 23.1% |
| 3 | 0.5% probiotic component | 500 | 0.169±0.088 | **31.4%** |
| 4 | 2% arginine | 100 | 0.226±0.104 | 8.1% |
| 5 | 5% arginine | 200 | 0.210±0.113 | 14.7% |
| 6 | 10% arginine | 500 | 0.193±0.075 | 21.4% |
| 7 | 0.1% probiotic component/2% arginine | 100 | 0.206±0.082 | 16.3% |
| 8 | 0.25% probiotic component/5% arginine | 200 | 0.146±0.091 | 40.7% |
| 9 | 0.5% probiotic component/10% arginine | 500 | 0.089±0.020 | 63.9% |

As could be seen from the above Table, in groups 7-9, a probiotic component/a chemical active compound having the same components and the same amount ratio but different concentrations was injected into the same volume of tumor. The pharmaceutical composition of group 7 showed no synergy (q < 1.00), while the pharmaceutical composition of group 8 showed a significant synergy (q > 1.00). The result of group 9 (q > 1.00) further indicated that the synergy was dependent on administration concentration.

Usually, if pharmaceutical compositions had the same amount ratio of active ingredients, administration dosage, they had the same co-administration effect of drugs. However, the probiotic component could obtain different co-administration effect of drugs by providing different co-administration pharmacology. Even if by intratumoral administration, the difference in drug efficacy of a probiotic component with a same dosage but different administration concentrations could even exceed the expected dynamic difference ($E_2/E_1$ > 200% in Table 13).

The above experiments further confirmed that the short-term drug efficacy of the probiotic component/co-administration substance composition of the present invention obtained by local intra-lesional administration was mainly due to the local synergy produced by their mixing and co-administering, wherein the pharmacological concentration of said probiotic component that provided the above synergy was the concentration that said probiotic component provided the local effect in the above examples, and thus the pharmacological constitution producing the local synergy by said probiotic component and its co-administration substance was not the amount ratio between them but the concentration ratio between them (above synergistic amount ratio + concentration required for the probiotic component to provide the local effect).

The concentrations of the local synergy provided in the above Examples. For methylene blue dye, the concentration was one that produced a weaker local effect (0.5-1.5%); for other weak local acting compounds, the concentration was one that produced a stronger local effect (1-35%), and for cytotoxic drugs, the concentration was one that produced a maximally general local chemical effect (0.1-5%).

In relation to its local effect, the composition of the probiotic component/co-administration substance of the present invention was also preferably administered at the administration volume /target region volume ratio described in Example 4.

**Example 9: Pharmacological study and optimization of short-term single-drug administration/ co-administration to lesion region in a common animal model**

BALB/c mice were used as experimental subjects, and breast cancer 4T1 cells were used as modeling cells. $0.5 \times 10^6$ cells/mouse were injected into the right axilla subcutaneously for transplantation tumor modeling, and the average tumor volume of successfully modeled nude mice was 160.1 mm³. The model animals were randomly divided into 21 groups, and the drugs were administered by intravenous injection (series A) and intratumoral injection (series B) according to the drugs and doses in Table 14, respectively. All the drugs administered were aqueous liquids, which were formulated according to the method of Example 1. Each group was dosed twice. On the 7th day after final administration, the animals were euthanized, and the tumor tissues were taken out after dissection to determine the tumor weight, and the tumor inhibition rates $r_A$ and $r_B$ were calculated relative to the corresponding negative control groups in series A and series B.

Table 14 Effects of different drug combinations and administration routes on tumor weights and tumor inhibition rates

| Groups | Drug types | Composition | rA | rB |
|---|---|---|---|---|
| 01 | negative control | physiological saline | -. | - |
| 1 | cytotoxic positive control | 1% 5-fluorouracil | **38.1%** | 44.1% |
| 2 | non-specific cytotoxic positive /local action positive control | 75% ethanol | **2.8%** | 21.3% |
| 3 | immunoenhancement positive control | $4 \times 10^4$U/ml interleukin-12 | **5.3%** | 4.6% |
| 4 | a weak local acting compound | 1% methylene blue | **3.7%** | 13.4% |
| 5 | inactivated probiotics | 10% heat inactivated Saccharomyces boulardii | 1.6% | 25.1% |
| 6 | probiotic semi-fluid-like component | semi-fluid formed after heat treatment and cooling treatment of 10% β-glucan particle | - | 27.3% |
| 7 | probiotic water-soluble component | 10% disrupted Saccharomyces boulardii supernatant components | - | 23.5% |

(continued)

| Groups | Drug types | Composition | rA | rB |
|---|---|---|---|---|
| 8 | a mixture of probiotic (water-insoluble/water-soluble) components | 10% disrupted Saccharomyces boulardii | - | 26.7% |
| 9 | inactivated probiotics/ a weak local acting compound | 10% heat inactivated Saccharomyces boulardii /1% methylene blue | 2.4% | 61.8% |
| 10 | probiotic semi-fluid-like component/a weak local acting compound | semi-fluid formed after heat treatment and cooling treatment of 10% β-glucan particle/1% methylene blue | - | 65.4% |
| 11 | probiotic water-soluble component/ a weak local acting compound | 10% disrupted Saccharomyces boulardii supernatant components/1% methylene blue | - | 60.3% |
| 12 | probiotic mixture/ a weak local acting compound | 10% disrupted Saccharomyces boulardii /1% methylene blue | - | 62.1% |
| 13 | inactivated probiotics/ a weak local acting compound/cytotoxic drug | 10% heat inactivated Saccharomyces boulardii /1% methylene blue /1% 5-fluorouracil | - | 98.6% |

In the drug treatment of solid tumors, chemotherapeutic drugs could attack their targets upon entering the target region, thus show chemotherapeutic efficacy in a short period of time. As could be seen from Table 14, the drugs of groups 1-5 showed therapeutic effect and pharmacological behavior in the common animal model, consistent with the results of the corresponding drugs in the immunodeficient animal model in Experiment 2, complying with the local effect pharmacology in terms of cytotoxic effect, chemical ablation, immune enhancement, and chemical ablation-like, respectively.

The drug of group 9 showed therapeutic effect ($r_{9B}>r_{4B}$ and $r_{9B}>r_{5B}$), co-administration effect($q>1.00$) and co-administration behavior ($r_{9B}/r_{9A}>200\%$) in the common animal model, consistent with the results of the corresponding drug in the immunodeficiency animal model in Experiment 5, i.e. the probiotic component could also provide local drug efficacy synergy and local safety synergy to the weak local acting compound under the above conditions of providing local action. This was further confirmed by the results of groups 10-13.

A study example of using normal tissue from animal as a local lesion model was given in the following experiment.

BALB/c mice were used as experimental subjects, and the model animals were randomly divided into 4 groups, having 6 animals in each group. The drugs were administered according to Table 15. Each group was dosed once, and the drug was administered into outer lateral muscle mass of the right leg at an injection volume of 100 ul/mouse. On the 7th day after drug administration, the animals were euthanized. The specimens of the outer lateral muscle mass of the right leg were taken out by autopsy, and perform a general pathological analysis. The sections were measured for the area of abnormal regions such as necrosis and nodules that distinguished from normal muscle.

Table 15 Dosing components for each group

| Groups | Drug types | Specific components | Area of abnormal regions mm$^2$ |
|---|---|---|---|
| 1 | chemical ablation agent | 75% ethanol aqueous solution | 33.16±13.44 |
| 2 | probiotic component | 10% heat inactivated Saccharomyces boulardii | 34.01±14.24 |
| 3 | probiotic component/chemical active substance | 10% heat inactivated Saccharomyces boulardii /1% methylene blue | 49.61±23.27 |
| 4 | probiotic component/chemical active sub stance 1/chemical active substance2 | 10% heat inactivated Saccharomyces boulardii /1% methylene blue /1% 5-fluorouracil | 78.00±4.31 |

The results of the short-term drug efficacy from the above tests and other similar tests showed that the probiotic component described in the present application as a local active ingredient had a consistent pharmacology in the common animal model and in the immunodeficiency animal models of Examples 2-8, all of them destroyed the pharmacological target (tissue within the drug penetration region) through providing local effect or local synergy.

Based on the results of Examples 2-9 above and the results of other similar tests, the local pharmaceutical composition of the present invention comprising the probiotic component and its co-administration substance (also abbreviated as the composition of the present invention), when used within a local lesion, exceeded any expectations of a prior art non-local pharmaceutical composition comprising a probiotic component/a co-administration substance (abbreviated as the prior art composition) in the following respects.

1) the co-administration pharmacology exceeded the expectations: the probiotic component and its co-administration substance in the composition of the present invention were co-administrated through components having local effects, the co-administration pharmacology of which included the co-administration of their respective local effects, and the resulting short-term synergy was mainly local synergy, which exceeded the pharmacology expectation of any co-administration involving the non-local activity (e.g. immunological effect, antiviral effect, tumor cytotoxic effect, or tumor vascular disruption effect, etc.) between the probiotic component and its co-administration substance in the prior art.

2) the pharmacological method exceeded the expectations: the probiotic component and its co-administration substance in the composition of the present invention must be strictly limited to local administration in order to produce short-term synergy, whereas any expected pharmacological method of co-administration of the probiotic component with the co-administration substance in the prior art was not necessary to be limited to local administration, preferably systemic administration.

3) the preferred embodiment of active ingredients exceeded expectations: in terms of the preferred number of component species, the compositions of the present invention preferably comprised 3 or more components (e.g., probiotic component/weak local acting compound/cytotoxic drug), although a 2-component synergistic composition (e.g., probiotic component/weak local acting compound) was already beyond expectations. In terms of the choice of co-administration substance species, the probiotic component surprisingly did not provide local synergy to ethanol, but did provide local synergy to weak local acting compounds or/and cytotoxic drugs. In terms of specific drug preferences, in addition to the aforementioned preferred embodiments where the probiotic component provided a local effect, it was also preferred that the co-administration substance comprised one or more drugs that were weak local acting compounds, which provided primarily local effect rather than systemic effect, such as one or more of the weak local acting compounds, weak local acting compounds and cytotoxic agents, weak local acting compounds and immune enhancers, etc.

4) expectations of the co-administration pharmacological constitutions of the active ingredients: the composition of the present invention must include the local effect pharmacological concentration of the probiotic component, and thus the parameter of the co-administration pharmacological constitutions thereof was the concentration ratio rather than the amount ratio in prior art compositions. The pharmacological concentration at which the probiotic component provides a co-administration action was the concentration at which it provides a local effect in the above Examples. The pharmacological concentrations of said weak local acting compound were: 5-25% for amino acid nutrients, 0.5-3% or 0.5-1.5% for methylene blue dyes, 3-10% for quinines, 3-20% for weak acids, 1-10% for strong alkalis, 1-20% for weak alkalis, and 3-25% total concentration for pH buffer systems of acidifiers or/and basifiers, respectively.

5) the technical effect exceeded expectations: the compositions of the present invention could provide local activity (local effect or local synergy) that cannot be provided by prior art compositions, at least in the administration region, a higher local therapeutic effect that cannot be provided by prior art compositions were provided. Preferably, the pharmaceutical compositions of the present application could be used for the destructive treatment (e.g., tumor load reduction) of any diseased tissue (e.g., comprising tumor cells or/and fibroblasts). More preferably, the pharmaceutical compositions of the present application could be used for the treatment of any local lesion that was difficult to be treated with systemic drugs (cytotoxic drugs, antiviral drugs, antibacterial drugs, angiogenesis inhibitors, immune drugs, etc.) and for which intra-lesional administration was practical.

**Example 10: Pharmacological study and optimization of medium- and long-term single-drug administration/co-administration to lesion region in a common animal model**

BALB/c mice were used as experimental subjects, and breast cancer 4T1 cells were used as modeling cells. $0.5 \times 10^6$ cells/mouse were injected into the right axilla subcutaneously for transplantation tumor modeling, and the average tumor volume of successfully modeled nude mice was 107.4 mm$^3$. The model animals were randomly divided into 17 groups.

The drugs, formulated according to the method of Example 1, were administered according to Table 16. Each group was dosed twice with an interval of 7 days, and the injection volumes were 300 μl/mouse/time for groups 13 and 14, 100 µl/mouse/time for other groups. The drugs were administered by subaxillary administration of the right forelimb for group 15, and by intratumoral injection for other groups. Group 16 was administered sequentially (the first dose was a probiotic semi-fluid-like component/cytotoxic drug/ weak local acting compound, and the last dose was inactivated probiotics), while the other groups were administered with the same drug twice. On days 7 and 21 after the last dose, the tumor volumes of each group were measured ($V_{7d}$, $V_{21d}$), and the relative tumor proliferation rates ($R_{7d}$, $R_{21d}$) were calculated relative to the negative control group, respectively, so that the short-term therapeutic effectiveness ($100\%-R_{7d}$) and the medium- and long-term therapeutic effectiveness ($100\%-R_{21d}$) could be calculated, respectively.

Table 16 Tumor inhibition results on day 7 and day 21 after the administrations of different drugs

| Groups | Drug category | Specific components | $R_{7d}$ | $R_{21d}$ |
|---|---|---|---|---|
| 01 | negative control | physiological saline | - | - |
| 1 | cytotoxic positive control | 1% 5-fluorouracil | 51% | **93%** |
| 2 | non-specific cytotoxic positive/local action positive control | 85% ethanol | 47% | **95%** |
| 3 | immunoenhancement positive control | $4\times10^4$U/ml interleukin-12 | **95%** | 96% |
| 4 | weak local acting compound | 1% methylene blue | **87%** | 105% |
| 5 | inactivated probiotics | 2.5% heat inactivated Saccharomyces boulardii | 76% | 61% |
| 6 | probiotic semi-fluid-like component | semi-fluid formed by heating 5% β-glucan particle and cooling | 71% | 65% |
| 7 | a mixture of probiotic water-insoluble /water-soluble components | 2.5% disrupted Saccharomyces boulardii | 73% | 63% |
| 8 | inactivated probiotics/ cytotoxic drug | 2.5% heat inactivated Saccharomyces boulardii /1% 5-fluorouracil | 50% | 51% |
| 9 | probiotic semi-fluid-like component/ cytotoxic drug | semi-fluid formed by heating 5%% β-glucan particle/1% 5-fluorouracil and cooling | 47% | 53% |
| 10 | probiotic water-soluble component/ cytotoxic drug | 2.5% disrupted Saccharomyces boulardii /1% 5-fluorouracil | 43% | 48% |
| 11 | inactivated probiotics/ cytotoxic drug/ a weak local acting compound | 2.5% heat inactivated Saccharomyces boulardii /1% 5-fluorouracil /1% methylene blue | 23% | 12% |
| 12 | probiotic semi-fluid-like component/ cytotoxic drug/ a weak local acting compound | semi-fluid formed after heat treatment and cooling treatment of 5% β-glucan particle/1% 5-fluorouracil /1% methylene blue | 22% | 11% |
| 13 | a dilution of a probiotic semi-fluid-like component | a dilution of 1 volume of semi-fluid formed after heat treatment and cooling treatment of 5% β-glucan particle plus 2 volumes of water | 93% | 92% |
| 14 | a dilution of a probiotic semi-fluid-like component/cytotoxic drug | a dilution of 1 volume of semi-fluid formed after heat treatment and cooling treatment of 5%% β-glucan particle/1% 5-fluorouracil plus 2 volumes of water | 71% | 93% |
| 15 | probiotic semi-fluid-like component | semi-fluid formed by heating and cooling of 5% β-glucan particle | 95% | 94% |
| 16 | inactivated probiotics+ a probiotic semi-fluid-like component/cytotoxic drug/ a weak local acting compound | semi-fluid formed after heat treatment and cooling treatment of 5% β-glucan particle/1% 5-fluorouracil /1% methylene blue, and 2.5% heat inactivated Saccharomyces boulardii | 27% | 22% |

It was well known that the effect dynamics of chemotherapeutic drugs were characterized by when the drug existed, its attack occurred; while if the drug was metabolized, then the attack no longer occurred. The pharmaceutical effect of immune enhancers showed almost no change of tumor body. For groups 1 and 2, the ratio of relative tumor proliferation rate R (R21 /R7) on days 21 and 7 was greater than 1.50, indicating that the short-term drug effect on tumor inhibition was significantly reduced. The relative tumor proliferation rate in group 3 was consistently high, corresponding to the expectation that use of immune enhancers alone did not show significant therapeutic effect. The positive controls (5-fluorouracil, ethanol, and interleukin-12) in groups 1-3 showed medium- and long-term drug efficacies, consistent with the effect dynamics (R21/R7) expected from their pharmacology (cytotoxic pharmacology, immune enhancing pharmacology, and local effect pharmacology, respectively), thus constructing a comparative study system.

The short-term drug efficacies of the pharmaceutical compositions of groups 1-7 on day 7 were consistent with the results in Example 9. The probiotic components in groups 5-7 showed short-term drug efficacies that were consistent with an expectation of local active ingredients that were similar to the chemical ablation agent in group 2, and would seem to show medium- to long-term drug efficacies consistent with the chemical ablation agent of group 2. Surprisingly, the probiotic component showed completely different effect dynamics (R21/R7 significantly less than 100%), which greatly exceeded the expected medium- and long-term pharmaceutical efficacies of all positive controls in pharmacology. Different probiotic components showed almost identical effect dynamics, independent of their bacterial immunogenicity. Similarly, groups 8-12 showed that probiotic component/co-administration substance resulted in local synergy ($q_{7d}$>1.00), medium- and long-term synergy ($q_{21d}$>1.00), and effect dynamics ($R_{21}/R_7$<100 % ) completely different from those of the above positive controls.

Compared group 13 with group 5 (same administration component and dosage), the administration concentration of the probiotic component was not the preferred concentration for its local effect pharmacology and showed a short-term difference in drug efficacy >200%. Compared group 14 with group 6 (same administration component and amount ratio, dosage), the administration concentration of the composition was not the preferred concentration for its local synergy pharmacology, and also showed a short-term difference in drug efficacy greater than 200%. The medium- and long-term drug efficacy ($R_{21}$) of groups 13 and 14 was also considerably lower than those of groups 5 and 6. Further, the effect dynamics of groups 13 and 14 ($R_{21}/R_7$ approximate to or greater than 100%) were significantly different from those of groups 5 and 6 (($R_{21}/R_7$ significantly less than 100%). The results of group 15 were similar to those of group 13, as if the local effects of the probiotic component outside the lesion (short-term drug efficacy) and its secondary effect (medium- and long-term drug efficacy, effect dynamics) were also too weak. The medium- and long-term pharmaceutical efficacies of the sequentially administered combination in group 16 (probiotic semi-fluid-like component/cytotoxic drug/ weak local acting compound once, and inactivated probiotics once) was between those of the second administration of two identical drugs (inactivated probiotic and probiotic semi-fluid-like component/cytotoxic drug/weak local acting compound, which showed different short-term (local effect) therapeutic potency in groups 5 and 12 respectively), further suggesting that the medium- and long-term therapeutic effects of these drugs were correlated with their short-term (local effect) therapeutic effects.

The above results illustrated that the probiotic components of groups 5-12 showed medium- to long-term effects beyond the pharmacological expectation of groups 1-3 and even beyond their simple local effect pharmacological expectation, that is, a new pharmacology different from the chemical action or immune enhancement effect. The generation of this new pharmacology must at least satisfy the pharmacological conditions of the local effects in Examples 2-4 above (comprising preferably the probiotic component with minimized bacterial immunogenicity, etc.), and thus could reasonably infer that it was a secondary effect (synergy) of the local effect.

In summary, the short-term effect of the composition of the present application after intra-lesional administration was mainly a local effect (or a local synergy), while the long-term effect was mainly a secondary effect resulting from a local effect (or a local synergy), such as a secondary immunological effect. The composition of the present application was completely different from immune drugs such as vaccines in which the substances that function as antigens comprised in situ immune substances such as in situ antigens that were produced after administration. In addition, the probiotic component, like other exogenous substances, might also have a non-specific antigenic action for enhancing the drug efficacy of the in situ immune substance.

The compositions of the present application had chemical/immunopharmacological functions including local effect (or local synergy) and secondary immunological effect thereof, thus greatly exceeding the drug efficacy expectations regarding the pharmaceutical effect of probiotic components in prior art and becoming therapeutic agents rather than prior art immune enhancers. By limiting intra-local lesion administration, a concentration threshold for the local effect, a volume threshold for the local effect, local synergistic combinations, etc., the compositions of the present application may provide effective treatment for any local lesion disease, avoid high frequency of large dosage administration within a course of treatment necessary for cytotoxic drugs, and had high safety and compliance. The compositions of the present application could be used for the treatment of local lesion diseases that were difficult to treat with cytotoxic drugs, antiviral drugs, antibacterial drugs, angiogenesis inhibitors, immune drugs, etc., such as solid tumors with interstitial ratio greater than 25%. In contrast to other drugs, the compositions described in the present application could be used as histotoxic/immune drugs, and further be used to treat the above indications by taking fibroblasts as an action target, while fibroblasts and the deposits they produced such as fibers were often one of the main features of refractory local lesion diseases.

**Example 11: Pharmacological study and optimization of local single-drug administration/co-administration outside a lesion region in a common animal model**

Despite inapparent drug efficacy by extra-tumoral administration in Example 9, the study on extra-tumoral administration was not abandoned. BALB/c mice were used as experimental subjects, and breast cancer 4T1 cells were used as modeling cells. $0.25 \times 10^5$ cells/mouse were injected into the right axilla subcutaneously for transplantation tumor modeling. On the 5th day after modeling, the test animals with visually obvious tumors were randomly divided into 12 groups, having 10 animals in each group. The drugs were administered according to the drugs and dosages in Table 17, the drugs were formulated according to the method of Example 1; groups 10 and 11 were administered different drugs one day at two places (a composition of probiotic component/co-administration substance was administered intratumorally, and probiotic component alone was administered intradermally in the left axilla of the animals on the same day), and all the other groups were subaxillary administered intra-dermally twice at an interval of 7 days, each time with the same drug. In groups 1-11, on days 7 and 21 after the last dose the tumor volume ($V_{7d}$, $V_{21d}$) were measured, and the relative tumor proliferation rates ($R_{7d}$, $R_{21d}$) were calculated relative to the negative control group on days 7 and 21 after the dose, so that the short-term therapeutic effectiveness (100%-$R_{7d}$) and the medium- and long-term therapeutic effectiveness (100%-$R_{21d}$) could be calculated, respectively. Further, based on the short-term therapeutic effectiveness and the medium- and long-term therapeutic effectiveness, the actual/expected ratio for short-term pharmaceutical efficacies q7d and the actual/expected ratio for medium- and long-term pharmaceutical efficacies q21d were calculated respectively.

Table 17 Tumor inhibition results on days 7 and 21 after the administration of different drugs

| Groups | Drug category | Specific components | Dose | $R_{7d}$ | $R_{21d}$ |
|---|---|---|---|---|---|
| 01 | negative control | physiological saline | 120 | - | - |
| 1 | cytotoxic positive control | 1% 5-fluorouracil | 120 | 73% | 93% |
| 2 | local action positive control | 85% ethanol | 120 | 92% | 107% |
| 3 | immunoenhancement positive control | $4 \times 10^4$U/ml interleukin-12 | 120 | 99% | 97% |
| 4 | a weak local acting compound | 20% lysine | 120 | 96% | 102% |
| 5 | inactivated probiotics | 10% heat inactivated Saccharomyces boulardii | 120 | 94% | 71% |
| 6 | probiotic water-insoluble particle | 10% β-glucan particle | 120 | 95% | 75% |
| 7 | inactivated probiotics/ a weak local acting compound | 10% heat inactivated Saccharomyces boulardii /20% lysine | 120 | 97% | 74% |
| 8 | inactivated probiotics | 10% heat inactivated Saccharomyces boulardii | 10 | 96% | 95% |
| 9 | inactivated probiotics/ a weak local acting compound | 10% heat inactivated Saccharomyces boulardii /20% lysine | 10 | 93% | 91% |
| 10 | probiotic component as single-drug + probiotic component/co-administration substance composition | 10% heat inactivated Saccharomyces boulardii +10%probiotic water-insoluble particle/20% lysine | 120 | 14% | 23% |
| 11 | probiotic component as single-drug + probiotic component/co-administration substance composition | 10% probiotic water-insoluble particle+10% probiotic water-insoluble particle/20% lysine | 120 | 11% | 21% |
| *: Dose (μl/mouse/time) | | | | | |

As could be seen from Table 17, the positive controls (5-fluorouracil, ethanol, and interleukin-12) in groups 1-3 showed results consistent with their pharmacological expectations (cytotoxic pharmacology, immune enhancing pharmacology, and local effect pharmacology, respectively), none of which provided meaningful medium- to long-term drug efficacy. The short-term drug efficacy of groups 5-7 was mainly local effect or local synergy, and the non-significant results were consistent with that of group 2. Surprisingly, non-negligible medium- and long-term drug efficacy were observed for groups 5-7, which showed a new pharmacology substantially different from any pharmacology of the control groups 1-3. Groups 8 and 9 using the conventional vaccine dose (20 mg/kg) failed to observe the same medium- and long-term drug efficacy as groups 5 and 6 using the superconventional vaccine dose (240 mg/kg), showing that the new pharmacology of the latter was more closely related to the local effect. The short-term drug efficacy in groups 10 and 11, which administered intratumorally a composition of probiotic component/co-administration substance, and administered extratumorally a probiotic component alone on the same day, were mainly due to the intratumoral local synergy, while the medium- and long-term drug efficacy were a combination effects of this intratumoral local synergy and the secondary effect of the extratumoral local effect shown in groups 5 and 6.

It was observed that most of the experimental animals in groups 5 and 6 produced strong struggling reactions upon injection, suggesting that the probiotic component alone should be used under analgesic conditions to avoid safety risks, while group 7 unexplainably did not observe the strong reactions described above, suggesting that synergic drug compositions of the probiotic component with weak local acting compounds and/or cytotoxic drugs had a safety profile beyond that expected from the single-drug effect.

The following Examples further investigated the local effect of the compositions of the present application at the axillary subcutane.

BALB/c mice were used as experimental subjects, and randomly divided into two groups: group A and group B with 6 animals in each group. 10% heat-inactivated Saccharomyces boulardii and 10% heat-inactivated Saccharomyces boulardii/20% lysine were administered to groups A and B, respectively, and each group was administered once to the right foreleg axillary intracutane with an injection volume of 100ul/animal. Seven days after the administration, the animals were euthanized, and the specimens of intracutaneous nodules in the axilla of the right foreleg of the mice were removed by autopsy, sectioned and rinsed, and the area of abnormal regions distinguished from normal muscle, such as the transection of necrosis and nodules, was measured.

The results showed that the areas of abnormal region of groups A and B were $30.15\pm11.31mm^2$ and $45.74\pm13.71mm^2$, respectively, which were consistent with the results of Example 9. The probiotic component in the technical solution of the present application was similar to highly concentrated ethanol, and the drug efficacy first shown after local administration was mainly local effect (or local synergy), which was consistent with the local effect (or local synergy) shown after intratumoral administration in Examples 2, 5 and 9, which was mainly a local chemical effect (or local chemical synergy) similar to chemical ablation. The short-term effects of the compositions of the present application after extra-tumorally local administration were mainly the destruction of tissue in the subcutaneous drug penetration region, while the medium- and long-term effects were mainly the secondary effect resulting from the destruction. The medium to long term effects across the distance observed in groups 5-7 in the Table above indicated that the secondary effect mainly included a vaccine effects Thus, the composition of the present application might provide an extra-tumoral vaccine effect completely different from that of a classical vaccine, in which the substance acting as an antigen comprised a non-specific antigen produced by tissue destruction after drug administration In addition, like other exogenous substances, the probiotic component also had a non-specific antigenic effect for enhancing the drug efficacy of the vaccine.

From the above, it was clear that when used in suitable locality of extra-local lesion, the compositions of the present invention comprising probiotic components (compositions of the present invention) were completely different from conventional compositions (usual compositions) showing systemic-acting pharmacology (e.g., cytotoxic pharmacology, immune-enhancing pharmacology as above) or local-acting pharmacology (e.g., chemical ablation as above): 1) target pharmacology: the compositions of the present invention could provide a sufficiently strong secondary effect or a secondary synergy (including, but not limited to, antigenic effect of bad tissue and other secondary immune responses yet to be investigated) of local effect, which greatly exceed the pharmacological expectations of the effects of the usual compositions (e.g., little antigenic effect of bad tissue due to chemical ablation); 2) pharmacological constitution: the conditions that must be met for the compositions of the invention to produce sufficiently strong local effect outside the local lesion were the same as local effect of the probiotic components of the above Examples in the local lesion in terms of pharmacological constitutions. This greatly exceeded the expected pharmacological constitution of the usual compositions (e.g., stronger immunogenicity or even maximized preferred immune enhancing components, preferred high dose cytotoxic drugs, etc.); 3) technical effects: the compositions of the present invention could produce significant medium- to long-term cross-distance inhibition of local lesions similar to that of therapeutic vaccines, which greatly exceeded the expected technical effects of the existing usual compositions..

In addition, although no relationship between the extra-tumoral administration volume and the intra-tumoral target region volume had been found, a necessary condition for its administration volume was the ability to form the nodules required as an antigen, e.g., when the total volume of the tumor was $\geq2.85cm^3$, then the administration volume was > 0.01 ml/kg person, 0.015-0.25 ml/kg person, preferably 0.020-0.25 ml/kg person, which required the administration volume of the drug comprising the probiotic component for extra-tumoral local injection in the present application was 2 folds or 3--100 folds, preferably 5-100 folds of the administration volume of conventional vaccines.

**Example 12: Further study of secondary effect (synergy) and optimization of the scheme**

BALB/c mice were used as experimental subjects, in which modeled animals were used as series A and unmodeled animals as series B. Breast cancer 4T1 cells were use as modeling cells for modeled animals. $0.25 \times 10^5$ cells/animal were injected into the right axilla subcutaneously for transplantation tumor modeling, and the average tumor volume of successfully modeled nude mice was 173.6mm$^3$. The modeled animals were randomly divided into 18 groups, with 10 animals in each group, and the drugs according to Table 18 was administered via intratumoral injection. BALB/c mice were additionally taken as experimental subjects without modeling, and randomly divided into 18 groups, with 10 animals in each group, the drugs according to Table 18 was administered via intramuscular injection into the right hind thigh. All the study drugs comprising probiotic components were formulated as aqueous formulations according to the preparation method of Example 1, and the other drugs were formulated as aqueous solutions according to the existing well-known methods. Each group was administered twice at an interval of 3 days, and the dosage was 50ul/mouse/time.

The tumor volume of each group of series A was measured on the 10th and 30th day after the 1st drug administration and the tumor inhibition rate ($r'_{10d}$, $r'_{30d}$) was calculated relative to the negative control. On the 30th day after the first administration, each group of series A and each group of series B were subcutaneous cell transplantation of $1 \times 10^5$ breast cancer 4T1 cells/mouse and $0.2 \times 10^5$ fibroblast 3T3 cells/ mouse respectively to left anterior axilla and left posterior axilla. The tumor incidence rates of 4T1 cell transplantation ($S_{4T1}$) and of 3T3 cell transplantation ($S_{3T3}$) in each group were observed on the 10th day after the cell transplantation, wherein the tumor incidence rate S = (number of tumor-bearing animals/number of cell transplanted animals) $\times$ 100%.

Table 18 Data of tumor inhibition rate and tumor incidence rate of different groups

| Groups | Types of drug combination | Specific components | Series A | | | | Series B | |
|---|---|---|---|---|---|---|---|---|
| | | | $r'_{10d}$ | $r'_{30d}$ | $S_{3T3}$ | $S_{4T1}$ | $S_{3T3}$ | $S_{4T1}$ |
| 01 | negative control | physiological saline | 0 | 0 | 100% | 100% | 100% | 100% |
| 02 | cytotoxic positive control of tumor | 1% 5-fluorouracil aqueous solution | 38.5% | 6.1% | 100% | 100% | 100% | 100% |
| 03 | immune-enhancement control | medical Lentinan injection -Jinling Pharmaceutical Co., Ltd. | 8.2% | 5.7% | 100% | 100% | 100% | 100% |
| 04 | local action positive control | Anhydrous ethanol | 42.1% | 3.2% | 100% | 100% | 100% | 100% |
| 05 | vaccine adjuvant control | 0.5% aluminum hydroxide adjuvant aqueous suspension | 4.7% | 3.9% | 100% | 100% | 100% | 100% |
| 06 | polysaccharide control | 5% starch aqueous solution | 5.1% | 4.7% | 100% | 100% | 100% | 100% |
| 1 | inactivated probiotics | 2.5% heat inactivated Saccharomyces boulardii | 33.5% | 33.9% | 100% | 20% | 100% | 100% |
| 2 | probiotic component semi-fluid | semi-fluid formed after heat treatment and cooling treatment of 5% β-glucan | 41.2% | 40.1% | 100% | 20% | 100% | 100% |
| 3 | probiotic water-soluble component 1 | 5% hydroxymethyl β-glucan | 43.5% | 51.3% | 100% | 20% | 100% | 100% |
| 4 | probiotic water-soluble component 2 | 20% Saccharomyces cerevisiae ribonucleic acid aqueous solution | 40.3% | 52.7% | 100% | 20% | 100% | 100% |

47

(continued)

| Groups | Types of drug combination | Specific components | Series A | | | | Series B | |
|---|---|---|---|---|---|---|---|---|
| | | | $r'_{10d}$ | $r'_{30d}$ | $S_{3T3}$ | $S_{4T1}$ | $S_{3T3}$ | $S_{4T1}$ |
| 11 | weak local acting compound/ cytotoxic drug-co-admin istration drug 1 | 20% arginine /1% 5-fluorouracil aqueous solution | 75.3 % | 5.4 % | 100 % | 100 % | 100 % | 100 % |
| 12 | weak local acting compound/ cytotoxic drug co-administrati on drug 2 | 1% methylene blue /1% 5-fluorouracil aqueous solution | 63.7 % | 9.1 % | 100 % | 100 % | 100 % | 100 % |
| 21 | inactivated probiotics/co-administrati on drug 1 | 2.5% heat inactivated Saccharomyces boulardii/20% arginine /1% 5-fluorouracil aqueous suspension | 85.7 % | 68.5 % | 100 % | 0 | 100 % | 100 % |
| 22 | inactivated probiotics/ co-administrati on drug 2 | 2.5% heat inactivated Saccharomyces boulardii/1% methylene blue /1% 5-fluorouracil aqueous suspension | 83.2 % | 66.9 % | 100 % | 0 | 100 % | 100 % |
| 23 | probiotic component semi-fluid / co-administrati on drug 1 | semi-fluid formed after heat treatment and cooling treatment of 5% β-glucan /20% arginine /1% 5-fluorouracil aqueous suspension | 87.6 % | 74.1 % | 100 % | 0 | 100 % | 100 % |
| 24 | probiotic component semi-fluid / co-administrati on drug 2 | semi-fluid formed after heat treatment and cooling treatment of 5% β-glucan /1% methylene blue /1% 5-fluorouracil aqueous suspension | 82.4 % | 73.4 % | 100 % | 0 | 100 % | 100 % |
| 25 | probiotic water-soluble component 1/ co-administrati on drug 2 | 5% water-soluble Saccharomyces cerevisiae β-glucan /1% methylene blue /1% 5-fluorouracil aqueous solution | 89.2 % | 78.6 % | 100 % | 0 | 100 % | 100 % |
| 26 | probiotic water-soluble component 2/ co-administrati on drug 2 | 20% Saccharomyces cerevisiae ribonucleic acid aqueous solution/1% methylene blue /1% 5-fluorouracil aqueous solution | 91.3 % | 79.5 % | 100 % | 0 | 100 % | 100 % |

As could be seen from Table 18, the tumor inhibition rates $r'_{10d}$, $r'_{30d}$ in series A respectively represented the short-term drug efficacy and medium- and long-term drug efficacy against local lesions, which resulted from the drugs administered in the lesion. As mentioned previously, the short-term drug efficacy of the probiotic component and the composition thereof was directly related to the local effect, while the medium- and long-term drug efficacy, especially the medium- and long-term drug efficacy that was significantly higher than that expected for chemotherapeutic drugs, was related to the secondary effect or a secondary synergy of local effect, especially to the secondary immunological effect.

In series A, each of the controls in groups 02-06 showed pharmaceutical efficacies that were consistent with their respective pharmacological expectations after administration via intratumoral injection, namely cytotoxic effect, immune enhancing effect, chemical ablation effect, vaccine adjuvant effect, and conditioned local effect (polysaccharide), respectively, in which cytotoxic effect (group 02) and chemical ablation effect (group 04) but not the other effects showed short-term drug efficacy ($r'_{10d}$), while none of the effects showed medium- to long-term drug efficacy ($r'_{30d}$). The probiotic component used in groups 1-4 as single-drug showed significant short- and medium- and long-term therapeutic effects. Unexpectedly, the inactivated probiotics had the highest short-term drug efficacy ($r'_{10d}$), but lowest medium- and long-term drug efficacy ($r'_{30d}$).

Based on the pharmaceutical efficacies ($r'_{10d}$, $r'_{30d}$) of composition (A/B) and of the corresponding components (A, B) in series A, the actual/expected ratios of the short-, medium- and long-term pharmaceutical efficacies (q10d, q30d) of the composition co-administration could be calculated, wherein $q = r'_{A/B}/[r'_A + r'_B - (r'_A \times r'_B)]$. $q_{30d}$ of the compositions formed by the probiotic components/co-administration substance in groups 21-26 were all greater than 1.0, even greater than 1.15, with the medium- and long-term pharmaceutical efficacies greater than the expected sum of the medium- and long-term pharmaceutical efficacies of the components, and there was a secondary synergy of local effect when the drugs were co-administered. Consistent with the above-mentioned groups 3-4, the short-term pharmaceutical efficacies ($r'_{10d}$) of groups 22-26 were inferior to that of group 21, while their medium- and long-term pharmaceutical efficacies ($r'_{30d}$) were higher.

In addition, $S_{3T3}$ and $S_{4T1}$ in Table 18 represented transplant rejections against transplanted cells 3T3 and 4T1, respectively. $S_{3T3}$ and $S_{4T1}$ from controls of groups 01-06 were 100%, regardless of series A or series B, indicating that no immunotherapeutic effect was produced. In series A, the probiotic component used in groups 1-4 produced immunological effects that were not yet sufficient to completely suppress nonpathogens ($S_{3T3}$ was 100%), but were sufficient to eliminate or at least strongly suppress pathogens ($S_{4T1}$ was much less than 100%), showing significant specificity. This indicated that the short-term (local) effects generated by the related drugs were limited to local lesions, while the medium- and long-term (secondary) effects were not limited to local lesions, but included therapeutic immunological effects. The pharmaceutical compositions of groups 21-26 produced immunological effects consistent with the corresponding probiotic components in groups 1-4, respectively, but had a stronger immunological effect against the pathogen (4T1) and a lower tumorigenic rate. In series B, the non-specific immunological effects generated from the probiotic components in groups 1-4 and the compositions in groups 21-26 were not sufficient to potently inhibit any of the transplanted cells (4T1 and 3T3).

By comparing especially the graft rejections of the composition (A/B) and the corresponding constituents (A, B) of series A in the Table against the same pathogen [$S_{4T1}$ or $S'_{4T1}=(1-S_{4T1})$], the actual/expected ratio of the immunological co-administration effect in the co-administration pharmaceutical efficacies of the composition, could be calculated: $q_{4T1}=S'_{A/B4T1}/[S'_{A4T1}+S'_{B4T1}-(S'_{A4T1} \times S'_{B4T1})]$. The actual/expected ratios $q_{4T1}$ of the compositions in groups 21-26 were all greater than 1.00, showing immune synergy, especially specific immune synergy. The probiotic component and the composition comprising the probiotic component first produced a local effect at the lesions, suggesting that the immunological effect or immune synergy, especially specific immunological effect or specific immune synergy, in the medium- and long-term pharmaceutical efficacies, should be an environment-dependent secondary effect of the local effect.

Based on the above results and the results of other similar tests, when used for the treatment of local lesions, the local pharmaceutical composition comprising the probiotic component described in the present invention (referred to as compositions of the present invention) exceeded any of the prior art non-specific immunotherapeutic pharmaceutical compositions (referred to as prior art compositions, such as the above cytotoxic drugs, immune enhancers, chemical ablation agents, vaccine adjuvants, conditioned local agents, etc.) as expected in the following aspects.

1) its pharmacology exceeded expectations: the pharmacology, especially the medium- and long-term pharmacology, of the composition of the present invention comprised a secondary effect (or a secondary synergy), especially pathogen-specific immunological effect, of a special local effect (or a local synergy), which was not found in the prior art composition.

2) its pharmacological constitution exceeded expectations: on the basis of the above Exampled probiotic component local effect pharmacological constitution, it was not necessary to maximize the local effect provided by the probiotic component, or even preferably minimize the local effect provided by the probiotic component, such as the probiotic component was preferably selected from the components having very weak bacterial immunogenicity and local effect (water-soluble component and semi-fluid-like component), the probiotic component pharmacological concentration was preferably selected from lower rather than higher administration concentration (the administration concentration of water-soluble polysaccharide was limited to 3-15% or 3-5%, administration concentration of nucleic acid was limited to 10-25%, administration concentration of semi-fluid component was limited to 4-12%), multiple (preferably 2 or more) co-administration drugs in specific combination relationship, etc..

3) the technical effect exceeded expectations: significant, even effective medium- and long-term drug efficacy and cross-distance drug efficacy, especially the obvious specific immunological effect similar to the therapeutic vaccine, was generated under conditions (for example, there was no short-term synergistic drug efficacy, even there was short-term antagonistic drug efficacy) that a prior art composition did not generate, which allowed the composition of the present invention have a range of indications greatly exceeded that of the prior art composition.

**Example 13: Study of dosage form and optimization**

BALB/c nude mice were used as experimental subjects, and breast cancer cells 4T1 were used as modeling cells. $0.25 \times 10^5$ cells/mouse were injected into the right axilla subcutaneously for transplantation tumor modeling, and the average tumor volume of successfully modeled nude mice was 291.7mm$^3$. The model animals were randomly divided into 3 groups, where A, B and C were given physiological saline, 5 % inactivated Saccharomyces boulardii aqueous suspension, aqueous suspension obtained by mixing 5 % inactivated Saccharomyces boulardii and physiological saline, as the study drugs, respectively. The above drugs were formulated according to the preparation method of Example 1. Each group was injected a drug intratumorally twice with an interval of 2 days, and the injection volume was 150 μl/mouse/time. On the 10th day after the first drug administration, the animals were euthanized, and the tumor tissues were taken out after dissection to determine the tumor weight, and the tumor inhibition rate (r) was calculated relative to the negative control group.

The results showed that the drug in group B was in local dosage form and did not comprise osmotic regulator, while the drug in group C was in non-local dosage form and comprised osmotic regulator - sodium chloride, in which the tumor inhibition rates in groups B and C were 19.8% and 41.3%, respectively, and group C was significantly lower than group B. This indicated that osmotic regulator would interfere with the immune enhancement effect of the composition, thus reduce the local effect. In the present experimental conditions, the probiotic component was even changed from providing an effective therapeutic effect in group B to no therapeutic effect in group C. From the above, it was clear that the selection of dosage form for the present application compositions was fundamentally different from the prior art compositions. Specifically, in the prior art, compositions comprising probiotic components were usually in oral dosage form or conventional injectable dosage form, the former usually needs to comprise solid excipients, while the latter usually needs to comprise osmolality raising agent (salt or monosaccharide) of the probiotic component to ensure that osmolality of the drug liquid was the same or similar to the blood osmolality. The pharmaceutical compositions described in the present application only needed to meet the requirement of providing this local effect, and in its preferable embodiments, it could comprise no solid excipients or no osmolality enhancers to avoid reducing the local effect. In fact, the above described dosing form was also the preferred dosage form for locally acting drugs.

**Example 14 Comparative study and further optimization in the middle-aged and elderly patient model**

Eight-month-old mice were used as experimental subjects in the middle-aged and elderly patient model, $0.5 \times 10^5$ mouse breast cancer 4T1 cells/mouse were injected into the right axilla subcutaneously for transplantation tumor modeling, and the mean tumor volume of successfully modeled nude mice was 117.3 mm$^3$. The model animals were randomly divided into 18 groups. Each group was administered once by intratumoral injection according to the drug grouping in Table 19 with an injection volume of 50 μl/mouse. The local lesion volume (V) was measured on day 3 and day 21 after drug administration, and the tumor inhibition rates ($r'_{3d}$, $r'_{21d}$) were calculated at each of time points relative to the negative control group, and the results were shown in the following Table. The drugs to be administered were prepared according to the conventional preparation method for aqueous solution or according to the preparation method in Example 1. The actual/expected ratio $q_{3d}$ for short-term drug efficacy and the actual/expected ratio $q_{21d}$ for medium- and long-term drug efficacy were calculated based on the drug efficacies ($r'_{3d}$, $r'_{21d}$) at different times (day 3, day 21), respectively.

Table 19 Short-acting and long-acting data after dosing in different groups

| Group No. | Drug types | Component, dosage | Tumor inhibition rate | |
|---|---|---|---|---|
| | | | $r'_3$ | $r'_{21}$ |
| 01 | negative control | physiological saline | 0 | 0 |
| 1 | Saccharides control 1 | 10% starch aqueous solution | 3.1% | 3.9% |
| 2 | Saccharides control 2 | 20% glucose aqueous solution | 3.1% | 4.3% |
| 3 | Saccharides control 3 | 30% glucose aqueous solution | 20.8% | 8.2% |
| 4 | probiotic component 1 | 2.5% hydroxymethyl glucan aqueous solution | 3.2% | 4.5% |
| 5 | probiotic component 2 | 5% hydroxymethyl glucan aqueous solution | 2.9% | 3.7% |
| 6 | probiotic component 3 | 10% hydroxymethyl glucan aqueous solution | 4.3% | 6.5% |
| 7 | probiotic component 4 | 20% hydroxymethyl glucan aqueous solution | 7.6% | 7.4% |
| 8 | probiotic component 5 | 10% β-glucan (small molecule) aqueous solution | 3.1% | 2.8% |

(continued)

| Group No. | Drug types | Component, dosage | Tumor inhibition rate | |
|---|---|---|---|---|
| | | | $r'_3$ | $r'_{21}$ |
| 9 | analogue | 10% β-D-glucan (barley) aqueous solution (100ul) | 3.9% | 5.1% |
| 10 | local medicine | 1% methylene blue /1% 5-chlorouracil aqueous solution | 45.9% | 31.1% |
| 11 | Saccharides control 1 /local medicine | 10% starch /1% methylene blue /1% 5-chlorouracil aqueous solution | 44.7% | 17.1% |
| 12 | Saccharides control 2 /local medicine | 20% glucose /1% methylene blue /1% 5-chlorouracil aqueous solution | 43.6% | 14.3% |
| 13 | Saccharides control 3 /local medicine | 30% glucose /1% methylene blue /1% 5-chlorouracil aqueous solution | 63.2% | 19.3% |
| 14 | probiotic component 1 /local medicine | 2.5% hydroxymethyl glucan /1% methylene blue /1% 5-chlorouracil aqueous solution | 61.3% | 53.2% |
| 15 | probiotic component 2 /local medicine | 5% hydroxymethyl glucan /1% methylene blue /1% 5-chlorouracil aqueous solution | 67.1% | 71.8% |
| 16 | probiotic component 3 /local medicine | 10% hydroxymethyl glucan /1% methylene blue /1% 5-chlorouracil aqueous solution | 59.3% | 81.6% |
| 17 | probiotic component 4 /local medicine | 20% hydroxymethyl glucan /1% methylene blue /1% 5-chlorouracil aqueous solution | 47.9% | 83.8% |
| 18 | probiotic component 5 /local medicine | 10% β-glucan (small molecule) /1% methylene blue /1% 5-chlorouracil aqueous solution | 46.4% | 46.7% |
| 19 | Analogue/local medicine | 10% β-D-glucan (barley) /1% methylene blue /1% 5-chlorouracil aqueous solution | 33.7% | 31.6% |

The middle-aged and elderly patient model was not consistent with the commonly used young patient model in terms of tumor formation and immunocompetence. As could be seen from Table 19, the saccharide controls in groups 1-3 showed short-term drug efficacies ($r'_{3d}$>15%) only at very high concentrations (30%), while no medium- to long-term drug efficacies were shown at any concentration ($r'_{21d}$ < 15%), consistent with the expectation that saccharide nutrients might provide a local effect only at very high concentrations (30-50%), and would not provide meaningful secondary effect. Neither meaningful short-term drug efficacies ($r'_{3d}$<15%) nor medium- to long-term drug efficacies ($r'_{21d}$ <15%) was observed for the probiotic components (comprising analogues) in groups 4-9, suggesting that when the drugs were administered locally in elderly patients, any systemic mechanisms (e.g., chemotherapeutic mechanisms such as cytotoxicity) that could produce short-term drug efficacies and medium- to long-term drug efficacies were negligible, and the local effects were also minimized. The local effect of the drug combinations in group 10 was significantly effective ($r_{3d}$ > 40%), but the effectiveness of the local effect decayed over time ($r'_{21d}$ < 2/3×$r'_{3d}$).

The actual/expected ratio $q_{3d}$ for short-term drug efficacies and the actual/expected ratio $q_{21d}$ for medium- and long-term drug efficacies were calculated based on the drug efficacies ($r'_{3d}$, $r'_{21d}$) at different times (day 3, day 21), respectively. The saccharide controls in groups 11-13 showed local synergy ($q3d$ > 1.00) only at very high concentrations (30%), while no medium- to long-term synergy was shown at any concentrations ($q_{21d}$ was less than 1.00), which was consistent with the expectation that saccharide nutrients provide local synergy only at very high concentrations (30-50%) but provide no meaningful secondary synergy. The probiotic component and their analogues in groups 14-19 were similar to saccharide controls in groups 11 and 12 in terms of concentration, both had minimal local effects, and their co-administration with same local medicine seem to produce similar results. However, the short-term drug efficacies ($r'_{3d}$) of the probiotic component (comprising analogues) co-administrated with a same local medicine was significantly higher and the difference in the medium- and long-term drug efficacies ($r'_{21d}$) was significantly wider compared to the same concentration of the saccharide control. In fact, the $q_{21d}$ of the probiotic component (comprising analogs) co-administrated with the same local medicine was greater than 1.00, which greatly exceeded the expected medium- and long-term synergy based on the saccharide control/local medicine effects. In addition, despite of the very similar structures, the probiotic component/local medicine could produce short-term effects and medium- to long-term synergy beyond those expected from analogues/local medicine of other sources.

Considering that the probiotic components (comprising analogs) themselves did not provide medium- and long-term drug efficacy under the conditions of the Example, the co-administratin pharmacology from above results exceeded the pharmacological expectations of synergy in the prior art. The probiotic components (comprising analogs) with minimized bacterial antigenicity, minimized local effects ($r'_{3d}$<15%), and minimized medium- and long-term pharmaceutical effeicacy ($r'_{21d}$< 15%) might provide a previously undiscovered but useful pharmacological function in related technical solutions - i.e., to provide medium- to long-term pharmacological synergistic activity for a co-administration substance that did not necessarily have local synergy, or had only weak local synergy, under the conditions that the probiotic components per se did not necessarily show strong, preferably showed a minimized local effect. In related technical solutions, the range of indications for the medium- and long-term synergistic pharmaceutical compositions comprising probiotic components (comprising analogs) had the following requirements: for middle-aged and elderly patients, the pharmacological constitutions having the following requirements: probiotic components with minimized local effect (e.g., water-soluble components and semi-fluid components), pharmacological concentrations with minimized local effect (e.g., administration concentrations limited to 3-15% for water-soluble polysaccharides, administration concentrations limited to 10-25% for nucleic acids, to 4-12% for semi-fluid component, etc.).

The local pharmaceutical compositions comprising probiotic components described herein (abbreviated as the compositions of the present invention), in addition to exceeding the expectations of any non-specific immunotherapeutic pharmaceutical compositions in the above Examples, based on the above results of the present examples (especially medium to long-term results) and other results of similar trials, were also exceeded the expectations of prior art local pharmaceutical compositions comprising local acting drugs (e.g., ethanol, conventional saccharide nutrients, etc.) (abbreviated as prior art compositions) particularly in the following aspects.

1) The preferred co-administration pharmacology exceeded expectations: the preferred co-administration pharmacology of the prior art compositions based on the existing definition of synergy (synergy was that the similar effects of multiple specific active ingredients were enhanced each other via co-administration) was local synergy under conditions favoring the local effects of the components (e.g., the local effects of conventional saccharide nutrients were larger or even maximized), while the preferred co-administration pharmacology of the compositions of the present invention exceeding the existing definition of synergy was that a medium to long-term synergy was generated under conditions that did not clearly favor the local effect of the components (e.g., the local effect of the probiotic component was small or even minimized, and the probiotic component and its co-administration substance did not have a short-term synergy, or even had a short-term antagonistic effect), and the medium to long-term synergy was likely to include immunological effects that went beyond the local effects of each components.

2) Its pharmacological constitution exceeded expectations: as described in the above Examples, the pharmacological constitution exceeded expectations for the pharmacological constitution of any non-specific immunotherapeutic pharmaceutical composition. It was generally believed that local effect was positively correlated with local irritation. However, the preferred probiotic semi-fluid component or probiotic water-soluble component had significantly lower local irritation (proportion of animals struggling violently during local administration) compared to the probiotic particle component (inactivated probiotics, probiotic water-insoluble particles), and the preferred co-administration substance was not ethanol, which was a stronger local irritant, but a weak local acting compound, which was a weaker local irritant, etc.

3) The technical effect exceeded expectations: the composition of the present invention could produce obvious and even effective medium- and long-term drug efficacy and systemic drug efficacy (cross-distance drug efficacy), which greatly exceeds the expected technical effect of the prior art compositions that could only produce short-term local drug efficacy. The medium- and long-term drug efficacy and systemic drug efficacy were particularly suitable for middle-aged and elderly patients, especially the elderly. In addition, the preferred compositions of the present invention showed almost no local irritation, whereas the local effect of classical chemical ablation agents (e.g. ethanol) was positively correlated with local irritation.

Wherein the co-administration substance capable of providing said probiotic component a local synergy or/and medium to long-term synergy could may also be a drug (chemical active ingredient or/and biologically active ingredient) or combination of drugs which was selected from other pharmaceutically suitable and provide meaningful local effect. For example,

1) a combination selected from a group comprising an alkali metal hydroxide, an alkaline inorganic salt, and one or more selected from methylene blue and its analogues, immunomodulators, cytotoxic drugs, such as the following combinations: a combination of alkali metal hydroxide/alkaline inorganic salt/ methylene blue and its analogues with a concentration ratio ($W_{alkali\ metal\ hydroxide}$/W alkaline inorganic salt /W methylene blue and its analogues) of (1-5%)/(3-15%)/(0.35-5%); a combination of alkali metal hydroxide/basic inorganic salt/immunomodulator with a

concentration ratio ($W_{\text{alkali metal hydroxide}}/W_{\text{basic inorganic salt}}/W_{\text{immunomodulator}}$) of (1-5%)/(3-15%)/(1-15%); a combination of alkali metal hydroxide/basic inorganic salt/ cytotoxic drug with a concentration ratio ($W_{\text{alkali metal hydroxide}}/W_{\text{basic inorganic salt}}/W_{\text{cytotoxic drug}}$) of (1-5%)/(3-15%)/(0.1- 10%).

2) a combination selected from a group comprising an alkaline organic compound, an alkaline inorganic salt, and one or more selected from methylene blue and its analogues, immunomodulators, cytotoxic drugs, such as the following combinations: a combination of alkaline organic compound/alkaline inorganic salt/methylene blue and its analogues with a concentration ratio ($W_{\text{alkaline organic compound}}/W_{\text{alkaline inorganic salt}}/W_{\text{methylene blue and its analogues}}$) of (5-25%)/(3-15%)/(0.35-5%); a combination of alkaline organic compound/alkaline inorganic salt/immunomodulator with a concentration ratio ($W_{\text{alkaline organic compound}}/W_{\text{alkaline inorganic salt}}/W_{\text{immunomodulator}}$) of (5-25%)/(3-15%)/(1-15%); a combination of alkaline organic compound/alkaline inorganic salt/cytotoxic drug with a concentration ratio ($W_{\text{alkaline organic compound}}/W_{\text{alkaline inorganic salt}}/W_{\text{cytotoxic drug}}$) of (5-25%)/(3-15%)/(0.1-10%).

3) a combination selected from a group comprising an alkaline organic compound, methylene blue and its analogues, and one or more selected from immunomodulators, cytotoxic drugs, non-basic amino acids and acid salts thereof, such as, a combination of alkaline organic compound/methylene blue and its analogues/immunomodulator with a concentration ratio ($W_{\text{alkaline organic compound}}/W_{\text{methylene blue and its analogues}}/W_{\text{immunomodulator}}$) Of (5-25%)/(0.35-3.5%)/(1-15%); a combination of alkaline organic compound/methylene blue and its analogues/cytotoxic drug with a concentration ratio ($W_{\text{alkaline organic compound}}/W_{\text{methylene blue and its analogues}}/W_{\text{cytotoxic drug}}$) of (5-25%)/(0.35-3.5%)/(0.1-10%); a combination of alkaline organic compound/methylene blue and its analogues/non-basic amino acids and acid salts thereof with a concentration ratio ($W_{\text{alkaline organic compound}}/W_{\text{methylene blue and its analogues}}/W_{\text{non-basic amino acids and acid salts thereof}}$) of (5-25%)/(0.35-3.5%)/(10-25%).

4) a combination selected from a group comprising a cytotoxic drug and at least two selected from alkaline organic compounds, immunomodulators, polyols, methylene blue and its analogs, such as a combination of cytotoxic drug /alkaline organic compound/immunomodulator with a concentration ratio ($W_{\text{cytotoxic drug}}/W_{\text{alkaline organic compound}}/W_{\text{immunomodulator}}$) of (0.1-10%)/(5-25%)/(1-15%); a combination of cytotoxic drug/polyol/ methylene blue and its analogs with a concentration ratio ($W_{\text{cytotoxic drug}}/W_{\text{polyol}}/W_{\text{methylene blue and its analogs}}$) of (0.1-10%)/(5-25%)/(0.35-3.5%); a combination of cytotoxic drug/ methylene blue and its analogs /immunomodulator with a concentration ratio ($W_{\text{cytotoxic drug}}/W_{\text{methylene blue and its analogs}}/W_{\text{immunomodulator}}$) of (0.1-10%)/(0.35-5%)/(1-15%)。

The above mentioned was only a preferable Example of the invention, and was not used to limit the invention. Any modification, equivalent replacement, improvement, etc. made within the spirit and principles of the invention shall be included in the scope of protection of the invention.

## Claims

1. A use of a probiotic component as an active ingredient capable of providing a therapeutic effect in the manufacture of a local pharmaceutical composition for the treatment of local lesion diseases, wherein said therapeutic effect comprises a local treatment involving a local effect (or a local synergy) or/and an immunotherapy.

2. A local pharmaceutical composition for the treatment of local lesion diseases comprising a probiotic component capable of providing a therapeutic effect, and a pharmaceutically acceptable suitable carrier, but not comprising a salt or monosaccharide dedicated to increasing blood osmolarity to a normal physiological level, wherein said therapeutic effect comprises a local treatment involving a local effect (or a local synergy) or/and an immunotherapy.

3. The use or the pharmaceutical composition according to claim 2 or 3, wherein said pharmaceutical composition further comprises a chemical active ingredient or/and a biologically active ingredient capable of synergizing with said therapeutic effect of said probiotic component, and in said pharmaceutical composition, an amount ratio of said probiotic component to said chemical active ingredient (weight concentration of the probiotic component/weight concentration of the chemical active ingredient) is (1-110)/(1-100) or (1.5-100)/(0.1-1), or/and an amount ratio of said probiotic component to said biologically active ingredient (weight concentration of the probiotic component/weight concentration of the bioactive ingredient) is (60-180)/(0.1-60).

4. A local pharmaceutical composition for the treatment of local lesion diseases, comprising a probiotic component capable of providing a therapeutic effect, a chemical active ingredient or/and a biologically active ingredient capable of synergizing with said therapeutic effect of said probiotic component, and in said pharmaceutical composition, an amount ratio of said probiotic component to said chemical active ingredient (weight concentration of the probiotic component/weight concentration of the chemical active ingredient) is (1-110)/(1-100) or (1.5-100)/(0.1-1), or/and an amount ratio of said probiotic component to said biologically active ingredient (weight concentration of the probiotic component/weight concentration of the biologically active ingredient) is (60-180)/(0.1-60), wherein said therapeutic effect comprises a local treatment involving a local effect (or a local synergy) or/and an immunotherapy.

5. The use or the pharmaceutical composition according to any one of claims 1-4, wherein said probiotic component is selected from: a preparation having a minimized bacterial immunogenicity and derived from natural probiotics or engineered probiotics thereof, or engineered analogues of the preparation, wherein said preparation is preferably one or more selected from a group comprising: a probiotic water-soluble component, a probiotic semi-fluid-like component, a probiotic component water-insoluble particle, inactivated probiotics, and derivatives thereof.

6. The use or the pharmaceutical composition according to claim 5, wherein said probiotic water-soluble component is selected from a group comprising one or more of a probiotic or disrupted probiotic supernatant component, a probiotic extract, a probiotic intracellular water-soluble component, water-soluble derivatives of water-soluble components or non-water-soluble components from probiotic cell wall components, a probiotic ribonucleic acid, and derivatives thereof;

said probiotic semi-fluid-like component is preferably selected from one or more of probiotic components whose aqueous mixture is capable of forming a semi-fluid-like composition, such as one or more of probiotic polysaccharides and analogues thereof;
said probiotic component water-insoluble particle is selected from a group comprising one or more of a disrupted probiotic precipitation component, a probiotic cell wall polysaccharide particle, a probiotic cell wall polysaccharide nanoparticle, and derivatives thereof;
said inactivated probiotics is preferably selected from but not limited to inactivated probiotics having intact morphology,
wherein said probiotic water-soluble component is selected from a group comprising one or more of: a probiotic water-soluble β-glucan, preferably selected from a water-soluble β-glucan of greater than 90% purity, water-soluble derivatives of a probiotic β-glucan, a probiotic ribonucleic acid, and derivatives thereof.

7. The use or the pharmaceutical composition according to any one of claims 1-6, wherein said probiotic is selected from a group comprising one or more of natural or/and engineered: probiotic Bacillus, probiotic Lactobacillus, probiotic Bifidobacterium, probiotic Fungus, wherein:

said Bacillus comprises one or more selected from a group comprising: Bacillus licheniformis, Bacillus subtilis, Bacillus pumilus, Bacillus natto;
said Lactobacillus comprises one or more selected from a group comprising: Lactobacillus acidophilus, Lactobacillus casei, Lactobacillus plantarum, Lactobacillus pumilus and Lactobacillus fermentum;
said Bifidobacterium comprises one or more selected from a group comprising: Bifidobacterium longum, Bifidobacterium adolescentis, Bifidobacterium breve, Enterococcus faecium, and Streptococcus fecalis;
said Fungus comprises one or more selected from a group comprising: yeasts and *Brettanomyces*, wherein said yeasts comprises one or more selected from a group comprising: Saccharomyces cerevisiae, Torulaspora delbrueckii, Wickerhamomyces, Pichia, Candida utilis, Kluyveromyces marxianus.

8. The use or the pharmaceutical composition according to any one of claims 1-7, wherein said probiotics comprise selected from Saccharomyces cerevisiae or/and Brettanomyces bruxellensis.

9. The use or the pharmaceutical composition according to any one of claims 1-8, wherein said probiotic component is present in the pharmaceutical composition at a concentration of > 0.1%, ≥0.25%, 0.25-25%, preferably 0.5-25%, more preferably 1-5%, 5-15% or 15-25%.

10. The use or the pharmaceutical composition according to claim 9, wherein

if said probiotic component comprises said inactivated probiotics, then said inactivated probiotics is present in the pharmaceutical composition at a concentration of > 0.3%, >_0.75%, 0.75-15, preferably 1.5-15% or 5-15%;
if said probiotic component comprises said probiotic water-soluble component, then said probiotic water-soluble component is present in the pharmaceutical composition at a concentration of > 0.1, or 0.15-25%, preferably 0.35-5%, 5-15% or 15-25%;
if said probiotic component comprises said probiotic water-insoluble component particle, then said probiotic water-insoluble component particle is present in the pharmaceutical composition at a concentration of > 0.5, or 0.5-15%, preferably 1.5-15% or 5-15%;

if the pharmaceutical composition is a semi-fluid-like composition, then said probiotic semi-fluid-like component is present in the pharmaceutical composition at a concentration of > 2.5%, 2.6-25%, preferably 5-15%.

11. The use or the pharmaceutical composition according to any one of claims 3-10, wherein said chemical active ingredient is one or more selected from a group comprising a weak local acting compound and/or a cytotoxic agent, and wherein when said chemical active compound comprises a cytotoxic agent, the amount ratio of the probiotic component to the cytotoxic agent ($W_{probiotic\ component}$ /$W_{cytotoxic\ agent}$) is (1-110)/(1-100); when said chemical active compound comprises a weak local acting compound, the amount ratio of the probiotic component to the weak local acting compound ($W_{probiotic\ component}$ /$W_{weak\ local\ acting\ compound}$) is (1-90)/(1-100).

12. The use or the pharmaceutical composition according to claim 11, wherein when said weak local acting compound is selected from a group comprising one or more of: an amino acid nutrient, a vital dye, a quinine drug, a low concentration acidifier, a low concentration basifier, a pH buffering system comprising an acidifier or/and a basifier, and wherein when said weak local acting compound comprises said amino acid nutrient, the amount ratio of the probiotic component to the amino acid nutrient, ($W_{probiotic\ component}$/$W_{amino\ acid\ nutrient}$) is (1-20)/(1-100),

when said weak local acting compound comprises said vital dye, the amount ratio of the probiotic component to the vital dye, ($W_{probiotic\ component}$/$W_{vital\ dye}$) is (7-90)/(1-100);
when said weak local acting compound comprises said quinine drug, then the amount ratio of the probiotic component to the quinine drug, ($W_{probiotic\ component}$/$W_{quinine\ drug}$) is (2-90)/(1-100);
when said weak local acting compound comprises said acidifier or/and basifier, the amount ratio of the probiotic component to the acidifier or/and basifier, ($W_{probiotic\ component}$/$W_{acidifier\ or/and\ basifier}$) is (2-60)/(1-100).

13. The use or the pharmaceutical composition according to claim 12, wherein said amino acid nutrient is amino acids or salts thereof selected from a group comprising, or oligopeptides and polypeptides comprising or consisting of arginine, lysine, glycine, cysteine, alanine, serine, aspartic acid, glutamic acid, and said amino acid nutrient is present at a concentration of >2.5%, or 5-30%, preferably 5-25%.

14. The use or the pharmaceutical composition according to claim 12, wherein said vital dye is one or more selected from a group comprising Bengal Red or/and following methylene blue-like dyes: methylene blue, patent blue, isosulphur blue, neo-methylene blue, and wherein a concentration of said Bengal Red is 2.5%-20%; a concentration of said methylene blue-like dyes is ≥ 0.25%, or 0.25-2.5%, preferably 0.5-2.5%.

15. The use or the pharmaceutical composition according to claim 12, wherein said acidifier is selected from one or more of a strong acid or/and a weak acid, and the amount ratio of said probiotic component to said acidifier (weight concentration of the probiotic component/weight concentration of the acidifier) is (1-20)/(0.5-50), and the concentration of said acidifier is ≥0.5%, 0.5-2% (strong acid), or 2-15% (weak acid), wherein said strong acid is, for example, hydrochloric acid; said weak acid is, for example, glycolic acid, acetic acid, lactic acid, citric acid, malic acid; said basifier is, for example, selected from one or more of strong or/and weak alkalis, and the amount ratio of said probiotic component to said basifier (weight concentration of the probiotic component/weight concentration of the basifier) is (1-20)/(0.5-50), and the concentration of said basifier is ≥ 0.5%, 0.5-5% (strong alkali), or 2-15% (weak alkali), wherein said strong alkali is, for example, sodium hydroxide, potassium hydroxide; said weak alkali is, for example, sodium dihydrogen phosphate, sodium bicarbonate, sodium carbonate.

16. The use or the pharmaceutical composition according to claim 11, wherein said cytotoxic agent comprises one or more selected from a group comprising: drugs that disrupt DNA structure and function, such as cyclophosphamide, carmustine, metal platinum complexes, doxorubicin drugs, topotecan, irinotecan; drugs intercalated in DNA to interfere with RNA transcription, such as antitumor antibiotics; drugs that interfere with DNA synthesis, such as 5-fluorouracil (5-Fu), furofluorouracil, difurofluorouracil, cytosine arabinoside, cyclic cytidine, 5-azacytidine; drugs that affect protein synthesis, such as colchicine drugs, vincristine drugs, and taxane drugs, and a concentration of said cytotoxic agent is ≥ 0.1%, 0.1-15%.

17. The use or the pharmaceutical composition according to any one of claims 1-16, wherein said biologically active ingredient is selected from one or more of: antigens, immunomodulatory antibodies, cytokines, and adjuvants.

18. A method for treating a local lesion disease comprising the steps of locally administering a therapeutically effective amount of the pharmaceutical composition according to any one of claims 3-22 to an individual in need thereof within a local lesion or/and outside a local lesion, said locally administering outside a local lesion is conducive to producing a secondary immunological effect associated with a local effect (or a local synergy).

19. The method according to claim 18, wherein

if said probiotic component comprises said inactivated probiotics, then the amount of said inactivated probiotics in the pharmaceutical composition must be such that it is administered at a local concentration of > 0.3%, >_0.75%, 0.75-15, preferably 1.5-15% or 5-15%;

if said probiotic component comprises said probiotic water-soluble component, then the amount of said probiotic water-soluble component in the pharmaceutical composition must be such that it is administered at a local concentration of > 0.1, or 0.15-25%, preferably 0.35-5%, or 5-15%;

if said probiotic component comprises said probiotic water-insoluble component, then the amount of said probiotic water-insoluble component in the pharmaceutical composition must be such that it is administered at a local concentration of > 0.5, or 0.5-15%, preferably 1.5-15% or 3.5-15%.

20. The use, the pharmaceutical composition or the method according to any one of claims 1-19, wherein said local lesion comprises tumor cells or/and fibroblasts.

21. The use, the pharmaceutical composition, or the method according to any one of claims 1-20, wherein said local lesion disease comprises a tumor, a non-tumor enlargement, a local inflammation, an endocrine gland dysfunction, and a skin disease, wherein said tumor comprises malignant and non-malignant solid tumors.

22. The use, the pharmaceutical composition, or the method according to claim 21, wherein said solid tumor comprises one or more of the following tumors and secondary tumors thereof: breast cancer, pancreatic cancer, thyroid cancer, nasopharyngeal cancer, prostate cancer, liver cancer, lung cancer, intestinal cancer, oral cancer, esophageal cancer, gastric cancer, laryngeal cancer, testicular cancer, vaginal cancer, uterine cancer, ovarian cancer, brain tumor, and lymphoma.

23. The use, the pharmaceutical composition, or the method according to any one of claims 1-22, wherein the applicable patient for said treatment is selected from a group comprising one or more of: an immunosuppressed patient, a patient who can be administered locally within the lesion, a patient whose local lesion tissue is amenable to a chemical ablation-like, a patient who can produce a secondary immune substance within the local lesion, and a patient who can produce a secondary immune substance within the administration region outside a lesion.

24. A pharmaceutical composition comprising a probiotic component and a pharmaceutically acceptable suitable carrier, for the treatment of a local lesion disease, wherein said local lesion disease comprises a solid tumor.

25. A pharmaceutical kit comprising one or more containers containing the pharmaceutical composition according to any one of claims 2-22, and optionally instructions or labels on how to administer said pharmaceutical composition to an individual in need thereof.

<table>
<tr><td colspan="2" align="center">**INTERNATIONAL SEARCH REPORT**</td><td>International application No.<br>**PCT/CN2021/122132**</td></tr>
</table>

**A. CLASSIFICATION OF SUBJECT MATTER**

A61K 35/741(2015.01)i; A61K 35/742(2015.01)i; A61K 35/747(2015.01)i; A61K 35/745(2015.01)i; A61K 36/064(2006.01)i; A61K 45/06(2006.01)i; A61K 39/00(2006.01)i; A61P 35/00(2006.01)i; A61P 37/04(2006.01)i; A61P 29/00(2006.01)i; A61P 5/00(2006.01)i; A61P 17/00(2006.01)i; A61K 35/12(2015.01)i; A61K 36/00(2006.01)i; A61K 36/02(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNABS; CNTXT; CNKI; USTXT; EPTXT; WOTXT; VEN; ISI web of knowledge; STNext: 成都夸常奥普医疗科技有限公司, 邹方霖, 邹礼常, 王建霞, 王艺羲, 益生菌, 局部, 肿瘤, 癌症, 载体, 芽孢杆菌, 乳酸杆菌, 双歧杆菌, 真菌, 酵母菌, 协同, 氨基酸, 染料, 活性成分, CHENGDU KUACHANGAOPU MEDICAL, ZOU Fanglin, ZOU Lichang, WANG Jianxia, WANG Yixi, Probiotics, topical, tumor, cancer, carrier, Bacillus, Lactobacillus, Bifidobacterium, fungal, yeast, synergistic, amino acid, dye, active ingredient

**C. DOCUMENTS CONSIDERED TO BE RELEVANT**

| Category* | Citation of document, with indication, where appropriate, of the relevant passages | Relevant to claim No. |
|---|---|---|
| X | WO 2012074351 A1 (UNIVERSITI PUTRA MALAYSIA) 07 June 2012 (2012-06-07) claims 1-3 and 20 | claims 1-17, 20-23 (in part), 24, and 25 |
| A | CN 110870914 A (CHENGDU KUACHANG AOPU MEDICAL TECHNOLOGY CO., LTD.) 10 March 2020 (2020-03-10) entire document | claims 1-17, 20-23 (in part), 24, and 25 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 November 2021** | **29 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<div style="text-align:center">**INTERNATIONAL SEARCH REPORT**</div>

| International application No. |
|---|
| **PCT/CN2021/122132** |

| Box No. II      Observations where certain claims were found unsearchable (Continuation of item 2 of first sheet) |
|---|

This international search report has not been established in respect of certain claims under Article 17(2)(a) for the following reasons:

1. ☑   Claims Nos.: **18、 19、 20-23**（部分）
   because they relate to subject matter not required to be searched by this Authority, namely:

   [1]   The subject matter of claim 18 relates to a method for treatment of a local lesion disease, which is a method for treatment for a living human body or animal body, and falls within the subject matter defined in PCT Rule 39.1(iv) that does not warrant a search conducted by the international searching authority.

   [2]   On the basis of the same reasoning, claims 19 and 20-23 (in part) fall within the subject matter defined in PCT Rule 39.1(iv) that does not warrant a search conducted by the international searching authority.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

International application No.

**PCT/CN2021/122132**

| Patent document cited in search report | | | Publication date (day/month/year) | Patent family member(s) | | | Publication date (day/month/year) |
|---|---|---|---|---|---|---|---|
| WO | 2012074351 | A1 | 07 June 2012 | EP | 2646042 | A1 | 09 October 2013 |
| | | | | EP | 2646042 | A4 | 23 July 2014 |
| | | | | BR | 112012021624 | A2 | 07 February 2017 |
| | | | | KR | 20130132398 | A | 04 December 2013 |
| | | | | KR | 101747815 | B1 | 15 June 2017 |
| | | | | SG | 183544 | A1 | 30 October 2012 |
| | | | | JP | 2014500263 | A | 09 January 2014 |
| | | | | JP | 5913353 | B2 | 27 April 2016 |
| | | | | US | 2016030492 | A1 | 04 February 2016 |
| | | | | US | 2013323215 | A1 | 05 December 2013 |
| | | | | US | 9480721 | B2 | 01 November 2016 |
| | | | | AU | 2010365001 | A1 | 30 August 2012 |
| | | | | AU | 2010365001 | B2 | 12 December 2013 |
| | | | | IN | 201206771 | P1 | 17 January 2014 |
| | | | | SG | 183544 | B | 20 April 2016 |
| CN | 110870914 | A | 10 March 2020 | EP | 3845247 | A1 | 07 July 2021 |
| | | | | KR | 20210055052 | A | 14 May 2021 |
| | | | | CN | 112789059 | A | 11 May 2021 |
| | | | | WO | 2020043185 | A1 | 05 March 2020 |
| | | | | IN | 202117014254 | A | 07 May 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106387398 A

- CN 108524925 A

**Non-patent literature cited in the description**

- **BURGI Y.** Cancerres. *Pharmacology; Drug actions and reactions,* 1978, vol. 38 (2), 284-285

- **ZHENGJUN JIN.** Equal probability and curve and ''Q50. *Journal of the Second Shanghai Medical College,* 1981, vol. 1, 75-86